# EUROPEAN PATENT SPECIFICATION

(11) **EP 1 010 759 B1**
(45) Date of publication and mention of the grant of the patent: **28.03.2007**
(21) Application number: 98933947.8
(22) Date of filing: 27.07.1998
(51) Int. Cl.: C12N 15/51, C12N 7/02, C07K 14/18, C07K 16/10, A61K 39/29, G01N 33/576, C07K 14/01, C12N 7/00

(54) **NON-B NON-C NON-G HEPATITIS VIRUS GENE, POLYNUCLEOTIDE, POLYPEPTIDE, VIRION, METHOD FOR SEPARATING VIRION, AND METHOD FOR DETECTING VIRUS**
GEN, POLYNUKLEOTID, POLYPEPTID UND VIRION DES NON-B, NON-C, NON-G-HEPATITIS VIRUS, EINE METHODE ZUR ABTRENNUNG DIESES VIRIONS UND METHODEN FÜR DIE VIRUSDETEKTION.
GENE DU VIRUS DE L'HEPATITE NON-B, NON-C, NON-G, POLYNUCLEOTIDE, POLYPEPTIDE ET VIRION CORRESPONDANTS, PROCEDE DE SEPARATION DU VIRION, ET PROCEDE DE DETECTION DE CE VIRUS

(30) Priority: 25.07.1997 JP 23324697; 09.10.1997 JP 31419697; 13.03.1998 JP 8296298
(43) Date of publication of application: 21.06.2000
(73) Proprietor: Tamura, Ryoji, Kitakatsushika-gun, Saitama 340-0203 (JP)
(72) Inventor: OKAMOTO, Hiroaki, Shimotsuga-gun, Tochigi 329-0414 (JP); NISHIZAWA, Tsutomu, Nara-shi, Nara 630-8043 (JP)
(74) Representative: Bannerman, David Gardner
(86) International application number: PCT/JP1998/003340
(87) International publication number: WO 1999/005282

(56) References cited:
- WO-A-93/15111
- JP-A- 1 279 844
- JP-A- 9 299 078
- KUWADA S K ET AL: "NON-A, NON-B FULMINANT HEPATITIS IS ALSO NON-E AND NON-C" GASTROENTEROLOGY, SAUNDERS, PHILADELPHIA, PA,, US, vol. 89, no. 1, 1994, pages 57-61, XP000561262 ISSN: 0016-5085
- FIORDALISI G ET AL: "HIGH PREVLENCE OF GB VIRUS C INFECTION IN A GROUP OF ITALIAN PATIENTS WITH HEPATITIS OF UNKNOWN ETIOLOGY" JOURNAL OF INFECTIOUS DISEASES, CHICAGO, IL, US, vol. 174, no. 1, 1 July 1996 (1996-07-01), pages 181-183, XP000647922 ISSN: 0022-1899
- DATABASE EMBL SEQUENCES [Online] Accession No B21160, 20 September 1997 (1997-09-20) FENG J. ET AL.: "A. thaliana genomic clone F9A20" XP002191508
- BIOCHEM. BIOPHYS. RES. COMMUN., Vol. 241, (18-12-1997), T. NISHIZAWA et al., "A Novel DNA Virus (TTV) Associated with Elevated Transaminase Levels in Posttransfusion Hepatitis of Unknown Etiology", p. 92-97, XP002917290
- THE LANCET, Vol. 352, (18-07-1998), P. SIMMONDS et al., "Detection of a Novel DNA Virus (TTV) in Blood Donors and Blood Products", p. 191-195, XP002917299
- HEPATOL. RES., Vol. 10, (1998), H. OKAMOTO et al., "Molecular Cloning and Characterization of a Novel DNA Virus (TTV) Associated with Posttransfusion Hepatitis of Unknown Etiology¹", p. 1-16, XP002918456
- HEPATOL. RES., Vol. 10, (1998), K. TAKAHASHI et al., "Partial-2.4kb Sequences of TT-Virus (TTV) Genome from 8 Japanese Isolates: Diagnostic and Phylogenetic Implications¹", p. 111-120, XP002918171
- J. MED. VIROL., Vol. 56, (1998), OKAMOTO H. et al., "Fecal Excretion of a Nonenveloped DNA Virus (TTV) Associated with Posttransfusion Non-A-G Hepatitis", p. 128-132, XP002917300

## Description

### Technical Field

The present invention relates to a gene, a polynucleotide, a polypeptide, an antibody, and an antigen, which have become available by discovery of an etiologic blood-borne infectious hepatitis virus whose etiology could not be identified by conventional diagnostic methods, as well as methods for the production, detection and assay of a virus gene, an antibody and an antigen, by utilizing the gene, the polynucleotide, the polypeptide, the antibody and the antigen which have been available, a virus particle and method for isolation of the virus particle.

### Background Art

To date, hepatitis B virus (abbreviated as "HBV" hereinafter) and hepatitis C virus (abbreviated as "HCV" hereinafter) have been discovered as etiologic viruses of blood-borne infectious hepatitis. Diagnostic methods for both viruses have already been established, and they were early introduced into the screening of blood for blood transfusion in this country. As a result, it has become possible to substantially completely prevent novel infection cases in blood recipients for both hepatitis viruses.

However, even after the diagnostic methods for HBV and HCV were established, cases suspected of cryptogenic viral hepatitis constitute 5-10% of all hepatitis cases. These cases have not been considered to be caused by hepatitis A virus, which is a hepatitis virus of non-blood-borne infectious type, hepatitis E virus, hepatitis F virus, which has been reported only in India and its existence itself is doubted, and hepatitis D virus, which is a defective hepatitis virus, and possible existence of unknown hepatitis virus has been suggested.

Gene sequences of viruses considered to be etiologic agents of these hepatitis cases were reported in succession by Abbott, U.S.A. in 1995, and then by Genelabs Technologies in 1996, and designated as GBV-C and HGV, respectively. However, afterwards these viruses became to be considered to be identical to each other based on comparison of their sequences (abbreviated as "GBV-C/HGV" hereinafter). Studies on the involvement of GBV-C/HGV in cryptogenic viral hepatitis are being actively performed also in this country. As a result, it has thus far been considered at least that GBV-C/HGV does not account for all of the causes of non-B, non-C hepatitis of unknown etiology, because expression of hepatitis symptoms has been slight in its infection cases while it may be transmitted via hematic route, and therefore an unknown virus might be responsible for the cryptogenic hepatitis Existence of an unknown hepatitis virus causing blood-borne infectious hepatitis has been suggested as described above, and it has been desired to discover this unknown hepatitis virus, and elucidate genetic, molecular-biological and epidemiological characteristics of the virus, thereby realizing more complete prophylactics, diagnostic methods and therapies for hepatitis.

In other words, in order to develop diagnostic methods and therapeutic methods of hepatitis caused by an unidentified virus, it is desired to obtain genetic information for the virus, to determine virus-specific gene sequences and amino acid sequences, to determine locations of epitopes, to establish methods for the production of biological materials containing the epitopes, to establish methods for the production of antigens which specifically react with antiviral antibodies, to provide specific antibodies for the virus, to establish methods for isolating and collecting virus particles, to establish methods for treating the virus particles to attenuate their biological activity while maintaining their immunological activity, and to develop methods for assaying genes, antibodies, and antigens, and methods for producing neutralizing antibody-derived vaccines, which utilize the aforementioned biological materials derived from the virus to be obtained.

Kuwada *et al* (American Journal of Gastroenterology (1994) Vol 89(1): 57-61) aimed to define the roles of the hepatitis C and E viruses (HCV and HEV) in non-A, non-B (NANB) fulminant hepatitis. None of the 15 patients had detectable HCV or HEV RNA or elevated HCV and IgM-HEV antibody titers in their acute phase sera. Three patients, all with features of autoimmune hepatitis, had raised IgG-HEV antibody titers. No evidence of HBV DNA was found. The authors concluded that there was no evidence implicating HCV or HEV in presumed viral NANB FHF or as agents contributing to or causing the liver failure in nonviral NANB FHF patients with autoimmune hepatitis, drug-induced hepatotoxicity, or halothane hepatotoxicity.

WO9315111 discloses the isolation and characterization of a novel cDNA clone, 20E, which encodes a previously unknown polypeptide antigen recognized by antibodies in the serum of certain patients with non-A, non-B hepatitis (NAMBH). WO9315111 states that the nucleic acid sequence is not represented in the genome of the Hepatitis C Virus (HCV) and neither is the nucleic acid sequence represented in the human genome. WO9315111 states that the data suggest that the RNA sequence corresponding to clone 20E is contained within the genome of an external infective agent other than HCV, and therefore may represent an additional etiologic agent or contributing factor to the development of NANBH in human patients. WO9315111 suggests that the 20E nucleic acid and corresponding polypeptide and respective variants thereof will be useful in a variety of procedures directed at prevention and diagnosis of NANBH.

Fiordalisi *et al* (Journal of Infectious Diseases (1996) Vol 174(1): 181-3) evaluated the prevalence of the recently discovered GB virus C(GBV-C) in a cohort of 49 Italian patients with acute or chronic hepatitis of unknown etiology (non-A-E hepatitis) and in a control group of 100 healthy blood donors. They concluded that GBV-C may be an important agent, contributing, at least in Italy, to a significant number of the cases of hepatitis of unknown etiology.

Okamoto *et al* (Hepatology Research (1998) Vol 10, pages 1-16) disclose that the genomic DNA of a novel virus named TT virus (TTV), associated with post-transfusion hepatitis of unknown etiology, was cloned from plasma of a blood donor with an elevated transaminase level but without serological markers of known hepatitis viruses, and its sequence of 3739 bases was determined. They concluded that their results indicate that TTV would be responsible for a part of acute and chronic liver disease of unknown etiology. These sequences are available via GenBank under accession number AB008394.1 (GI:2959440).

### SUMMARY OF THE INVENTION

The inventors have studied forms of viral hepatitis of which the etiologic virus has not yet been identified, and therefore for which means for diagnosis, prevention and treatment have not been developed.

The inventors have also isolated genes from a previously unknown type of Hepatitis virus and determined their sequences, thereby providing genes, polynucleotides, polypeptides, methods for isolating virus particles, virus particles, viral antigens, and antiviral antibodies, which can be utilized for diagnosis and treatment, and methods for detecting the virus.

The present inventors assumed that, in the blood of patients suffering from hepatitis for which a cause cannot be identified by conventional diagnostic methods of viral hepatitis, unknown hepatitis virus particles or a part thereof should be present. Based on this assumption, the present inventors attempted to isolate a viral gene from such blood. Specifically, the present inventors established a hypothesis that the viral gene would exist in blood of hepatitis patients, but does not exist in the human genome and does not exist in the blood of most of normal persons, or that it does not exist before the crisis of hepatitis, but does exist after the crisis. The inventors searched for candidate genes in the blood of hepatitis patients, based on this hypothesis, and isolated them. Further, the candidate genes were selected according to the following criteria, and a gene satisfying all of the criteria was ultimately considered to be a novel viral gene:
(1) the gene exists in blood of a plurality of hepatitis patients for which the cause of hepatitis has not been elucidated,
(2) there are cases that blood transfusion recipients have become positive for the gene due to transfusion with blood positive for the gene, while the gene has not existed in their blood before the blood transfusion, and crisis of hepatitis has been observed in them after the blood transfusion,
(3) the obtained gene sequence is not homologous to gene sequences of known hepatitis viruses or other known viruses, and
(4) when blood positive for the gene is analyzed by density gradient centrifugation, which is widely used as a method for isolating and collecting virus particles, a fraction positive for the gene is found in a usual virus particle fraction.

Asymptomatic carriers, i.e., infection cases which are positive for a hepatitis virus but do not exhibit symptoms of hepatitis and appear healthy, are known in infections with known hepatitis, viruses, and it was considered that such scenario would be well possible for the infection with as yet unknown hepatitis viruses. Therefore, the selection criteria do not contain a criterion merely that the genes be not found in normal persons." Instead, as can be seen from the actual selection criteria, we focussed on correlation with the onset of a hepatitis crisis.

Using the aforementioned criteria, the inventors isolated a viral DNA sequence associated with hepatitis. This sequence can be found in viral fractions obtained from the blood of infected patients using density gradient centrifugation, confirming that the sequence is indeed viral. The inventors determined the sequence of this novel gene, and on the basis of this sequence they designed oligonucleotides suitable as PCR primers. A method for detecting the gene using this primers was established. In particular, it is possible to amplify by PCR a nucleic acid sequence of about 3500 to 4000 nucleotides, and preferably of about 3600 to 3900 nucleotides, using as primers the oligonucleotides of SEQ ID NO:57 and SEQ ID NO:60, or the oligonucleotides of SEQ ID NO:57 and SEQ ID NO:61. The 5' and 3' ends of the amplified nucleic acid sequence typically should share 70% or more homology with nucleotides 3-300 and 2402-3739, respectively, of SEQ ID NO:1. Preferably, the degree of homology is at least 80%.

In a first embodiment of the invention, there is provided an isolated non-B non-C, non-G hepatitis virus DNA which shows at least 60% homology to the sequence of SEQ ID NO: 1.

In an aspect of the invention, from said DNA a fragment of about 200 to 350 nucleotides in length can be amplified by PCR using as primers the oligonucleotides of SEQ ID NO:6 and SEQ ID NO:8, or the oligonucleotides of SEQ ID NO:7 and SEQ ID NO:8.

In a preferred aspect of the invention, the DNA consists of SEQ ID NO:1 or an allogeneic variant thereof selected for instance from the non-B non-C non-G hepatitis virus sequences shown in SEQ ID NO:45 (Genotype 1), SEQ ID NO:46 (Genotype 2), SEQ ID NO:47 (Genotype 3), SEQ ID NO:48 (Genotype 4), SEQ ID NO:49 (Genotype 5), SEQ ID NO:50 (Genotype 6). Also disclosed are further variants, shown in SEQ ID NO:51 (Genotype 7), SEQ ID NO:52 (Genotype 8), SEQ ID NO:53 (Genotype 9) and SEQ ID NO:54 (Genotype 10).

On the basis of the DNA sequences of the invention, specific cognate oligonucleotides can be designed which hybridize specifically to the gene of the present invention or its complementary sequence. In a preferred aspect of the invention, there are provided specific oligonucleotides as shown in SEQ ID NO:2 (RD037), SEQ ID NO:3 (RD038), SEQ ID NO:4 (RD051), SEQ ID NO:5 (RD052), SEQ ID NO:6 (NG059), SEQ ID NO:7 (NG061), SEQ ID NO:8 (NG063).

The present invention also provides a method for detecting a non-B, non-C, non-G hepatitis virus gene by PCR amplification using the oligonucleotides of the present invention as primers. A first preferred method of detection involves the use of one of the following primer pairs: the oligonucleotides of SEQ ID NO:2 and SEQ ID N0:3; the oligonucleotides of SEQ ID NO:4 and SEQ ID NO:5. A second preferred method of detection method involves the use of one of the following primer pairs: the oligonucleotides of SEQ ID NO:6 and SEQ ID NO:8; the oligonucleotides of SEQ ID NO:7 and SEQ ID NO:8.

The present invention also provides a method for differentiating non-B non-C non-G hepatitis virus genotypes, wherein a biological sample is subjected to both the first and second preferred methods of detection defined above, and the results obtained from the two detection methods are compared.

The present invention also provides a method for differentiating non-B non-C non-G hepatitis virus genotypes, comprising hybridization using oligonucleotide probes specific for a given genotype and the sequences of which are present in any one of SEQ ID NO:45 (Genotype 1), SEQ ID NO:46 (Genotype 2), SEQ ID NO:47 (Genotype 3), SEQ ID NO:48 (Genotype 4), SEQ ID NO:49 (Genotype 5), SEQ ID NO:50 (Genotype 6), SEQ ID NO:51 (Genotype 7), SEQ ID NO:52 (Genotype 8), SEQ ID NO:53 (Genotype 9) and SEQ ID NO:54 (Genotype 10).

The present invention further provides a polypeptide encoded by an open reading frame present in the DNA of the present invention. In a preferred embodiment, such polypeptides have the amino acid sequence shown in SEQ ID NO:9 or SEQ ID NO:10, or fragments or homologs thereof (with at least 65% homology) that are still specific to non-B non-C non-G hepatitis viruses. In a further preferred embodiment, such polypeptides contain an non-B non-C non-G hepatitis virus-specific epitope.

Furthermore, the present invention provides for a method for the isolation of non-B non-C non-G hepatitis viruses containing the DNA of the invention, which method is based on the density of the viral particles. The invention also provides for viral particles so isolated. The invention also provides peptides obtained from the particles, wherein said peptides are encoded within an open reading frame of a DNA of the invention: such peptides must also be counted among the polypeptides of the invention.

The present invention further provides:
- a recombinant gene expression which comprises all or a part of a nucleotide sequence encoding the amino acid sequence son in SEQ ID NO:9 or SEQ ID NO: 10;
- a cell transformed with said expression vector;
- a method for producing a non-B non-C non-G hepatitis virus antigenic peptide comprising the culture of said transformant cell under conditions in which said peptide is expressed and collecting the expressed peptide;
- an antigenic peptide so obtained or a fragment thereof;
- a method for immunologically detecting a non-B non-C non-G hepatitis virus antibody using a polypeptide of the present invention;
- a method for producing an antibody, comprising the immunization of an animal with the polypeptide of the invention or with an isolated particle of the invention; antibodies so obtained; a method for immunologically detecting a non-B non-C non-G hepatitis virus using said antibodies.

It should be noted that the antigenic peptides and the viral particles of the invention might prove useful for the formulation of vaccines.

In an alternative embodiment, there is provided an isolated non-B non-C, non-G hepatitis virus DNA consisting of the sequence of SEQ ID NO:62. Also for this gene, there are provided:
- a polynucleotide having a nucleotide sequence complementary to said DNA;
- specific cognate oligonucleotides designed designed on the basis of said DNA and which hybridize specifically to the gene of the present invention or its complementary sequence,
- a method for detecting a non-B non-C non-G hepatitis virus gene by PCR amplification using the aforementioned oligonucleotides as primers,
- a polypeptide encoded by the open reading frame present in the nucleotide sequence of this invention.
- a recombinant expression vector comprising a nucleotide sequence encoding a polypeptide of the invention;
- a cell transformed with said expression vector;
- a method for producing a non-B non-C non-G hepatitis virus antigenic peptide comprising the culture of said transformant cell under conditions in which said peptide is expressed and collecting the expressed peptide;
- an antigenic peptide so obtained or a fragment thereof;
- a method for immunologically detecting a non-B non-C non-G hepatitis virus antibody using a polypeptide of the present invention;
- a method for producing an antibody, comprising the immunization of an animal with the polypeptide of the invention or with an isolated particle of the invention; antibodies so obtained; a method for immunologically detecting a non-B non-C non-G hepatitis virus using said antibodies.

### DETAILED DESCRIPTION OF THE INVENTION

The present invention relatesto a previously unknown virus which is different from currently known viruses. The term "non-B non-C non-G hepatitis virus" used in the present invention means an etiologic virus transmitted via blood-borne infection, and causing hepatitis symptoms. It is distinct from hepatitis B, C, and G viruses, which are similarly blood-borne infectious viruses. It is also different from hepatitis A virus, hepatitis E virus, and hepatitis F virus, which are of the non-blood-borne infectious type, and hepatitis D virus, which is a defective virus. This it could be also called non-A, non-B, non-C, non-D, non-E, non-F, non-G hepatitis virus.

"Hepatitis symptoms" are symptoms generally representative of hepatitis, such as abnormal hepatic function values and appearance of icterus.

The virus of the present invention is identified as a hepatitis virus based on the context of its discovery, but it does not necessarily mean that it cannot cause other diseases.

"HNT22" is a non-B, non-C, non-G hepatitis virus having a gene as shown in SEQ ID NO: 1 or an allogeneic variant thereof. HNT22 can also be defined as a non-B, non-C, non-G hepatitis virus having a virus gene of the following (a) or (b):
(a) a non-B, non-C, non-G hepatitis virus gene having a nucleotide sequence from which a sequence having a length of from about 200 nucleotides to about 350 nucleotides can be amplified by PCR utilizing an oligonucleotide as shown in SEQ ID NO: 6 and an oligonucleotide as shown in SEQ ID NO : 8 as primers, or
(b) a non-B, non-C, non-G hepatitis virus gene having a nucleotide sequence from which a sequence having a length of from about 200 nucleotides to about 350 nucleotides can be amplified by PCR utilizing an oligonucleotide as shown in SEQ ID NO: 7 and an oligonucleotide as shown in SEQ ID NO: 8 as primers.

"TUS01" is a non-B, non-C, non-G hepatitis virus having a gene in as shown in SEQ ID NO: 62 or an allogeneic variant thereof.

Both HNT22 and TUS01 are as yet unknown viruses isolated from non-A, non-B, non-C, non-D, non-E, non-F, non-G hepatitis patients, and they were isolated from patients of hepatitis whose etiology has so far been considered unknown, and virologically or molecular biologically different from any known viruses.

HNT22 arid TUS01 can be generically defined as a virus having a gene comprising a nucleotide sequence of nucleotides 3-300 of SEQ ID NO: 1 or a nucleotide sequence having high homology thereto in its 5' end portion, and a nucleotide sequence of nucleotides 2902-3738 of SEQ ID NO: 1 or a nucleotide sequence having high homology thereto in its 3' end portion, and having a total length, including the 5' end and the 3' end nucleotide sequences, of about 3500 nucleotides to about 4000 nucleotides, preferably about 3600 nucleotides to about 3900 nucleotides.

In the present invention, hepatitis that is positive for HNT22 is called "HNT22 type hepatitis", and hepatitis that is positive for TUS01 is called "TUS01 1 type hepatitis".

It has been known that viruses are, in general, more likely to undergo mutations and that such mutations are more likely to be fixed in their genes as compared with other, higher organisms. Accordingly, a number of strains (allogeneic variants) may exist within the same species, and genotypes sharing a substantial gene sequence are also present. The terms HNT22 and TUS01 are used in the present invention as generic names of viruses including a possible great number of such variant strains and genotypes.

Consequently, the definition of a virus should be limited to include only specifically exemplified sequences, but also be extended to those having gene sequences or amino acid sequences in a range considered to be substantially homologous. The substantially homologous range can be defined based on a ratio of homology which enables clear differentiation of one virus from the other virus by comparing their gene sequences or amino acid sequences. The substantially homologous range can also be defined by referring to sequence diversity within a species of known similar viruses.

According to the aforementioned criteria, a gene having a nucleotide sequence which shares homology with those of the HNT22 genes or the TUS01 genes of 55% or more, preferably 60% or more, more preferably 70% or more, particularly preferably 80% or more may be considered to be substantially homologous to the gene of the present invention, and fall within the scope of the present invention.

When genetic conservation within the HNT22 genes and homology with other viruses were actually determined for the HNT22 genes of the present invention, the homology within the HNT22 genes (conservation) was 55% or more, and the highest homology with other viruses was less than 60%.

'The term 'homology' used hereinmeans homology of nucleotide sequences and amino acid sequences, and it specifically means a ratio of a number of conformed nucleotides or amino acids to a number of nucleotides or amino acids contained in the whole sequences expressed in terms of percentage when sequences to be compared are aligned so that they should show the best conformance with a necessary deletion.

The hepatitis C virus (HCV), which causes blood-borne infectious hepatitis like the virus of the present invention, exhibited a homology of 60% or more within the species. Further, it has been reported that HCV of the same genotype exhibited a homology of 80% or more except for the partial high mutation region. From these facts, it is considered to be appropriate that the range of the gene of the present invention should be defined in terms of the aforementioned homology.

Similarly, with regard to the homology of the encoded amino acid sequences, viruses sharing with those of the present invention a homology of 65% or more, preferably 70% or more, more preferably 80% or more, particularly preferably 90% or more are considered substantially homologous and are included among the viruses of the invention.

The genotypic variants of the HNT22 genes may be classified on the basis of positions 1939-2160 in SEQ ID NO: 1 (referred to as nt1939-2160 hereinafter): HNT22 genes as shown in SEQ ID NO: 45 (Genotype 1), SEQ ID NO: 46 (Genotype 2), SEQ ID NO: 47 (Genotype 3), SEQ ID NO: 48 (Genotype 4), SEQ ID NO: 49 (Genotype 5), SEQ ID NO: 50 (Genotype 6), and SEQ ID NO: 51 (Genotype 7) can be mentioned.

By utilizing these nucleotide sequences, HNT22 viruses of a specific genotype can be detected and distinguished. Suitable methods for differentiation include DNA amplification and sequencing, as shown in the Example hereinafter; the use of oligonucleotides having a sequence specific for any of the genotypes as a primer to amplify only a gene sequence of the corresponding genotypes; the use of such an oligonucleotide as probe to selectively detect the corresponding gene sequence, and a combination of these methods. Oligonucleotides and reaction conditions that can be used for these methods can be selected according to methods known to those skilled in the art. For example, partial sequences characteristic of the genotypes (referred to as genotype-specific sequences hereinafter) can easily be selected by comparing the genotype sequences with each another. It is easy for those skilled in the art to select an oligonucleotide having a genotype-specific sequence or a sequence complementary thereto, and not hybridizing other HNT22 genotypes, or to select a condition under which an oligonucleotide having a genotype-specific sequence substantially fails to hybridize with other genotype sequences (Protein, Nucleic acid, Enzyme, Front Line of PCR, 1996, Kyoritsu Shuppan). Oligonucleotides so selected can be utilized as a primer or a probe to perform HNT22 genotype-specific amplification or detection.

HNT22 genes can be identified as having a nucleotide sequence which can be amplified by PCR utilizing as primers the oligonucleotide pair of SEQ ID NO: 6 (NG059) and SEQ ID NO: 8 (NG063), and/or the oligonucleotide pair of SEQ ID NO: 7 (NG061) and SEQ ID NO: 8 (NG063).

In particular, the HNT22 genes shown in SEQ ID NO: 1 can be identified as having a nucleotide sequence which can be amplified by PCR utilizing as primers the oligonucleotide pair of SEQ ID NO: 2 (RD037) and SEQ ID NO: 3 (RD038) and/or the oligonucleotide pair of SEQ ID NO: 4 (RD051) and SEQ ID NO: 5 (RD052).

The HNT22 genes of the present invention were obtained by the present inventors according to the following procedure.

On the one hand, it has been recognized that there are patient cases clearly exhibiting hepatitis symptoms while they are negative for all known hepatitis virus markers. Such cases might be infection cases of unknown and non-elucidated hepatitis virus.

It is difficult to differentiate between non-infected, healthy subjects and asymptomatic carriers, whe are infected but do not (yet) suffer from the symptoms of hepatitis crisis. Therefore, the inventors focussed on the correlation of candidate genes and the onset of a hepatitis crisis.

Therefore, the present inventors performed screening of genes which were present in hepatitis cases of unknown cause but absent in subject that were either healthy persons or asymptomatics carriers in order to search for unknown hepatitis virus. The Representational Difference. Analysis (Science 259, 946-950, 1993 abbreviated as "RDA method" hereinafter) was used to identify suitable candidate genes. Determination of their sequences indicated that they were not homologous to sequences of known viruses.

Subsequently, a gene detection system which utilized an oligonucleotide probe specific for a part of these gene sequences was devised, and the genes were confirmed to be detected in other hepatitis cases for which the cause was not elucidated.

Further a large number of sequences from cases positive for the genes were analyzed and compared several genotypes were present among the viruses, and a method for differentiating the genotypes utilizing sequences specific for the genotypes was devised.

Furthermore, it was confirmed that the genes were not found in most healthy persons. It was also confirmed that among the cases positive for the genes there were some cases which were negative for the genes before blood transfusion, but infected due to blood transfusion with blood positive for the genes and maintained as positive thereafter.

It was also verified that the genes were not those derived from the hosts.

From analyses utilizing density gradient centrifugation, a fraction positive for the genes was localized at a specific density corresponding to virus particles.

It was also verified that the virus was a single-stranded DNA virus by analysis based on the presence or absence of a reverse transcriptase reaction step, analysis utilizing a deoxyribonuclease, and experiments with restriction endonucleases.

All of the above results confirmed that the genes were transmitted by blood transfusion and maintained in the carriers. Moreover, hepatitis symptoms were observed in infection cases, while positive persons were uncommon among the subjects without symptoms. The sequences of the genes were not homologous to those of any already known virus genes. Taken together, the present inventors concluded that the viruses contained as yet unknown hepatitis virus genes.

Based on their structures, it was assumed that the aforementioned were sequences only a part of a virus gene. The complete sequence of the genes of the present invention were obtained by the known gene walking method.

Embodiments of the present invention will be explained hereinafter by referring to the HNT22 genes, but it will be readily understood that the TUS01 genes can be used similarly.

The present invention provides an oligonucleotide or polynucleotide which has a sequence specific for the HNT22 genes, and can hybridize to the complement of the HNT22 genes under stringent conditions. Because of its characteristics, it can be effectively used as a primer for amplification of the HNT22 genes or as a probe for capturing or detecting the HNT22 genes in samples.

The term "specific sequence" used herein means a sequence which is present in a nucleotide sequence of a gene of interest or in an amino acid sequence deduced from the nucleotide sequence, and is undetectable in sequences other than the sequence of interest. Whether a sequence is specific can be determined based on homology of sequence which is determined in a conventional manner, for example, by searching databases. Specifically, a sequence is specific if it exhibits a homology of 10% or less in respect to known sequences other than the gene of interest having the same length. Alternatively, specificity may be decided by determining whether a gene of interest can be detected, identified and amplified by utilizing the sequence. Specifically, when an oligonucleotide or polynucleotide having the sequence of a portion of the gene (or complementary sequence thereof) is used as a primer in a method for detection or amplification of genes, if the oligonucleotide or polynucleotide can detect, identify or amplify a gene of interest in a manner distinguishable from other genes with statistical significance, the sequence should be deemed specific for the gene of interest.

As for amino acid sequences, whether a sequence is a specific sequence or not can similarly be determined based on sequence homology. Specifically, a specific amino acid sequence is an amino acid sequence having such a length that it should show homology of about 10% or less when compared with known unrelated sequences. Alternatively, when an antibody raised by using a peptide containing is consisting of an amino acid sequence encoded by the gene of interest as an antigen and verified to bind it, but not to bind or to bind to a weaker degree with statistical significance, to other antigens not derived from the amino acid sequence, the amino acid sequence can be a deemed specific.

The terms "polynucleotide" and "oligonucleotide" used for the present invention mean a nucleotide polymer having a certain length, and include ribonucleotides and deoxyribonucleotides, for example, single- and double-stranded DNA, and single- and double-stranded RNA. These terms include not only unmodified polynucleotides) but also polynucleotides and oligonucleotides having a modification by, for example, methylation, capping, enzyme labeling, fluorescence labeling and the like

The polynucleotides and the oligonucleotides of the present invention include not only those derived from genomic DNA, but also polynucleotides and oligonucleotides obtained by synthesis, replication, transcription or amplification according to conventional methods.

The polynucleotides of the present invention include the exemplified sequences, sequences substantially homologous thereto, and polynucleotides having a sequence complementary thereto.

The oligonucleotides and the polynucleotides of the present invention can be used as primers for amplification of the HNT22 genes by PCR. In this method, a portion of the HNT22 genes between the primers can be amplified, thereby confirming that the portion of the HNT22 genes, i.e., HNT22 is present in a tested sample.

The present invention also provides an oligonucleotide probe or polynucleotide probe useful for capturing the HNT22 genes by hybridization, and detecting the HNT22 genes.

A suitable probe used for the capture or detection can be selected from the gene sequences in the same manner as the selection of the primers mentioned above, and a selected probe can be labeled in a conventional manner. It is also possible to detect a gene by capturing with the probe mentioned above a gene amplified with the primers mentioned above, and in particular, it becomes possible to distinguish HNT genotypes by utilizing a probe comprising a genotype-specific sequence.

The oligonucleotide of the present invention is usually an oligonucleotide having a relatively short sequence continuously present in a polynucleotide, and includes those having the sequence in homologous or complementary polynucleotides. Its length is generally 6-50 nucleotides, preferably 10-30 nucleotides, more preferably 15-20 nucleotides.

Because the HNT22 genes show diversity as described above, a nucleotide sequence in a region where a nucleotide sequence is relatively well conserved among virus strains may be a nucleotide sequence specific for the genes. It is easy for those skilled in the art to design an oligonucleotide based on the nucleotide sequences disclosed herein. Specific examples of such an oligonucleotide include, for example, a polynucleotide as shown in SEQ ID NO: 2 (RD037), SEQ ID NO: 3 (RD038), SEQ ID NO: 4 (RD051), SEQ ID NO: 5 (RD052) SEQ ID NO: 6 (NG059), SEQ ID NO: 7 (NG061), and SEQ ID NO: 8 (NG063).

The present invention also provides a method for detecting the HNT22 genes wherein PCR is performed by utilizing the oligonucleotides of the present invention as primers. Specific examples of the method include, for example, the use of the following oligonucleotide pairs as primers. SEQ ID NO: 2 (RD037) and SEQ ID NO: 3 (RD038), or SEQ ID NO: 4 (RD051) and SEQ ID NO: 5 (RD052) (first detection method), or again SEQ ID NO: 6 (NG059) and SEQ ID NO: 8 (NG063), or SEQ ID NO: 7 (NG061) and SEQ ID NO: 8 (NG063) (second detection method).

Samples used for PCR are prepared by extracting nucleic acids from biological samples such as plasma or sera collected from specimens by a known method. This preparation can be performed using a commercially available extraction kit.

Conditions for PCR are suitably selected according to known PCR methods using a thermostable DNA polymerase.

Amplification products resulting from PCR can be detected by a known method such as electrophoresis, and the HNT22 genes can be detected based on the presence of the amplification products.

The present invention also provides a method for differentiating HNT22 genotypes, wherein both of the aforementioned first detection method and the second detection method are performed for one sample, and the results obtained are compared. A further method for differentiating HNT22 genotypes is by hybridization performed with an oligonucleotide having a sequence present in the genes of Genotypes 1-6 and specific for the corresponding genotype.

The sequence specific for each genotype can be selected by those skilled in the art based on nucleotide sequences disclosed herein, or based on nucleotide sequences of genes detected by the methods for detection of genes mentioned above. Hybridization can be performed according to known methods. Specifically, nucleic acids are prepared from a biological sample, the prepared nucleic acids and an oligonucleotide are hybridized under stringent conditions, and the hybridisation products containing the oligonucleotide are detected.

The specific oligonucleotides mentioned above are of course only examples, and other oligonucleotides which achieve the same object as mentioned above can be readily selected based on the HNT22 genes disclosed herein by utilizing techniques known to those skilled in the art. It is also easy to select an oligonucleotide which can be utilized for detecting both of the HNT22 genes and the TUS01 genes by combining the nucleotide sequences of both genes and utilizing techniques known to those skilled in the art. For example, an oligonucleotide as shown in SEQ ID NO: 57 (NG054) and an oligonucleotide as shown in SEQ ID NO: 60 (NG065), or an oligonucleotide as shown in SEQ ID NO: 57 (NG054) and an oligonucleotide as shown in SEQ ID NO: 61 (NG021) can be mentioned as such oligonucleotides, and by performing PCR using these as primers, the HNT22 genes and the TUS01 genes can be detected. Similarly, those skilled in the art will be readily able to select oligonucleotides which can be utilized for detecting only the HNT22 genes, or only the TUS01 genes.

The present invention further provides a polypeptide having an amino acid sequence encoded within an open reading frame found in nucleotide sequences of the genes of the present invention (referred to as 'the polypeptide of the present invention' hereinafter).

The term "polypeptide" herein means a linearly linked polymer of amino acids, and its length is not particularly limited. Accordingly, the polypeptide of the present invention includes any of those usually referred to as oligopeptides, proteins, and peptides, and as for their origin, it includes not only naturally occurring ones, but also fragments of the naturally occurring ones and those prepared by various means such as chemical syntheses and recombinant expression techniques. As described above, it has been known that viruses show differences of gene sequences even within the same species, and therefore similarly show differences in their amino acid sequences. The degree of homology of an amino acid sequence necessary for the sequence to be deemed as one of the viruses of the present invention is 65% or more, preferably 70% or more, more preferably 80% or more, and particularly preferably 90% or more.

The term "open reading frame" (abbreviated as "ORF" hereinafter) used for the present invention means a region of nucleotide sequence encoding a polypeptide or a part thereof. This sequence is transcribed and translated into a polypeptide when it is placed under suitable conditions. Boundaries of a coding sequence are an initiation codon at the 5' end and a termination codon at the 3' end.

The amino acid sequence encoded by a gene sequence may be determined in the art by defining the ORF of the gene sequence. The ORF can be determined as follows. A triplet nucleotide sequence which could be an initiation codon, where translation into an amino acid starts, should be identified in an obtained gene sequence. This location is assumed as an initiation codon, and an ORF encoding a polypeptide of a certain size is determined within a range where a termination codon does not appear. Examples of amino acid sequences which are obtained in this manner include the amino acid sequences shown in SEQ ID NOS: 9 and 10.

The polypeptide of the present invention preferably comprises an amino acid sequence specific for HNT22. An amino acid sequence specific for HNT22 can be selected by those skilled in the art based on amino acid sequences encoded by the nucleotide sequences disclosed herein, or amino acid sequences encoded by nucleotide sequences of genes detected by the aforementioned methods for detection of genes.

The polypeptide of the present invention more preferably contains an HNT22-specific epitope.

The term "epitope" used in the present invention means an antigenic determinant. The antigenic determinant is a site which is present in an antigen molecule and directly binds to an antibody, and it is constituted by at least three amino acids, usually 5-10 amino acids in the steric configuration. Methods for determining a location of epitope and its configuration in a steric structure of a peptide are known in the art, and can be performed by those skilled in the art. Accordingly, the term "HNT22-specific epitope" used in the present invention means an antigenic determinant specific for HNT22, and it is an epitope composed of an amino acid sequence specific for HNT22. This epitope is usually composed of eight or more contiguous amino acids.

The expression "containing an epitope" used in the present invention means that such an epitope sequence as defined above is contained as a part of polypeptides, and specific examples of such polypeptides containing an epitope include those composed of an epitope sequence bound to a carrier peptide or a linker peptide.

The polypeptide of the present invention can be effectively used as an antigen for antibody-based tests or an immunogen for preparing antiviral antibodies. It is expected that viral polypeptides include an amino acid sequence which does not have an amino acid sequence specific for the virus and may cause non-specific antibody reactions when this portion is used as an antigen. Therefore, it is also important in the present invention to specify a sequence from the whole amino acid sequence suitable for antibody-based tests. As a method for identifying such a specific sequence, i.e., an epitope sequence, there is available a method wherein peptides each having a certain length that cover the whole sequence are synthesized, and the presence or the absence or the extent of reaction of each peptide with an antiviral antibody, namely, a serum of a patient infected with the virus is utilized. Instead of the above method involving synthesis, a phage library using λgt11 can be prepared and screened with a blood serum of the patient.

The present invention also provides a method for isolating non-B, non-C, non-G hepatitis virus particles wherein the particles are isolated based on density of the particles, virus particles isolated by the aforementioned method, and a non-B, non-C, non-G hepatitis virus peptide obtained from the aforementioned virus particles.

The term "virus particles" used in the present invention means a fraction positive for the gene of the present invention which is collected as a fraction of a particular density by density gradient centrifugation, which is used as a usual method for isolation of virus particles, and it is not limited to those having a particle structure in a morphological sense, or infectious particles.

Viruses have densities corresponding to their particle structure, and they can be separated from other coexisting substances based on their density. The most usual method for isolating virus particles based on density is a method utilizing density gradient centrifugation. In this method, a density gradient carrier layer is formed in a centrifugation tube using sucrose or the like, then a fraction containing viruses is overlaid on the layer, and ultracentrifugation is performed. This technique is based on the fact that when viruses migrated by centrifugal force reach a layer having the same density as the viruses, the centrifugal force and buoyancy reach equilibrium and the viruses stop migration and thereby the viruses are concentrated in that fraction.

The polypeptide can be obtained from virus particles obtained by the method mentioned above by a known method. For example, virus particles are treated with a denaturation agent, and a polypeptide is separated from other components.

The present invention also provides a gene expression vector into which the HNT22 gene or a partial sequence thereof is integrated. The term "recombinant expression vector" used herein means a vector for inserting an exogenous polynucleotide into a gene of a host cell so that the polynucleotide can be expressed. Specifically, it is a vector having control sequences enabling the integration of the polynucleotide. The term "partial" means that the sequence is composed of a part of the genes which encodes a peptide sufficient for exhibiting antigenicity.

The expression vector can be effectively used for obtaining a viral peptide having immunological activity or biological activity. Various vectors for integrating a gene desired to be expressed are currently known, and various host cells for the integration of the vectors and consequent expression of peptides are also known.

The gene expression vector provided by the present invention is a recombinant expression vector having an HNT22 gene or ORF thereof, wherein the ORF is operably linked to regulatory sequences compatible with a desired host. A recombinant gene expression system can be constructed by using the expression vector.

The term "transformant cell" used herein means a cell in which all or a part of a gene of the present invention is integrated into a gene of that cell, and which cell can express all of or a part of a polypeptide encoded by the gene of the present invention.

The transformant cell provided by the present invention can be obtained by directly introducing a polynucleotide containing all or a part of the gene of the present invention, or introducing a recombinant expression vector integrated with such a polynucleotide into a host cell to transform the host cell. In the present invention, known transfer vectors and host cells can be used.

The polypeptide produced by the transformant cell (HNT22 virus antigen peptide) can be obtained by culturing the transformant cell under conditions under which the HNT22 virus antigen peptide can be expressed, and collecting the expressed polypeptide.

The present invention provides a method for immunologically detecting an HNT22 antibody by using HNT22 particles, an HNT22 polypeptide, an HNT22 epitope, or a polypeptide containing the HNT22 epitope as an antigen. Examples of methods for detection of antibodies using peptide antigens include immunonephelometry, enzyme immunoassay, radiometric immunoassay, agglutination method and the like, and these methods can be used for the present invention.

The present invention also provides a method for preparing an HNT22 antibody by utilizing HNT22 particles, an HNT22 polypeptide, an HNT22 epitope, or a polypeptide containing the HNT22 epitope, which is purified or partially purified, as an immunogen. Conventional methods can be used for preparing the antibody by utilizing purified or partially purified virus particles or polypeptides. The invention also contemplates such an antibody.

The present invention provides a method for immunoassay of an HNT22 antigen by utilizing an antibody specifically binding to HNT22 and a kit therefor. As an example of the method for assaying the antigen by using the antibody, there can be mentioned a method comprising:
(1) a step of reacting an antigen in a sample with an antibody capable of binding to HNT22 which antibodies are immobilized on a solid phase of a reaction vessels or reaction carrier, so that the antigen in the sample should be captured by the antibody bound to the solid phase through antigen-antibody interaction,
(2) a step of further reacting the captured antigen, after an appropriate washing step, with an appropriately labeled antibody capable of binding to HNT22 and having specificity for HNT22, and
(3) a step of detecting the binding antibody, after an appropriate washing step, by utilizing the function of the labeled antibody.

As another method, there can be mentioned a method comprising:
(1) a step of reacting a sample to be assayed with a defined concentration of a suitably labeled polypeptide having an HNT22 epitope for a certain period of time,
(2) a step of reacting the sample which has undergone step (1) with an HNT22 antibody immobilized on a solid phase of a reaction vessel or reaction carrier, so that the antigen in the sample should sufficiently bind to the antibody on the solid phase, and
(3) a step of separating labels bound to the reaction vessel or reaction carrier and unbound labels, and measuring the bound labels or the unbound labels.

Other methods utilized for assaying the viral antigen such as agglutination, reversed passive agglutination , enzyme immunoassay, radiometric immunoassay, and fluorescence polarization and the like can also be used in the present invention.

Purified HNT22 particles, polypeptides containing an epitope and obtained from the purified virus particles by recombinant expression or by chemical synthesis, or antigenic fragments thereof, may prove useful in the development of vaccines by known methods.

### FIGURES

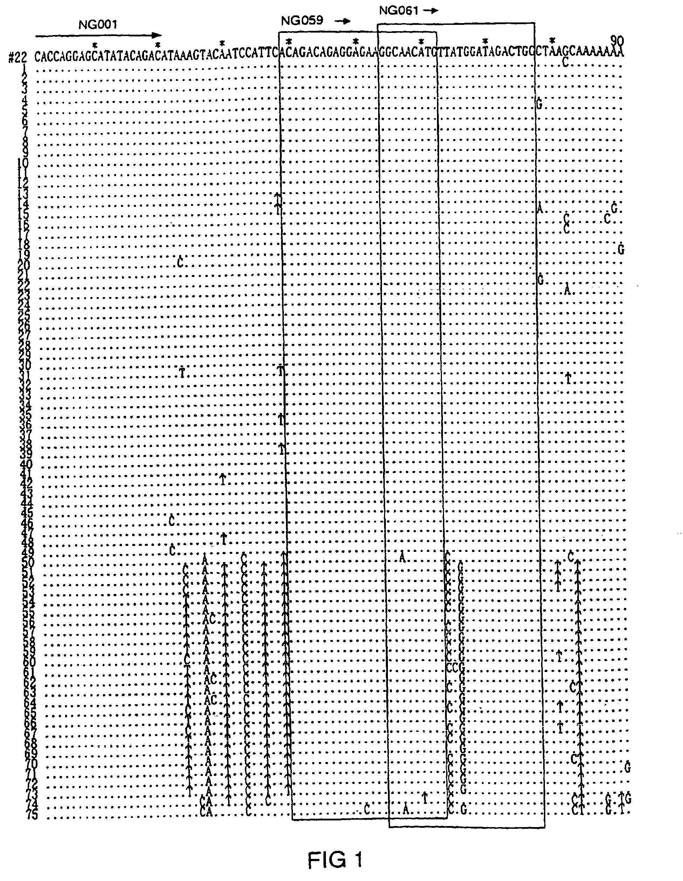

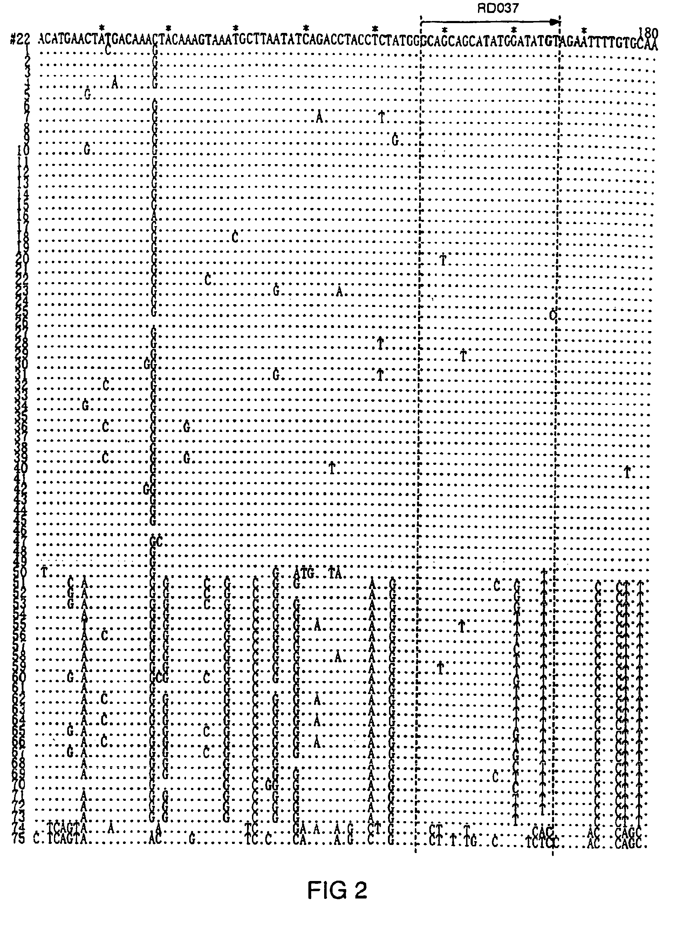

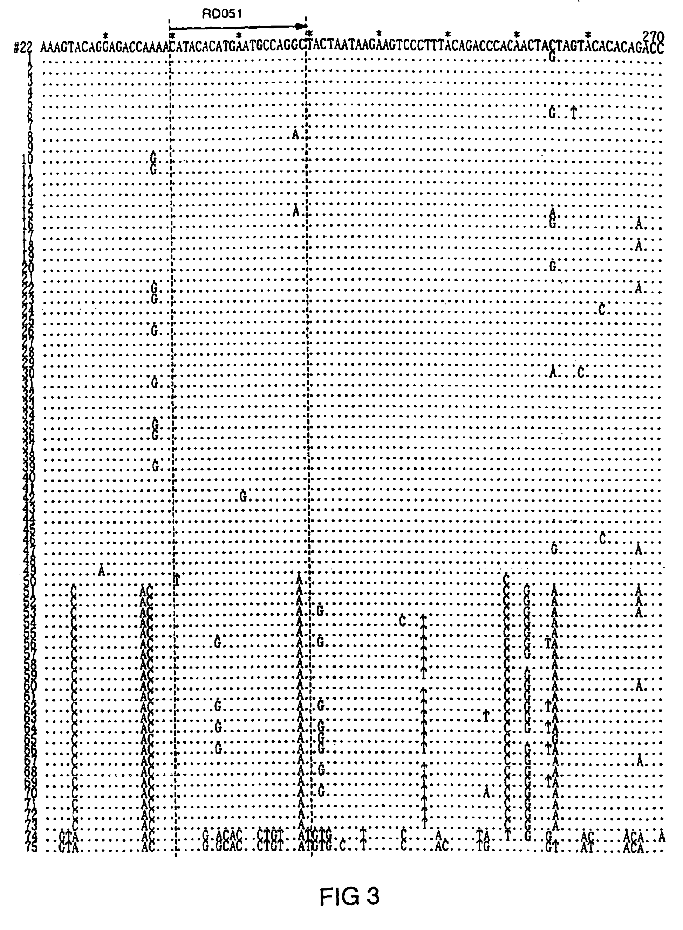

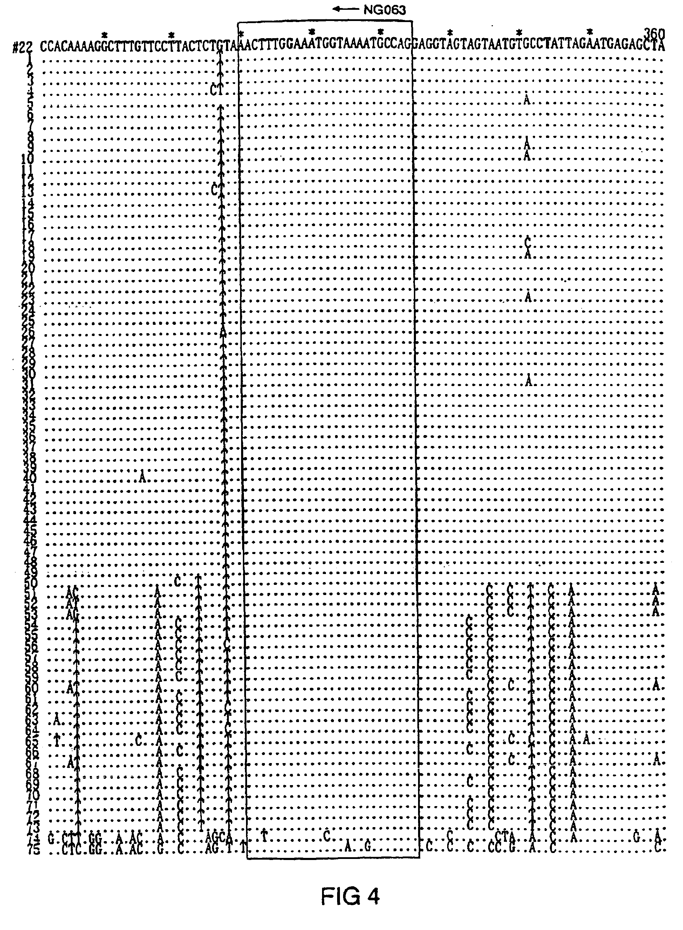

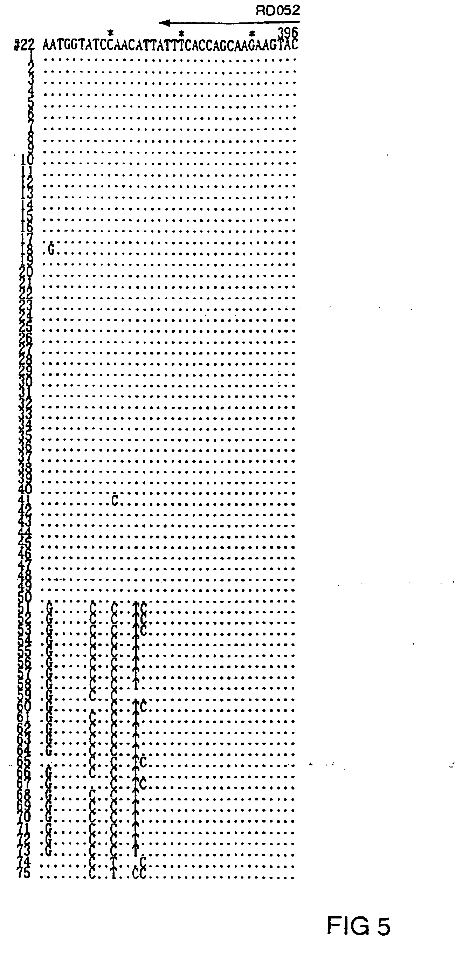

Figs. 1-5 show a comparison of the sequences of 396 nucleotides lying between NG001 (SEQ ID NO: 40) and a sequence complementary to RD052 (SEQ ID NO: 5) (corresponding to nt1 862-2257 in SEQ ID NO: 1) of the genes obtained from 75 HNT22 positive samples.
   The whole sequence of the corresponding region (nt78-299) of #22 (SEQ ID NO: 11) is shown at the top of the sequences of the samples, and thereunder nucleotides of Samples i-75 different from #22 are shown alphabetically while positions of matching nucleotides are shown with dots (.). Based on the homology of the sequences (corresponding to nt1939-2160 in SEQ ID NO: 1, nt78-299 in SEQ ID NO: 11), they can be classified into four groups, group of Samples 1-49 (designated as Genotype I), group of Samples 50-73 (designated as Genotype 11), Sample 74 and Sample 75.
   The locations of the primers used in Examples 2 and 3 are shown in the top row. It can be seen that mismatches are commonly present at the 3' end of the primers of RD037 (SEQ ID NO: 2) and RD051 1 (SEQ ID NO: 4) in Genotype II and Samples 74 and 75.
Fig. 6 represents extension of #22 clone sequence using gene walking (Example 4).
Fig. 7 represents a comparison of:
   (1) HNT22 gene sequence in blood for transfusion (SEQ ID NO: 11),
   (2) HNT22 gene sequence derived from a patient two weeks after blood transfusion, and
   (3) HNT22 gene sequence derived from a patient four weeks after blood transfusion for HNT22 infection cases caused by blood transfusion (Example 7). The compared sequences are completely consistent with one another.

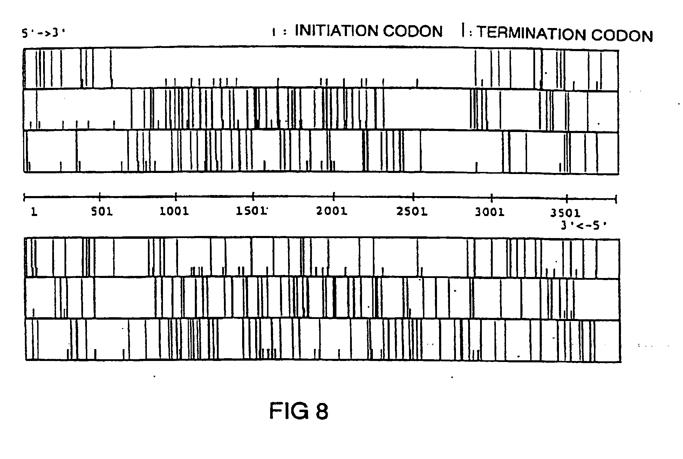

Fig. 8 represents the results of searching for open reading frames (ORF) of the HNT22 genes (Example 10).
   Upper 3 frames: candidate locations of initiation codons and termination codons in the nucleotide sequence shown in SEQ ID NO: 1. The short vertical bars indicate initiation codons, and the long vertical bars indicate termination codons. The three frames show sequences for each open reading frame, each open reading frame is shifted by one nucleotide for every sequence. Long ORFs were found in the first and the second frames.
   Lower 3 frames: candidate locations of initiation codons and termination codons in a sequence complementary to the nucleotide sequence shown in SEQ ID NO: 1. A long ORF was not recognized in any of the frames.

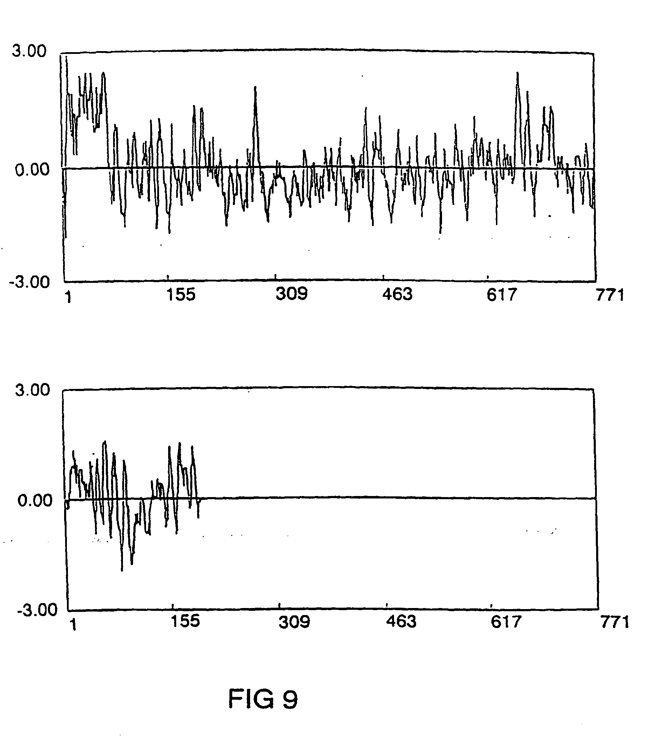

Fig. 9 represents hydrophilicity/hydrophobicity scores of polypeptides based on the amino acid sequences encoded by the open reading frames 1 and 2.

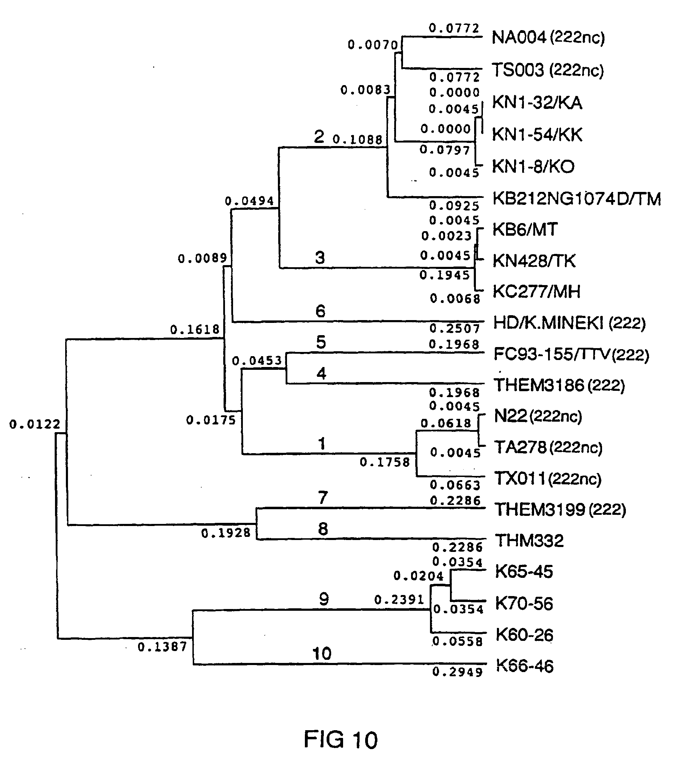

Fig. 10 represents a molecular phylogenetic tree based on the nucleotide sequences of the genes from HNT22 positive cases obtained in Example 12.

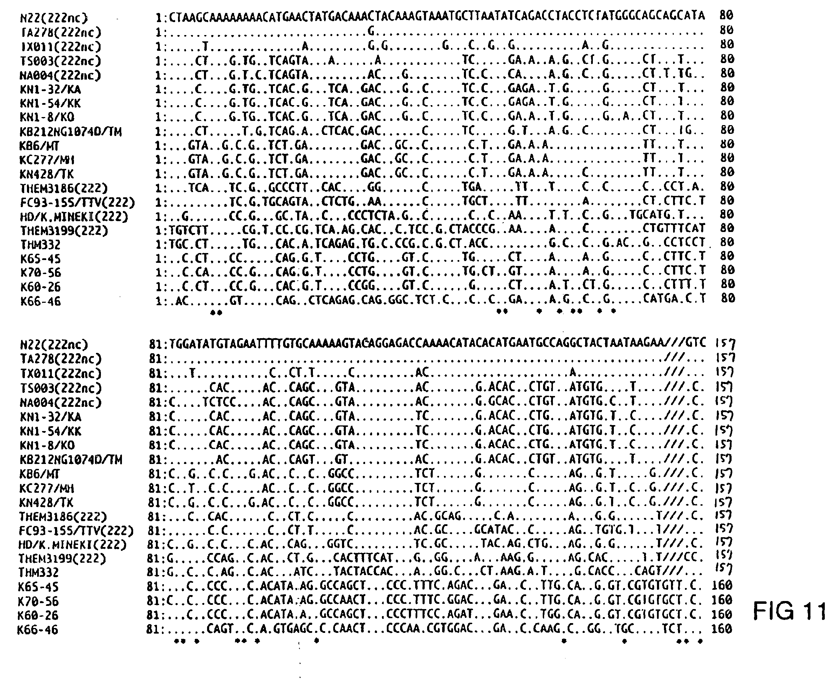

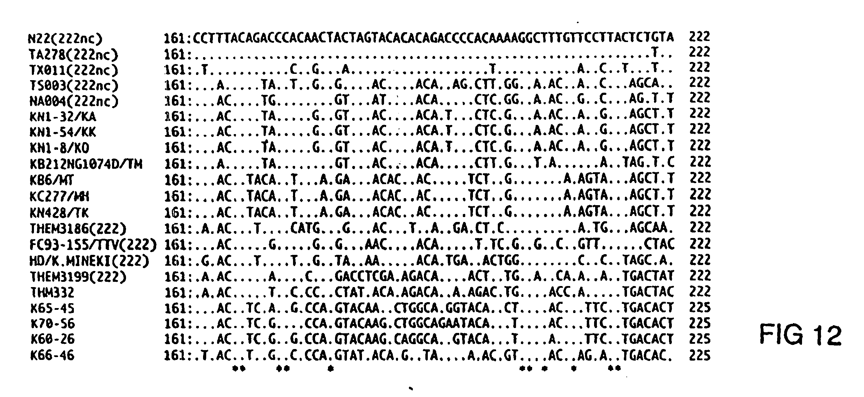

Figs. 11 and 12 represent comparison of nucleotide sequences of the genes from HNT22 positive cases obtained in Example 12.

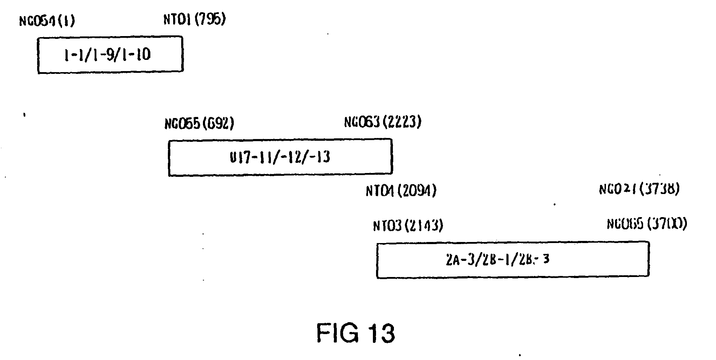

Fig. 13 represents the relative locations of the primers and the clones used for the sequencing of the full length TUSO1 gene. Names of the clones whose sequences were determined are indicated in the boxes. Names of the primers used for the amplification are indicated above the left and above right of the boxes, and nucleotide numbers, with the first nucleotide of the 5' end being defined as nucleotide 1, are indicated in parentheses.

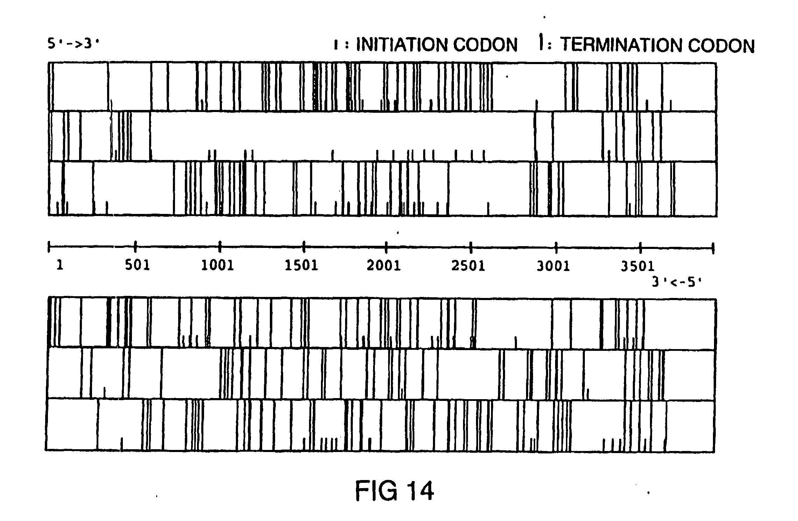

Fig. 14 represents the results of searching for open reading frame (ORF) of the TUSO1 gene.

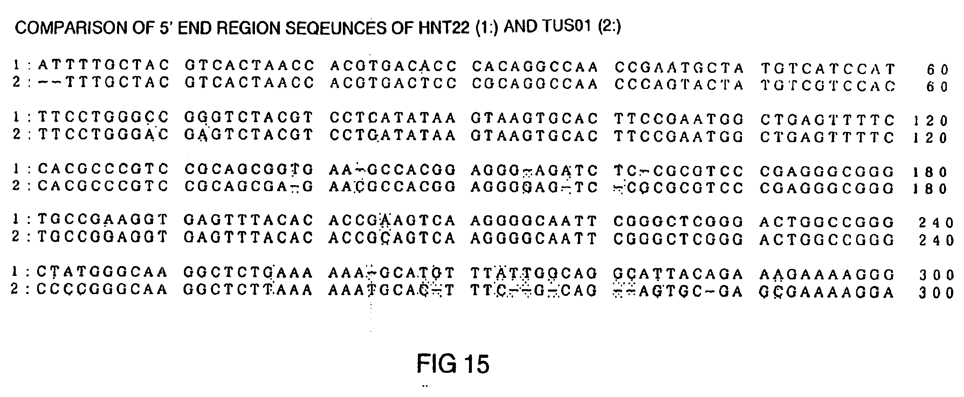

Fig. 15 represents a comparison of the 5' end region sequences of the HNT22 gene and the TUSO1 gene.

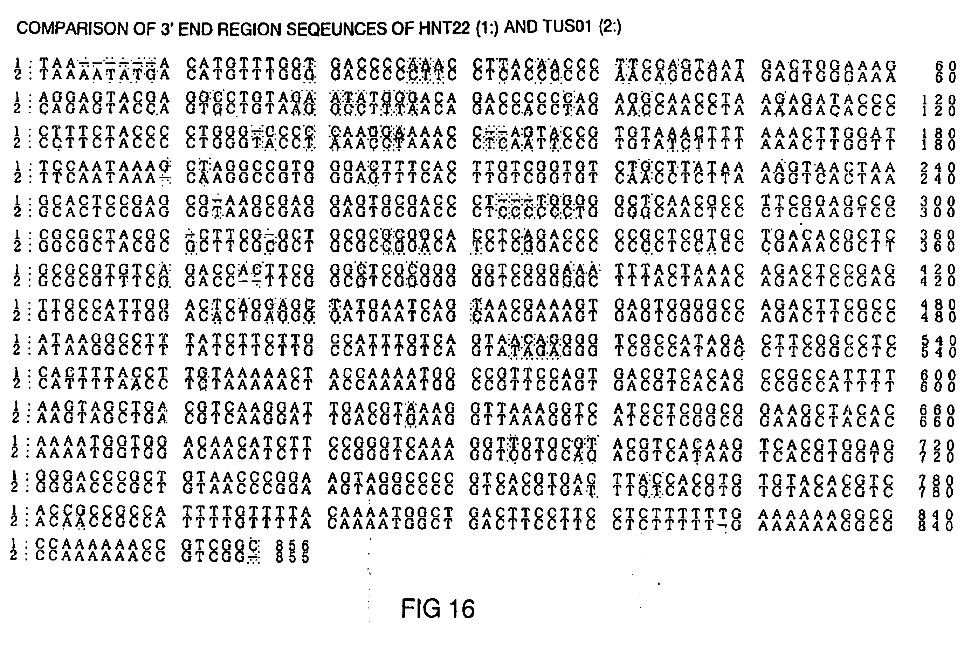

Fig. 16 represents a comparison of the 3' end region sequences of the HNT22 gene and the TUS01 gene.

### EXAMPLES

The present invention will be explained in detail hereinafter with reference to the following examples, but of course the present invention is not limited to these

### examples.

### Example 1: Isolation of Clone #22

For 3 cases clinically confirmed as post-transfusion non-A, non-B, non-C, non-D, non-G hepatitis (Cases 1-3), genes present only in blood samples of the patients after the appearance of symptons of post-transfusion hepatitis by the RDA method (subtraction) using blood of the patients upon or after the crisis of post-transfusion hepatitis and blood of the same patients before blood transfusion or before the appearance of symptons as samples. The RDA method is a method for detecting genes present in only one part of the comparison groups as described above, and it is a method for efficiently searching genes which differ between a tester which is a sample, and a driver which is a control.

In this example a sample taken after crisis of hepatitis of Case 2, of the above-mentioned three cases, which was clinically diagnosed as post-transfusion hepatitis whose cause was indistinct (see Example 6 for details) was used as a tester (A), and a sample of the same case before hepatitis crisis after blood transfusion (B1) and a sample of other acute hepatitis B patients (82) were used as drivers. Genes which had first appeared in the blood of the patient after crisis of post-transfusion hepatitis were discovered.

The procedure will be explained in detail hereinafter.

### (1) Extraction of nucleic acids

The above post-transfusion hepatitis case (Case 2) was negative for all known hepatitis virus markers. Its blood serum (mixture of 50 µl each of blood sera eight weeks and ten weeks after the blood transfusion of Case 2) was used as an unknown hepatitis virus positive tester (A).

As negative controls for the unknown virus, blood serum of the same patient from whom (A) was obtained before the crisis of hepatitis (two weeks after the blood infusion, B1), and serum of another patient with acute hepatitis B (B2) were used as drivers. First, nucleic acids were extracted from 100 µl each of the sera of the tester and drivers by using a commercially available nucleic acid extraction kit (ISOGEN-LS, Nippon Gene). That is, each serum (100 µl) and a nucleic acid extraction solution (300 µl) were transferred into a 1.5-ml Eppendorf tube, mixed by stirring for one minute, and left at room temperature for 5 minutes. After the mixture that remained on the wall of the tube was brought to the bottom of the tube by centrifugation, chloroform (80 µl) was added to the mixture, the mixture was stirred for one minute, and left at room temperature for 5 minutes. After the reaction was completed, the mixture was subjected to centrifugation for 15 minutes at 12000 revolutions/minute to afford a supernatant. The supernatant (210 µl) was transferred to another Eppendorf tube, glycogen (20 mg/ml, 1 µl, Boehringer Mannheim) and isopropanol (200 µl, Wako Pure Chemicals Industries) were added and then mixed. After the mixture was left to react at room temperature for 10 minutes, it was subjected to refrigerated centrifugation at 12000 revolutions/minute (4°C) for 10 minutes to afford a precipitate, which was then washed with 70% ethanol (Wako Pure Chemicals Industries) and dried in air.

### (2) Synthesis of cDNA

The nucleic acid obtained by the above procedure was dissolved in DEPC-water (10 µl, deionized water treated with diethyl pyrocarbonate, Sigma). A randomized hexamer solution (50 ng/µl, 1 µl) was added to this solution, warmed at 70°C for 5 minutes, and then promptly cooled on ice. After the cooling, a first strand buffer at 5-fold concentration (4 µl), 0.1 M DTT (2 µl), 10 mM dNTP (1 µl), RNase inhibitor (1 µl, 40 U/µl, RNasin, PROMEGA), and reverse transcriptase (1 µl, 200 U/µl, SuperScript II, GIBCO-BRL) were added to the solution, and allowed to react at 37°C for 60 minutes for synthesis of first strand cDNA. Then, to a tube containing the cDNA, DEPC-water (91 µl), a second strand buffer at 5-fold concentration (30 µl), 10 mM dNTP (3 µl), E. coli DNA ligase (1 µl, 10 U/µl), E. coli DNA polymerase (4 µl, 10U/µl), and E. coli RNaseH (1 µl, 20 U/µl) were added and mixed, and allowed to react at 16°C for two hours. Then, T4 DNA polymerase (2 µl, 5 U/µl) was added to the mixture and allowed to react at 16°C for 5 minutes to synthesize second strand cDNA. The reaction was stopped by adding EDTA (5 µl, 0.5 M). Subsequently, a mixture of phenol and chloroform (150 µl, 1:1) was added to the mixture in the tube, mixed, and subjected to centrifugation at 15000 revolutions/minute at room temperature for 5 minutes to precipitate proteins. Glycogen (1 µl, 20 mg/ml), 7.5 M ammonium acetate (78 µl, Wako Pure Chemicals Industries), and cold ethanol (562 µl, Wako Pure Chemicals Industries) was added to the obtained supernatant (156 µl), and immediately centrifuged at 15000 revolutions/minute at room temperature for 20 minutes to afford nucleic acids as precipitate. s The precipitates were washed with cold 70% ethanol (600 µl), dried in air, and then dissolved in TE buffer (50 µl, 10 mM Tris-HCl, pH 7.5, 1 mM EDTA). The solution was then subjected to gel filtration using a Microspin S-400 HR Column (Pharmacia). The obtained eluent (50 µl) was added with 1/10 volume of 3M sodium acetate (pH 5.2) and 2.5 volumes of ethanol, left to stand at -80°C for 20 minutes, and centrifuged at 15000 revolutions/minute at 4°C for 20 minutes to collect a cDNA/DNA fraction as a precipitate.

### (3) Sau3AI digestion

The cDNA/DNA fraction collected by the above procedure was dissolved in water (45 µl), H buffer at 10-fold concentration (5 µl, buffer suitable for the restriction endonuclease *Sau*3AI*,* Takara Shuzo) and *Sau*3AI (4 µl, 12 U/µl, Takara Shuzo) were added and mixed, and allowed to react at 37°C for 1.5 hours. After the reaction was completed, a mixture of phenol and chloroform (40 µl, 1:1) was added to the mixture, stirred, and subjected to centrifugation at 15000 revolutions/minute at room temperature for 15 minutes. 1/10 volume of 3 M ammonium acetate, pH 5.2 and 2.5 volumes of ethanol were added to the-supernatant (60µl), left to stand at -80°C for 20 minutes, and subjected to centrifugation at 15000 revolutions/minute at 4°C for 20 minutes to collect nucleic acids as precipitates.

### (4) Ligation of adapter

Adapters were introduced into the both ends of the fragments by ligating adapters R-Bam24 and R-Bam12 that were compatible with the nucleotide sequences of the ends cleaved with the restriction enzyme Sau3AI. That is, the nucleic acid fraction precipitates obtained by the above procedure were dissolved in water (16.1 µl), and 10-fold concentrated T4 ligase buffer (3 µl, NEB), the adapter R-Bam24 (6.0 µl, 10 OD/ml), and the adapter R-Bam12 (3.0 µl, 10 OD/ml) were added, then the ambient temperature was lowered from 50°C to 10°C during the course of one hour, and T4 ligase (1.5 µl, 400 U/µl, NEB) was added and allowed to react at 16°C overnight.

### (5) PCR amplification

Then, the gene fragments were treated with DNA polymerase to obtain gene fragments which were completely double-stranded over the full length, and these gene fragments were amplified by PCR using R-Bam24 as the primer as follows. The nucleic acid fraction into which the adapters were introduced by the above procedure was warmed at 70°C for 15 minutes to inactivate the T4 ligase present in the reaction mixture. Then, TaKaRa EX Taq Polymerase buffer at 10-fold concentration (20 µl, Takara Shuzo), 2.5 mM dNTP (24 µl), water (149.3 µl), and R-Bam24 (5.2 µl, 10 OD/ml) were added to the mixture, and allowed to react at 70°C for three minutes, and then TaKaRa EX Taq Polymerase (1 µl) was added to perform PCR with the following conditions. That is, after treatment at 72°C for 5 minutes, a cycle of 95°C for 1 minute and 72°C for 3 minutes was repeated for 30 cycles, followed by treatment at 72°C for 7 minutes and cooling to 4°C. After the completion of the reaction, nucleic acids were extracted with phenol and chloroform in the same manner as described above, and precipitated with ethanol. The precipitates of the PCR products obtained from eight tubes were combined and dissolved in TE buffer (100 µl), and subjected to gel filtration in the same manner as described above to afford an eluted solution, which was subjected to precipitation with ethanol.

### (6) Removal of adapter

The PCR products precipitated with ethanol after the gel filtration were dissolved in water (90 µl). To this solution was added H buffer at 10-fold concentration (10 µl) and then *Sau*3AI (7 µl), and allowed to react at 37°C for 15 minutes. Then, the nucleic acids were collected as precipitates in the same manner as described above, subjected to gel filtration using MicroSpin S-400 HR Column as described above, and precipitated with ethanol and collected. The product was dissolved again, and its absorbance at 260 nm was measured to confirm the yield.

### (7) Ligation of adapter (J-Bam24/J-Bam12) to tester

Then, the adapters J-Bam24 and J-Bam12 which are compatible with *Sau*3AI sequence were connected only to the tester amplified gene fragments as follows. That is, from the amplification products whose R-Bam24 and R-Bam12 tester were removed,T4 ligase buffer at 10-fold concentration (3 µl per 1.0 µg of the PCR products), and then the adapters J-Bam24 (13.2 µl) and J-Bam12 (6.6 µl) and water (4.9 µl) were added to those amplification products derived from the tester. The ambient temperature was lowered from 50°C to 10°C over the course of one hour to anneal them. To this reaction mixture, T4 DNA ligase (400 U/µl, NEB, 1.0 µl) was added, and allowed to react at 16°C overnight to ligate the adapters to the *Sau*3AI cleaved ends. Then, the mixture was treated at 70°C for ten minutes to inactivate the ligase. (8) Hybridization of gene fragments amplified from the tester withadapter and gene fragments amplified from the tester without adapter

The amplification products derived from the tester to which adapters were connected and the amplification products derived from the drivers from which adapters were removed by the procedure described were denatured by heat, thereby completely made into single-stranded DNA, and then re-annealed as follows. First, a mixture of phenol and chloroform (1:1, 30 µl) was added to the reaction mixture to remove proteins. To the obtained supernatant (17 µl), a driver gene amplification product whose adapters were removed (40 µg) was added, and precipitated with ethanol. The precipitates were added with EE buffer at 3-fold concentration (4 µl) and dissolved therein, and overlaid with mineral oil (30 µl, Sigma), and the DNA was denatured by a treatment at 98°C for ten minutes. After the denaturation, the mixture was immediately cooled on ice, and 5 M sodium chloride (Wako Pure Chemicals Industries, 1 µl) was added. Then, hybridization was performed at 67°C for about 22 hours.

During the above procedure, for genes commonly present in the tester and the drivers, like genes derived from human, original combinations of DNA, i.e., double-stranded DNA composed of re-associated two DNA strands derived from the tester, or two DNA strands derived from the driver (homo-double-stranded DNA), and double-stranded DNA composed of one DNA strand derived from the tester and one DNA strand derived from the driver (hetero-double-stranded DNA) were formed. In contrast, for DNA present only in the tester (considered to be an exogenous gene), hetero-double-stranded DNA was not formed and only homo-double-stranded DNA derived from the tester was formed because the corresponding allogeneic gene was not present in the driver. Further, only the double-stranded DNA having a gene sequence derived from the tester had the adapter sequences in both of the strands.

### (9) Amplification

Water (15 µl) was added to a sample which had undergone the hybridization of the above procedure, and then nucleic acids in the sample were precipitated with ethanol in the same manner as described above. To the precipitates was added 1/2 TE buffer (20 µl, 5 mM Tris-HCl, pH 7.5, 0.5 mM EDTA), and dissolved therein. To this solution (2 µl), Ex Taq Polymerase buffer at 10-fold concentration (20 µl), 2.5 mM dNTP (240 µl), water (147.7 µl), and TaKaRa EX Taq polymerase (1 µl) were added, and allowed to react at 72°C for 5 minutes. During this reaction, DNA was synthesized for the single strand portions which remained in the double-stranded DNA formed during the above operation, and termini were blunt-ended. To this sample was added J-Bam-24 (5.3 µl, 10 OD/ml) as a primer, and PCR was performed by 10 cycles of a cycle of 95°C for 1 minute and 70°C for 3 minutes, and leaving the mixture at 70°C for 7 minutes and then at 4°C. Nucleic acids were extracted from the amplified sample in the same manner as described above by using a mixture of phenol and chloroform, and precipitated with ethanol using glycogen as a coprecipitating agent. Under these conditions, utilizing the added J-Bam24 as a primer only the genes derived from the tester to which the J-Bam24 was ligated as an adapter can be amplified . As a result, DNA derived from the homo-double-stranded DNA of the tester specific genes can be amplified for the both DNA strands, and hence be amplified exponentially. On the other hand, the hetero-double-stranded DNA constituted by the genes common between the tester and the driver is amplified just multiplicatively only for the DNA strand having the driver gene sequence of which the tester is the template. The DNA strands derived from the homo-double-stranded DNA composed only of the driver genes cannot be amplified because the added primer cannot hybridize to them. Accordingly, the homo-double-stranded DNA having the tester specific gene sequences becomes predominant in the reaction mixture after the amplification.

### (10) Mung bean nuclease treatment

In order to obtain only tester specific genes, except for single strand driver gene sequence DNA present in the reaction mixture (a few double-stranded DNA derived from the driver will remain), mung bean nuclease (abbreviated as "MBN" hereinafter) treatment was performed as follows. TE buffer of a 1/2 concentration (10 µl) was added to nucleic acid fractions obtained by precipitatation from hybridization samples of the tester A and the driver B1, or the tester A and the driver B2 and the nucleic acid fractions dissolved therein. MBN buffer at 10-fold concentration (2 µl), water (13 µl), and MBN (0.5 µl, 300 U/µl, Takara Shuzo) was added to 5 µl of each solution and allowed to react at 37°C for 30 minutes. After the completion of the reaction, 50 mM Tris-HCl, pH 8.9 (80 µl) was added to the reaction mixtures and allowed to react at 95°C for 5 minutes to inactivate the nuclease. Aliquots of 5 µl and 10 µl taken from the above reaction mixtures, and the above hybridization sample not subjected to the nuclease treatment (1 µl). Ex Taq Polymerase buffer at 10-fold concentration (20 µl), 2.5 mM dNTP (24 µl), the primer J-Bam24 and TaKaRa Ex Taq DNA Polymerase (1 µl) was added to each aliquot and further water was added to give a total volume of 200 µl, and PCR was performed using the following conditions. Namely, a reaction cycle of 95°C for 1 minute and 70°C for 3 minutes was repeated 20 times, followed by treatment at 70°C for 7 minutes and cooling to 4°C. 10 µl was taken from the PCR products, and subjected to gel electrophoresis (1x TBE buffer) on 2.5% NuSieve 3:1 agarose (FMC BioProducts, USA). Since the results were the same for the combination of A and B 1 and the combination of A and B2, only the combination of A and B1 will be described hereinafter.

Nucleic acids were extracted from the remaining amplification products by the extraction method mentioned above using a mixture of phenol and chloroform, precipitated with ethanol, and collected. TE buffer (45 µl) was added to the precipitates and the precipitates dissolved therein. This solution was subjected to gel filtration using MicroSpin S-400 HR Column mentioned above, and the eluted solution was subjected to ethanol precipitation.

### (11) Removal of adapter

The precipitates obtained above were dissolved in water (63 µl), H buffer at 10-fold concentration (7 µl) and *Sau*3AI (6 µl, 12 U/µl) were added and allowed to react at 37°C for 1.5 hours to cleave the J-Bam24 and J-Bam12 adapter moieties. Proteins were removed from the reaction mixture by using a mixture of phenol and chloroform as described above, and nucleic acids were precipitated with ethanol. The precipitates were dissolved in TE buffer (90 µl), and subjected to gel filtration using MicroSpin S-400 HR Column to remove the cleaved adapters. The eluted solution, which contained the amplification products whose adapters were removed, was precipitated with ethanol, and the precipitates were dissolved in TE buffer at 1/2 concentration (10 µl, 5 mM Tris-HCl, pH 7.5, 0.5 mM EDTA). Absorbance at 260 nm of this solution was measured to determine the yield. It was thought that tester specific genes were contained in this fraction.

### (12) Re-subtraction

T4 DNA ligase buffer at 10-fold concentration (3 µl), adapters N-Bam24 (13.2 µl), and N-Baml2 (6.6 µl) were added to the candidate DNA fraction (1 µg) of the tester specific genes obtained in the above procedure and made up to a total volume of 28.5 µl, and then its temperature was lowered from 50°C to 10°C over the course of one hour. Then, T4 DNA ligase (1.5 µl, 400 U/µl) was added to the mixture and allowed to react at 16°C overnight to ligate the adapters. Then, the mixture was treated at 70°C for 10 minutes to inactivate the T4 DNA ligase, and after removing proteins in a conventional manner using a mixture of phenol and chloroform, subjected to precipitation with ethanol. Driver B1 DNA (40 µg) was added to the supernatant (17 µl, about 0.5 µg DNA), mixed, and then precipitated with ethanol. TE buffer at 3-fold concentration (4 µl) was added to the precipitates, the precipitates dissolved therein, overlaid with mineral oil (30 µl, Sigma), and heat-treated at 98°C for one minute to denature the DNA. After the denaturation, the mixture was immediately cooled on ice, and 5 M sodium chloride (1 µl, Wako Pure Chemicals industries) was added to perform hybridization at 67°C for about 21 hours. After the hybridization, nucleic acids were precipitated from the sample with ethanol in the same manner as described above. TE buffer of 1/2 concentration (20 µl) was added to the precipitates and the precipitates dissolved therein, and to 2 µl of this solution EX Taq DNA polymerase buffer at 10-fold concentration (20 µl), 2.5 mM dNTP (240 µl), water (147 µl), and TaKaRa Ex Taq DNA polymerase (1 µl) were added, and allowed to react at 72°C for 5 minutes so make the nucleic acid blunt-ended. N-Bam24 (5.3 µl, 10 OD/ml) as a primer, and PCR was performed with the condition that a cycle of 95°C for 3 minute and 70°C for 3 minutes was repeated for 10 cycles, and the mixture was left at 70°C for 7 minutes and then at 4°C. Nucleic acids were extracted from the amplified sample in the same manner as described above using a mixture of phenol and chloroform, addition of glycogen, and precipitation with ethanol. TE buffer at ½ concentration (10 µl) was added to this nucleic acid fraction and the nucleic acid fraction dissolved therein. 1 µl was taken from the solution, and to this TaKaRa Ex Taq DNA polymerase at 10-fold concentration (1 µl) was added and PCR was performed under the conditions of a cycle of 95°C for 1 minute and 70°C for 3 minutes was repeated for 20 cycles, followed by treatment at 70°C for 7 minutes and cooling to 4°C. The above procedure was used to further screen the tester specific genes. Again, the adapters were removed again from the DNA fraction obtained from this procedure, and the same procedure was repeated once more utilizing J-Bam24 and J-Bam12 as adapters to afford final candidates for the tester specific gene, Clone #22.

### (13) Isolation of Clone #22

The nucleotide sequences of the tester specific gene candidates obtained by the above procedure were determined as follows to isolate novel viral gene Clone #22. The #22 has *Sau*3AI cleaved sequences at the both ends. By utilizing these sequences, it was cloned into pT7BlueT vector (Navagen). This clone was transfected into E. coli TG-1, and transformant cells were screened. The plasmid DNA of the obtained transformants were analyzed. That is, for 60 clones in total, plasmid DNAs were prepared, and their nucleotide sequences were determined in the forwards direction and the reverse direction by using the Thermo Sequencer Fluorescent-labelled primer cycle sequencing kit (Amersham International plc. Buckinghamshire, England). Based on these sequences, the clones were classified. As a result, 13 clones having the same nucleotide sequence were obtained. A consensus sequence was searched for by aligning the sequences of these clones, and the sequence shown in SEQ ID NO: 11 was obtained. The clone having this sequence was designated as Clone #22. The full length of Clone #22 was 500-nucleotides long.

### Example 2: HNT22 gene detection method (1)

A plurality of oligonucleotides of 20-nucleotide length constituting the nucleotide sequence of HNT22 Clone #22 obtained in Example 1 were synthesized , and various combinations thereof were examined for their utility as primers for gene amplification. That is, PCR was performed for samples which were positive for the genes and from which the genes were isolated in order to search for sequences and combinations thereof capable of efficiently amplifying the genes. As a result, it was found that oligonucleotides having the sequences shown in SEQ ID NO: 2 (primer name: RD037) and SEQ ID NO: 3 (primer name: RD038) as well as oligonucleotides having the sequences shown in SEQ ID NO: 4 (RD051) and SEQ ID NO: 5 (RD052) could be effectively used as primers for gene amplification. RD037 and RD051 are sense primers, and RD038 and RD052 are anti-sense primers. Amplification is performed by using a pair of sense primer and anti-sense primer.

The details of the method used for detecting genes are as follows.

Nucleic acids were extracted from blood serum or plasma (100 µl) using a commercially available nucleic acid extraction kit (EX R&D, Sumitomo Metal Industries). Because it had been revealed that HNT22 virus is a DNA virus as described hereinafter (Example 8), viral genes were treated as DNA in this procedure. The extracted DNA was dissolved in TE buffer (10 µl), and the whole volume was used as a sample. AmpliTaq DNA polymerase buffer at 10-fold concentration (5.0 µl, Perkin Elmer), 10 mM dNTP (1.0 µl), primers RD037 and RD038 (0.5 µl for each, 10 OD/ml), and thermostable DNA polymerase (0.25 µl, AmpliTaq DNA Polymerase, Perkin Elmer) were introduced into a tube used exclusively for PCR, and distilled water was added to give a total volume of 50 µl. This reaction mixture was prepared upon each use.

To the tube containing the reaction mixture, the above extracted DNA sample (10 µl) was added, overlaid with mineral oil (50 µl), stirred, and then centrifuged in a refrigerated centrifugal separator at 6000 rpm for 30 seconds. After the centrifugation, the tube was mounted on a thermal cycler, and PCR was performed. PCR was performed with a treatment at 95°C for 2 minutes and 30 seconds, then a cycle of 94°C for 30 seconds, 55°C for 30 seconds, and 72°C for 45 seconds for 35 cycles, and a treatment at 72°C for 7 minutes after the last cycle.

When further amplification was performed, AmpliTaq DNA polymerase buffer at 10-fold concentration (5.0 µl, Perkin Elmer), 10 mM dNTP (1.0 µl), primers RD051 and RD052 (0.5 µl for each, 10 OD/ml) that were selected from a nucleotide sequence located more internal than the aforementioned RD037 and RD038, and thermostable DNA polymerase (0.25 µl, AmpliTaq DNA Polymerase, Perkin Elmer) were introduced into another tube used exclusively for PCR, and distilled water (37.5 µl) was added to give a total volume of about 45 µl. To this mixture, the aforementioned amplification product (5 µl) was added to afford a reaction mixture. This reaction mixture was prepared upon each use.

After the preparation of the reaction mixture, the mixture was overlaid with mineral oil (50 µl), mixed, and then centrifuged in a refrigerated centrifugal separator at 6000 rpm for 30 seconds. Then, the tube was mounted on a thermal cycler, and PCR was performed. PCR was performed with a cycle of 94°C for 30 seconds, 55°C for 30 seconds, and 72°C for 30 seconds for 25 cycles, and a treatment at 72°C for 7 minutes after the last cycle.

The amplified genes were detected by electrophoresis. That is, 10 µl of the amplification product obtained by the amplification with the above condition were taken, and subjected to agarose gel electrophoresis (2.5% NuSieve: Agarose EP = 3:1). After the electrophoresis, the agarose gel was stained with ethidium bromide, and presence or absence of the amplification product was confirmed under ultraviolet light. The amplification product could be detected as a 270 bp band when only the primers RD037 and RD038 were used, or as a 197 bp band when the amplification was performed using the primers RD051 and RD052 once or with additional amplification.

### Example 3: HNT22 gene detection method (2)

While HNT22 viral infection cases in Japanese patients could be sufficiently detected by the HNT22 gene detection method of Example 2, in order to further examine conservation of the amplified region, sequences within the range containing the region of a large number of samples were amplified by the method described hereinafter in Example 7 using primers NG001/RD038 (1st PCR) and NG001/RD052 (2nd PCR), and examined. As a result, it was found that a plurality of genotypes including the two major Genotypes I and II (designations decided by the present inventors as described in Example 12 hereinafter) exist for HNT22 virus. It was further found that mismatches are scattered in the sequences of the primers RD037 and RD051, in particular at the 3' end, in the genotype designated as Genotype II (Fig. 1 to Fig. 5). Based on an assumption that more specific and more sensitive gene detection methods can be developed by avoiding this mismatch sequence, further highly conserved sequences were searched for. As a result, it was found that oligonucleotides having the sequences shown in SEQ ID NO: 6 (primer name: NG059), SEQ ID NO: 7 (NG061), and SEQ ID NO: 8 (NG063) can be utilized as primers for gene amplification (Fig. 1 to Fig. 5), and novel HNT22 gene detection methods utilizing them have been completed.

The details of the method used for detecting genes are as follows.

DNA was extracted from blood serum or plasma (50 µl) in the same manner as in Example 2. The extracted DNA was dissolved in distilled water (20 µl), treated at 95°C for 15 minutes, and immediately cooled on ice for 2 minutes to afford a sample for measurement. AmpliTaq DNA polymerase buffer at 10-fold concentration (5.0 µl, Perkin Elmer), 2.5 mM dNTP (4 µl), primers NG059 and NG063 (0.5 µl for each, 10 OD/ml), and thermostable DNA polymerase (0.25 µl, AmpliTaq DNA Polymerase, Perkin Elmer) were introduced into a tube used exclusively for PCR, and further distilled water (29.75 µl) was added to give a total volume of 40 µl. The extracted DNA sample (10 µl) was added to the tube containing the reaction mixture, overlaid with mineral oil (50 µl), stirred, and then centrifuged in a refrigerated centrifugal separator at 6000 rpm for 30 seconds. After the centrifugation, the tube was mounted on a thermal cycler, and PCR was performed. PCR was performed with a cycle of 94°C for 30 seconds, 60°C. for 45 seconds, and 72°C. for 45 seconds for 35 cycles, and finished with a reaction at 72°C for 7 minutes after the last cycle.

When further amplification was performed, AmpliTaq DNA polymerase buffer at 10-fold concentration (5.0 µl, Perkin Elmer), 2.5 mM dNTP (4 µl), primers NG061 and NG063 (0.5 µl for each, 10 OD/ml), and thermostable DNA polymerase (0.25 µl, AmpliTaq DNA Polymerase, Perkin Elmer) were introduced into another tube used exclusively for PCR, and further added with distilled water (37.75 µl) to give a total volume of 48 µl. To this mixture, the aforementioned amplification sample (2 µl) was added, and the mixture was overlaid with mineral oil (50 µl), stirred, and then centrifuged in a refrigerated centrifugal separator at 6000 rpm for 30 seconds. Then, the tube was mounted on a thermal cycler, and PCR was performed. PCR was performed with a cycle of 94°C for 30 seconds, 60°C for 45 seconds, and 72°C for 45 seconds for 25 cycles, and finished with a reaction at 72°C for 7 minutes after the last cycle. The amplified genes were detected by agarose gel electrophoresis in the same manner as in Example 2. In this example, the HNT genes could be detected as a 286 bp band when amplified by PCR using the first primers NG059 and NG063, or as a 271 bp band when amplified by PCR using the primers NG061 and NG063.

Each PCR constituting the two-step PCR performed in this example may of course be performed alone.

Further, because the gene detection method of Example 3 can be utilized for amplification of many different genes of the HNT22 gene including Genotypes I and II, and amplification specific for Genotype 1 can be realized by the gene amplification method of Example 2, it is possible to classify samples for their genotypes by subjecting each sample to gene detection according to Example 2 and Example 3, and examining resulting detection patterns (Fig. 1 to Fig. 5).

### Example 4: Extension of identified gene sequence

An identified gene sequence was extended based on Clone #22. That is, by using a sample (serum) of a blood donor exhibiting abnormality of hepatic function (34 years old, male, ALT [alanine aminotransferase] value: 106 IU), which sample showed high HNT22 virus titer (HNT22 genes could be detected even in 10⁴⁻⁵-fold dilution) as determined by the two-step PCR consisting of a combination of PCR utilizing the primer pair of RD037 and RD038 and PCR utilizing the primer pair of RD051 and RD052, and the two-step PCR consisting of a combination of PCR utilizing the primer pair of NG059 and NG063 and PCR utilizing the primer pair of NG061 and NG063, which were established in Examples 2 and 3, the #22 sequence was extended in the 5' direction and the 3' direction by the walking technique. The virus strain having the obtained nucleotide sequence (SEQ ID NO: 1) was designated as T278.

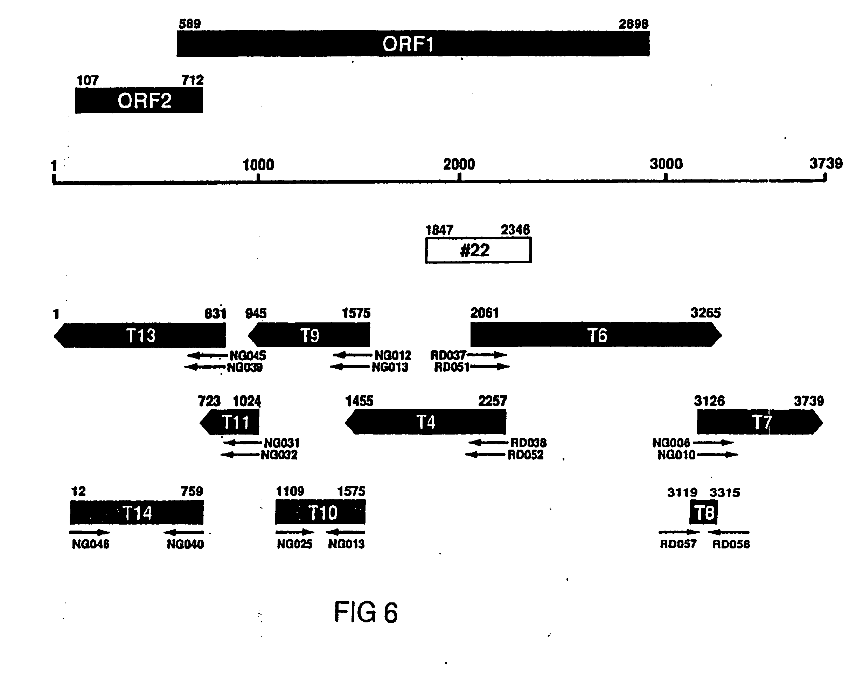

The details of the walking method performed in this example were as follows (Fig. 6).

### (1) Sequencing of Clones T4 and T6

Two-step single-sided PCR was performed using sense and anti-sense primers specific for the nucleotide sequence of Clone #22 and non-specific primers which had nucleotide sequences of 41-nucleotides long shown in SEQ ID NOS: 12-15 (SSP-G, SSP-A, SSP-T and SSP-C).

For the extension of the 5' end, two-step single-sided PCR utilizing a combination of either of specific anti-sense primer RD038 or RD052 and one of the above-mentioned non-specific primers was performed. In the PCR of the first step (1st PCR), a cycle of 94°C for 30 seconds, 42°C for 45 seconds, and 72°C for 2 minutes was repeated 5 times, the reaction products were purified by SizeSep-400 Column (Pharmacia Biotechnology), and a cycle of 94°C for 30 seconds, 55C for 45 seconds, and 72°C for 2 minutes was repeated 35 times for amplification. In the PCR of the second step (2nd PCR), 1/10 volume of the product of the 1st PCR was used, and a cycle of 94°C for 30 seconds, 55°C for 30 seconds, and 72°C for 2 minutes was repeated 30 times. PCR was performed by using TaKaRa Ex Taq DNA Polymerase (Takara Shuzo). The product was cloned into pT7Blue T vector in the same manner as in Example 1, and nucleotide sequences of the both strands were determined for three or more clones to obtain the sequence shown in SEQ ID NO: 16 (corresponding to nt1455-2257 shown in SEQ ID NO: 1, Clone T4), and the sequence shown in SEQ ID NO: 17 (corresponding to nt2061-3265 of SEQ ID NO: 1, Clone T6). The sequence shown in SEQ ID NO: 16 was obtained from the amplification product of PCR utilizing the combination of the primers SSP-A and RD052, and the sequence shown in SEQ ID NO: 17 was obtained from the amplification product of PCR utilizing the combination of the primers RD051 and SSP-C.

### (2) Sequencing of Clones T7, T9, T11, and T13

Using the newly identified Clones T6 and T4, the 5' nucleotide sequence was identified from Clone T4 and the 3' nucleotide sequence from Clone T6 in the same manner as (1), above. That is, for the 5' sequence, two-step single-sided PCR was performed using specific anti-sense primers which have the sequences of SEQ 1D NOS: 18 and 19 having a sequence specific for the 5' end of Clone T4 (NG012 and NG013) and non-specific primers which have the nucleotide sequences of SEQ ID NOS: 12-15 and 20, the resultant amplification products were cloned, and sequenced by the method described in Example 1. Specifically, PCR of the first step was performed using a combination of the anti-sense primer NG012 and any one of the aforementioned non-specific primers. For PCR, a cycle of 94°C. for 30 seconds, 55°C for 30 seconds, and 72°C for 2 minutes was repeated 5 times, the reaction products were purified by SizeSep-400 Column (Pharmacia Biotechnology), and a reaction cycle of 94°C for 30 seconds, 55°C for 45 seconds, and 72°C for 2 minutes was further repeated 35 times. The PCR of the second step was performed using 1/10 volume of the product of the PCR of the first step and NG013 as the specific anti-sense primer, and repeating a cycle of 94°C for 30 seconds, 55°C for 30 seconds, and 72°C for 2 minutes 30 times. The amplification product was cloned into pT7Blue T vector in the same manner as in Example 1, and nucleotide sequences of both strands were determined for three or more clones to obtain the sequence shown in SEQ ID NO: 21 (corresponding to nt945-1575 in SEQ ID NO: 1, Clone T9) (Fig. 6).

For the 3' sequence, sequence amplification by utilizing two-step single-sided PCR similar to the above one using specific sense primers NG006 and NG010 which have the sequences of SEQ ID NOS: 22 and 23 having a sequence specific for the 3' end of Clone T6 and non-specific primers which have the nucleotide sequences of SEQ ID NOS: 12-15 and 20, cloning into pT7blue T vector, and sequence analyses were performed to afford the sequence shown in SEQ ID NO: 24 (corresponding to nt3126-3739 in SEQ ID NO: 1, Clone T7) (Fig. 6).

For the 5' sequence, the same procedure was further repeated to obtain the sequence shown in SEQ ID NO: 25 (corresponding to nt723-1024 of SEQ ID NO: 1, Clone T11) by using the sequence of the aforementioned Clone T9, and the sequence shown in SEQ ID NO: 26 (corresponding to nt1-831 shown in SEQ ID NO: 1, Clone T13) by using the sequence of Clone T11. By aligning the sequences of these clones, the sequence of HNT22 shown in SEQ ID NO: 1 was obtained. In order to obtain Clone T11, an oligonucleotide as shown in SEQ ID NO: 27 (primer name: NG031) was used as an as anti-sense primer for the single-sided PCR of the first step, and an oligonucleotide as shown in SEQ ID NO: 28 (primer name: NG032) was used as an as anti-sense primer for the single-sided PCR of the second step. In order to obtain Clone T13, an oligonucleotide as shown in SEQ ID NO: 29 (primer name: NG045) was used as an as anti-sense primer for the single-sided PCR of the first step, and an oligonucleotide as shown in SEQ ID NO: 30 (primer name: NG039) was used as an as anti-sense primer for the single-sided PCR of the second step. As non-specific primers, the nucleotide sequences of SEQ ID NOS: 12-15 and 20 were used as above (Fig. 6).

In order to establish the sequences, the sequences corresponding to nt1 109-1575 (SEQ ID NO: 31, Clone T10), nt12-759 (SEQ ID NO: 32, Clone T14), and nt3119-331 S (SEQ ID NO: 33, Clone T8) were amplified by using oligonucleotides as shown in SEQ ID NO: 34 (primer name: NG013) and the nucleotide sequence shown in SEQ ID NO: 35 (primer name: NG025), oligonucleotides as shown in SEQ ID NO: 36 (primer name: NG040) and the nucleotide sequence shown in SEQ ID NO: 37 (primer name: NG046), and oligonucleotides as shown in SEQ ID NO: 38 (primer name: RD057) and the nucleotide sequence shown in SEQ ID NO: 39 (primer name: RD058) as the primer pair, respectively, amplification products were cloned as above, and the sequences were analyzed (Fig. 6).

The sequences of the obtained clones, were combined to afford the sequence of the full length 3739 bp shown in SEQ ID NO: 1. Upon constructing the sequence, when mutations were observed in sequences which overlapped between clones (different among clones), preference was given to the most frequent nucleotide.

For the sequence of nt1455-3054 in the obtained sequence, known gene sequences were searched for based on homology by using databases (DDBJ, National Genetics Institute, search programs: BLAST and FASTA). Among the 20 genes exhibiting the highest homology, only Simian cytomegalovirus major immediate early transcription unit IE94 was derived from viruses, and its homology was 50.7% even for the 383-nucleotide long fragment which exhibited the highest homology.

From the above, it was confirmed that any viral genes exhibiting high homology to the gene sequences of the present invention over the full length have not been known, and the genes found by according to the present invention have novel sequences.

### Example 5: Detection of HNT22 genes in blood donors who were negative for non-B, non-C, non-G hepatitis virus markers and exhibited abnormal alanine aminotransferase values and chronic hepatitis patients

For 207 persons who were negative for known hepatitis virus markers and exhibited activity of alanine transferase (ALT) of 100 international unit (IU/1) or more, 26 persons who exhibited activity of γ-glutamyl transpeptidase (γ-GTP) of 500 IU/1 or more, and 15 cases of non-B, non-C type chronic hepatitis all found in Japanese blood donors, the presence of the HNT22 genes was examined by using the gene detection method of Example 2.

As controls, 88 blood donors with normal hepatic function and 22 patients of chronic hepatitis C were also examined.

As a result, the HNT22 genes were found in 16 persons out of the 207 persons with abnormally high ALT values (7.7%), 4 persons out of the 26 persons with abnormally high γ-GTP values (15.4%), and 3 patients out of the 15 non-B, non-C type chronic hepatitis patients (20%).

On the other hand, the HNT22 genes were found in 3 persons out of the 88 blood donors with normal hepatic function (3.4%), and 2 patients out of the 22 patients of chronic hepatitis C (9.1 %).

### Example 6: Detection of HNT22 genes in post-transfusion hepatitis cases

For post-transfusion hepatitis cases negative for known hepatitis virus markers, the presence and the timing of the appearance of the HNT22 genes were examined in blood of patients before blood transfusion, after blood transfusion and before crisis of hepatitis, after crisis of hepatitis, and in blood for transfusion (if possible) by the method of Example 2.

### (1) Case 1

Post-transfusion hepatitis case, male, 63 years old.

This case was negative for all markers of HBV, HCV and GBV.C/HGV throughout the examination period. The ALT value, which is a hepatic function marker, was normal before blood transfusion, but abnormal values were observed eight weeks and nine weeks after the blood transfusion. Blood of this case was assayed for the HNT22 genes before the blood transfusion (represented as 0 weeks after blood transfusion) and 6, 8, 9, 10, 11, 12, 15, and 24 weeks after the blood transfusion. As a result, the HNT22 genes were detected from patient blood serum even before the blood transfusion (Table 1). The blood transfused to this case consisted of 4 units. When the presence of the HNT22 genes in these transfusion blood units was examined, the HNT22 genes were detected in 1 unit.

**Table 1**

| Blood collection (week) | ALT (IU/L) | HCV antibody | GBV-C/HGV RNA | HNT22 gene | |
|---|---|---|---|---|---|
| | | | | 1st PCR | 2nd PCR |
| 0 | 11 | - | - | + | + |
| 6 | 62 | - | - | NT | NT |
| 8 | 109 | - | - | NT | NT |
| 9 | 443 | - | - | + | + |
| 10 | 148 | - | - | + | + |
| 11 | 41 | - | - | - | + |
| 12 | 20 | - | - | - | + |
| 15 | 33 | - | - | + | + |
| 24 | 19 | - | - | - | + |

| | | | | | |
|---|---|---|---|---|---|
| NT: not tested | | | | | |

### (2) Case 2

Post-transfusion hepatitis case, male, 58 years old.

This case was negative for all markers of HBV, HCV and GBV-C/HGV throughout the examination period. The ALT value, which is a hepatic function marker, was normal before blood transfusion, but abnormal values were observed around 10 weeks after the blood transfusion. Blood of the patient of this case was assayed for the HNT22 genes 2, 6, 8, and 10 weeks after the blood transfusion. As a result, the HNT22 genes were detected from patient blood sera at 6, 8 and 10 weeks after the blood transfusion in the 1st PCR of the first amplification step (Table 2). In contrast, at 2 weeks, the titer was low, and detected only in the 2nd PCR. The blood transfused in this case was not preserved, and therefore presence of the HNT22 genes in the transfused blood could not be confirmed.

This example is a case where Clone #22 of HNT22 was isolated. It was found that the virus was present at two weeks in the blood which was used as a driver for the isolation. However, because it could be detected only in the highly sensitive second step of the amplification, the amount of the virus at that point was quite small, and therefore it substantially functioned as a driver.

**Table 2**

| Blood collection (week) | ALT (IU/L) | HCV antibody | GBV-C/HGV RNA | HNT22 gene | |
|---|---|---|---|---|---|
| | | | | 1st PCR | 2nd PCR |
| 2 | 36 | - | - | - | + |
| 6 | 37 | - | - | + | + |
| 8 | 55 | - | - | ++ | + |
| 10 | 180 | - | - | ++ | + |

### (3) Case 3

Post-transfusion hepatitis case, male, 56 years old.

This case was also negative for all markers of HBV, HCV and GBV-C/HGV throughout the examination period. The ALT value, which is a hepatic function marker, was normal before blood transfusion, but abnormal values were observed around 6 weeks after the blood transfusion. Blood of the patient of this case was assayed for presence of the HNT22 genes before the blood transfusion (represented as 0 week after blood transfusion) and 2, 4, 6, 8, 12, 13 weeks, 4 and 5 months after the blood transfusion by utilizing PCR comprising the two-step amplification according to the method of Example 2. Serial 10-fold dilutions of the samples were also prepared and assayed in the same manner, thereby the titer of the viral genes (considered to be the amount of the virus) was determined from the maximum dilution ratios where the gene was detected. As a result, while the blood 2 weeks after the blood transfusion was negative, the HNT22 genes were detected in each blood taken 6 weeks to 4 months after the blood transfusion (Table 3). On the other hand, aberration of hepatic function became significant from 6 weeks after the blood transfusion, and then gradually ameliorated, and this process conformed well with the variation of the viral amount. The blood transfused in this case was not preserved, and therefore presence of the HNT22 genes in the transfused blood could not be confirmed.

**Table 3**

| Blood collection (week) | ALT (IU/L) | HCV antibody | GBV-C/HGV RNA | HNT22 gene | |
|---|---|---|---|---|---|
| | | | | 1st PCR | 2nd PCR |
| 0 | 17 | - | - | - | - |
| 2 | 36 | - | - | - | - |
| 4 | 34 | - | - | - | + |
| 6 | 192 | - | - | - | + |
| 8 | 172 | - | - | - | + |

### (4) Case 4

Post-transfusion hepatitis case, female, 70 years old

This case was also negative for all markers of HBV, HCV and GBV-C/HGV throughout the examination period. The ALT value, which is a hepatic function marker, was normal before blood transfusion, but abnormal values was observed around 6 weeks after the blood transfusion. Blood of the patient of this case was assayed for presence of the HNT22 genes 4, 6, 8, 11, 12, 15, 16, 17 and 21 weeks after the blood transfusion by utilizing PCR comprising the two-step amplification according to the method of Examples 2 and 3, and the presence of the HNT22 genes and the titer of the viral genes were also determined as in Case 3 (similar results were obtained by the methods of Examples 2 and 3). As a result, while the blood at 4 weeks after the blood transfusion was negative, the titer increased from 6 weeks after the blood transfusion, and the highest titer was observed at 11 weeks when the aberration of hepatic function was the most significant. Then, as the virus disappeared, the hepatic function was normalized, and thus the variations of the viral amount and the hepatic function were conformed well with each other (Table 4). The blood transfused in this case was not preserved, and therefore presence in the HNT22 genes in the transfused blood could not be confirmed. When the presence of HNT antibodies was assayed by the method described in Example 13, it was positive after the crisis.

**Table 4**

| Blood collection (week) | ALT (IU/L) | HCV antibody | GBV-C/HG V RNA | HNT22 gene | | HNT antibody |
|---|---|---|---|---|---|---|
| | | | | 1st PCR | 2^{nd} PCR | |
| 4 | 14 | - | - | - | - | - |
| 6 | 15 | - | - | - | + | NT |
| 8 | 10 | - | - | + | + | NT |
| 11 | 140 | - | - | ++ | + | NT |
| 12 | 36 | - | - | - | + | NT |
| 15 | 16 | - | - | - | - | NT |
| 16 | 7 | - | - | - | - | NT |
| 17 | 4 | - | - | - | - | NT |
| 21 | 7 | - | - | - | - | + |

| | | | | | | |
|---|---|---|---|---|---|---|
| NT: not tested | | | | | | |

### Example 7: Analysis of blood transfusion infection cases

The facts that the HNT22 genes of the present invention were detected in Example 6 in the cases of post-transfusion hepatitis which was negative for known hepatitis markers and whose cause was indistinct, and that the variation of abnormal value of the hepatic function marker conformed well with the variation of the viral amount strongly suggested that HNT22 was a cause of the hepatitis. This example was performed with the aim of aim of more clearly demonstrating that HNT22 is transmitted by blood transfusion. That is, for cases having a blood transfusion history where the HNT22 genes were detected in blood after blood transfusion and all of the blood pilots used for the transfusion were preserved, blood after blood transfusion and the blood pilots were assayed for the genes by the method described hereinafter to identify blood transfusion pilots positive for the gene and considered to be the source of infection. Further, sequences of the genes obtained from the patients' blood and the pilots were determined, and compared to examine their sequence homology.

For the cases determined as positive for the HNT22 genes by the method described in Example 2 after blood transfusion, it was determined whether the HNT22 genes could be detected in blood before blood transfusion and blood pilots used for blood transfusion. As a result, while the HNT22 genes could not be detected in blood samples before blood transfusion, they were detected in all samples at 2 weeks after blood transfusion and thereafter. Therefore, it was strongly suggested that the infection was brought about by the blood transfusion.

Further, all of the ten blood pilots used were assayed for the HNT22 genes, and the HNT22 genes were detected in one pilot among them. Nucleic acids were extracted from all of the samples positive for HNT22 according to the method described in Example 2. Then, they were amplified as in Example 2 by replacing the sense primer RD037 in the 1st PCR of Example 2 with the primer NG001 as shown in SEQ ID NO: 40, and changing the PCR condition to 35 cycles of a cycle of 94°C for 30 seconds, 55°C for 30 seconds, and 72°C for 1 minute. When amplified under the above conditions, a 415 bp DNA fragment was produced in the positive cases. The second step of the amplification in Example 2 was performed by replacing the sense primer RD051 with NG001, and using PCR condition of a reaction cycle of 94°C for 30 seconds, 55°C for 30 seconds, and 72°C for 1 minute repeated 25 times. When amplified under these conditions, a 396 bp DNA fragment was produced in the positive cases. This 396 bp amplification product was cloned by inserting it into a plasmid vector as in Example 1, and nucleotide sequences were determined and compared for 3 clones for each amplification to examine homology.

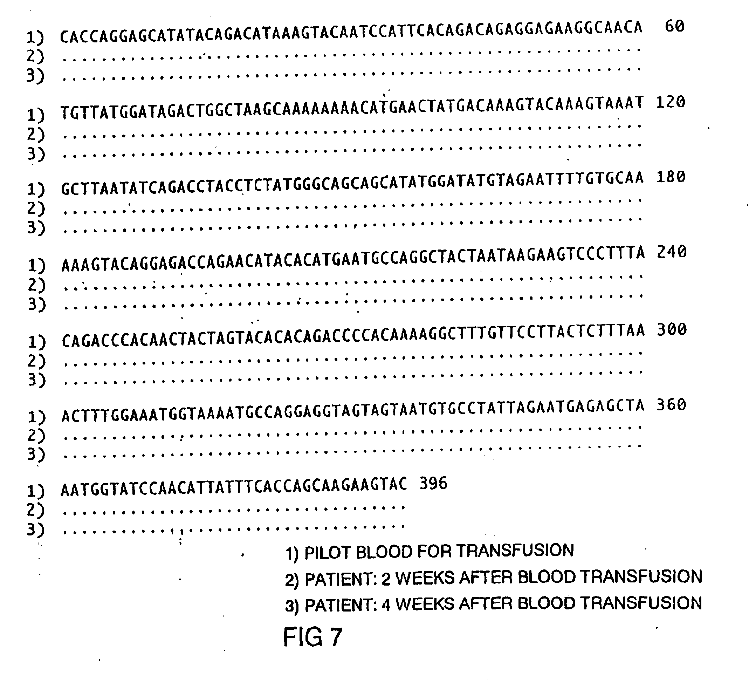

As a result, the sequences of the clones obtained from the blood pilots for blood transfusion that were positive for HNT22 completely conformed with the HNT22 gene sequence obtained from the patients transfused with the pilots (Fig. 7). This demonstrated that HNT22 virus was transmitted by blood transfusion, and became persistent. When this gene sequence was compared with the sequence of Clone #22, 3 nucleotides were different among 356 nucleotides other than those derived from the primers for both of the ends.

### Example 8: Verification of the fact that the virus of the present invention is DNA virus

As hepatitis viruses, there are DNA viruses such as HBV, and RNA viruses such as HCV. The type of virus that the virus of the present invention belongs to was determined by the following procedure.

### (1) PCR which omits reverse transcription reaction step

As described in Example 1, the samples of the isolated genes of the present invention underwent a step of reverse transcription reaction after the nucleic acid extraction. Accordingly, after the reverse transcription reaction, the samples contain DNA which were originally present and DNA which had been present as RNA before the reaction and were converted into DNA by the reverse transcription reaction. Therefore, if the virus of the present invention is an RNA virus, detection of the gene of the virus should become impossible or quite difficult when the reverse transcription reaction is omitted.

Based on the above, the present inventors performed a PCR according to the procedure of Example 2 that omitted the reverse transcription reaction to examine the above possibility. As a result, it was confirmed that the HNT22 genes could be detected at a similar level even without the reverse transcription reaction.

HNT22 was confirmed to be a DNA virus from the above.

### (2) Verification by deoxyribonuclease treatment

In addition to the analysis of the above (1), the present inventors treated a nucleic acid fraction extracted from a sample confirmed by the method of Example 2 to contain the HNT22 genes with a deoxyribonuclease, and subjected it to a reverse transcription reaction, and the resulted product was assayed as a sample for the HNT22 genes by the method of Example 2.

Specifically, a nucleic acid fraction extracted using the same commercially available kit as Example 2 (EX R&D, Sumitomo Metal) was treated with DNase I (Takara Shuzo) at 37°C for 30 minutes, and then the activity of DNase I was inhibited. For this sample, detection of the HNT22 genes was attempted by carrying out the procedure of Example 1 after the reverse transcription under the same conditions. As a result, it was found that the HNT22 genes became undetectable after the treatment of DNase I.

The results of the above (1) and (2) demonstrated that HNT22 virus of the present invention is a DNA virus.

### Example 9: Verification of the fact that the virus of the present invention is a single-stranded DNA virus

### (1) Verification utilizing restriction endonuclease

It has been known that among DNA virsues there are two kinds of viruses having different gene structures, i.e., those having single-stranded DNA and those having double-stranded DNA. The present inventors considered that it is important to determine which kind of the viruses the virus of the present invention belongs to in view of elucidating the identity of the HNT22 virus, and performed the following experiment.

If it is assumed that as the HNT22 virus gene exists in a double-stranded state in nature, there would be several sites specific for restriction endonucleases in its sequence. However, if the viral gene is single stranded, there would be no such restriction endonuclease specific sequences. Conversely, if extracted genes are susceptible to (cleaved by) restriction endonucleases specific for the above sequences, the genes of the virus of the present invention have a double-stranded structure.

Based on the above idea, the present inventors examined whether there is cleavage with a restriction endonuclease *Eco*RI for which a specific sequence would be present in the sequence of the HNT22, genes when they are assumed to be double-stranded, or with a restriction endonuclease *Bgl*II for which a specific sequence would not be recognized as control.

DNA (90 µl) was obtained using the method described in Example 1 from plasma (800 µl) obtained from patients confirmed to be positive for HNT22 virus by the methods of Examples 2 and 3. A 15 µl aliquot of each DNA was taken, 2 µl of *Eco*RI (Takara Shuzo) or *Bgl*II (Takara Shuzo) was added, and incubated at 37°C for 2.5 hours. In parallel, the same reaction was performed by adding phosphate buffer instead of the restriction endonucleases. After the treatment with the restriction endonucleases, a 3 µl aliquot was taken from each sample, 10-fold and 100-fold dilution samples were prepared from it, and it was attempted to amplify a HNT22 gene region containing the EcoRI restriction endonuclease specific sequence for each sample by using the primers NG001 and RD038 which contained an EcoRI restriction endonuclease specific sequence within them. As a result, no cleavage by restriction endonuclease was recognized in any of the samples.

### (2) Verification by mung bean nuclease treatment

Mung bean nuclease has been known to specifically cleave single-stranded DNA. Therefore, it is considered that if HNT22 exists as a single strand, it would be susceptible to mung bean nuclease.

Based on the above idea, the present inventors obtained DNA from the same patient used for the restriction endonuclease treatment experiment in the same manner, and treated it with mung bean nuclease. As a control, a partial region of HNT22 amplified by the method of Example 2 was introduced into phage M13, and a double-stranded phage and a single-strandedphage were prepared, and subjected to mung bean nuclease treatment. Then, the HNT22 genes were amplified according to the method of Example 2. As a result, the DNA obtained from the HNT22 positive patient and the single-stranded DNA derived from the phage could not be amplified, and thus they were susceptible to mung bean nuclease.

From the above results of (1) and (2), it was confirmed that the HNT22 genes exist as a single-stranded DNA, and that HNT22 virus is a single-stranded DNA virus.

### Example 10: Amino acid sequences encoded by HNT22 genes

Based on the HNT22 gene sequences identified in Example 4, the amino acid sequences encoded by the HNT22 genes were analyzed. Initiation codon sequence and termination codon sequence were searched for in the nucleotide sequences obtained, and opening reading frame (ORF) of the HNT22 genes was searched for by considering which combination thereof would afford an ORF in a usually expected size as a determination criterion. As a result, it was found that there were two ORFs, ORF1: nt589-nt2898 (770 amino acid residues), and ORF2: nt107-nt712 (202 amino acid residues) (Fig. 6 and Fig. 8). From the above, it can be assumed that the HNT22 genes encode polypeptides comprising the amino acid sequences shown in SEQ ID NOS: 9 and 10.

Based on these amino acid sequences, hydrophobicity/hydrophilicity characteristics of the encoded polypeptides were analyzed in a conventional manner (Fig. 9).

### Example 11 Isolation of virus particles

Blood that had been found to be positive for the HNT22 genes by the method of Example 2 was fractionated by sucrose density gradient centrifugation to analyze the density distribution of the HNT22 genes, and the possibility to be a virus particle was examined based on the density distribution analysis.
(1) Blood positive for the HNT22 genes (250 µl) and plasma positive for HBV (5 µl) as a control were mixed, and centrifuged at 15000 revolutions/minute for 5 minutes using a refrigerated microcentrifugal separator to precipitate impurities, which were then removed.
(2) The supernatant obtained in the procedure of the above (1) (0.2 ml) was added to a density gradient carrier formed in a centrifugation tube for Beckman SW60 rotor by overlaying layers of sucrose solution having gradient concentrations in the order of 60% (0.7 ml), 50%, 40%, 30%, 20%, and 10% (0.2 ml for each) by weight from the bottom, and TEN buffer (2.3 ml, 10 mM Tris-HCl, pH 8.0, 1 mM EDTA, 0.5 M NaCl) was overlaid thereon.
(3) After centrifugation at 40000 revolutions/minute at 10°C for 45 hours, a 300 µl portion was collected from the bottom of each tube, and subjected to density measurement using a refractometer.
(4) A 100 µl portion was collected from each density fraction, mixed with an extraction reagent (300 µl, Okamoto H. et al., J. Virol. 64:1298-1303, 1990) containing proteinase K (Boehringer Mannheim) and sodium dodecylsulfate- (SDS, Wako - Pure Chemicals Industries), and allowed to react at 70°C for 3 hours. The nucleic acid fraction was extracted by adding phenol, and further extracted with phenol and chloroform. Finally, the nucleic acids were precipitated by adding ethanol, collected, and then dissolved in TE buffer (10 mM Tris-HCl, pH 8.0, 1.0 mM EDTA) to a suitable concentration.
(5) By using the obtained DNA as samples, the HNT22 genes were amplified by the method of Example 2, and the HBV gene was amplified and detected as follows.

A DNA sample (5 µl) was added to a PCR tube containing an amplification reaction mixture comprising a primer S 1-2 as shown in SEQ ID NO: 41 and a primer S2-1 as shown in SEQ ID NO: 42 (0.5 µl for each, 10 OD/ml), which were selected from sequences within a surface antigen gene region of HBV, 2.5 mM dNTP (4 µl), buffer for thermostable DNA polymerase at 10-fold concentration (5.0 µl, buffer attached to AmpliTaq DNA polymerase, Perkin Elmer), thermostable DNA polymerase (0.25 µl, AmpliTaq DNA Polymerase, Perkin Elmer), and distilled water (34.75 µl), overlaid with mineral oil (50 µl), stirred, and centrifuged at 5000 rpm for 30 seconds, and the tube was mounted on a thermal cycler to performed PCR. PCR was performed with a cycle of 94°C for 30 seconds, 55°C for 30 seconds, and 72°C for 75 seconds repeated for 35 cycles. In this method, the HBV gene could be detected as a 250 bp long band in an electrophoresis gel. When more sensitive detection was required, a second PCR was performed using a portion of the above amplification product as follows. That is, the amplification product (5 µl) was added to the same reaction solution as above except that the primers used were changed to primer $088 as shown in SEQ ID NO: 43 and primer S2-2 as shown in SEQ ID NO: 44, and treated in the same manner as above. Then, PCR was performed with a cycle of 94°C for 30 seconds, 55°C for 30 seconds, and 72°C for 60 seconds, which was repeated 25 times. The amplification product provided by the second PCR was detected in the electrophoresis gel as a band of 228 bp.

As a result of the above assay, the HNT22 genes existed as a peak at a sucrose concentration of 54.5%, and density of 1.26 g/cm³, whereas the HBV as the control existed as a peak at a density of 1.26-1.20 g/cm³ as previously reported (Table 5). This density analysis indicated that the HNT22 genes existed in the virus particle fraction, and it was demonstrated that they can be collected in the density fraction mentioned above by density gradient centrifugation.

**Table 5: Distribution of HNT22 genes in sucrose density gradient centrifugation fractions**

| Sucrose density | Density | HNT22-DNA | | HBV-DNA | |
|---|---|---|---|---|---|
| (%) | (g/ml) | 1 st PCR | 2nd PCR | 1 st PCR | 2nd PCR |
| 60.7 | 1.29 | + | + | - | + |
| 54.5 | 1.26 | ++ | + | +++ | + |
| 45.2 | 1.20 | ± | + | +++ | + |
| 30.9 | 1.13 | ± | + | ++ | + |
| 19.5 | 1.08 | - | + | + | + |
| 12.0 | 1.05 | - | - | + | + |

| | | | | | |
|---|---|---|---|---|---|
| -: Negative, ±: Weakly positive, +: Positive, ++: Fairly positive, +++: Strongly positive | | | | | |

### Example 12: HNT22 genotype sequences

Because it had been suggested that HNT22 had several genotypes as mentioned in Example 3, the present inventors searched for genotypes of HNT22 by analyzing the amplified genes of 22 HNT22 positive cases which had been determined positive by the gene detection method of Example 3, and preparing a molecular phylogenetic tree based on the gene sequences.

The present inventors examined Japanese, Americans, and Frenchmen for the presence of the HNT22 genes by the method described in Example 3, and as a result obtained 199 positive cases (187Japanese cases, 8 American cases, 4 Frenchman cases). Genes amplified genes from these positive cases were cloned according to the method described in Example 1, and sequenced for at least 3 clones for each, and a phylogenetic tree was prepared by using commercially available software (Fig. 10, sequences are shown in Figs. 11-12). As a result, the HNT22 genes were classified into 10 types having the sequences shown in SEQ ID NOS: 45-54 (corresponding to nt1939-2160 of SEQ ID NO: 1). The present inventors designated them as Genotypes 1, 2, 3, 4, 5, 6, 7, 8, 9 and 10, respectively.

### Example 13: Anti-HNT22 antibody detection method by immunoprecipitation utilizing isolated HNT22 virus particles

It was demonstrated that the HNT22 particles can be isolated and collected by the method of Example 11. Based on the above finding, the present inventors studied the detection of antibodies by immunoprecipitation.

First, feces were collected from HNT infected patients who had been found to have the HNT22 genes in their blood, and suspended in a suitable solvent to prepare samples, and the presence of the HNT22 genes in the feces was examined according to the method of Example 3. As a result, it was found that the HNT22 genes were also present in the feces, their buoyant density was 1.35 g/cm³, and the gene sequence was identical to the gene sequence of HNT22 obtained from blood of the same patients. Therefore, it was decided to collect HNT22 particles from feces.

Distilled water was added to feces obtained from a patient to prepare a 15% (weight concentration) suspension. This suspension was centrifuged at 3000 rpm for 30 minutes using a refrigerated centrifugal separator (Hitachi) to afford a supernatant. This supernatant was further centrifuged at 10000 rpm for 5 minutes using a refrigerated microcentrifugal separator (High Speed Micro Refrigerated Centrifuge, Tomy Seikou). The supernatant was collected as HNT22 particle suspension. DNA titer of the particle suspension was 10⁵/ml.

The above HNT22 particle suspension (10 µl) was added to serum or plasma (50 µl), and allowed to react at 37°C for 24 hours. After the reaction, goat anti-human IgG (50 µl, #46340, Cappel) was added as secondary antibodies as its stock solution, and allowed to react at 37°C for one hour. After completion of the reaction, the mixture was centrifuged at 10000 rpm for 5 minutes using a refrigerated microcentrifugal separator (High Apeed Micro Refrigerated Centrifuge, Tomy Seikou) to be separated into a supernatant and precipitate. The supernatant was transferred into a separate Eppendorf tube, and DNA was extracted using a commercially available nucleic acid extraction kit (EX R&D, Sumitomo Metal). On the other hand, physiological saline (110 µl) was carefully added to the precipitates, and centrifuged at 10000 rpm for 5 minutes. The supernatant was discarded, and the residue was suspended in physiological saline (110 µl) added dropwise. DNA was extracted from this suspension in the same manner as above. The obtained DNA fraction was dissolved in distilled water (20 µl), and treated at 95°C for 5 minutes, and half of the obtained solution was used as a sample for HNT22 gene detection. Detection of the HNT22 genes was performed in the same manner as in Example 3. The same serum or plasma treated in the same manner without adding the HNT22 particles, and serum or plasma negative for HNT22 treated in the same manner as above were used as controls.

As a result, the HNT22 genes were found in the precipitate fraction of the HNT22 gene positive blood serum or plasma, whereas the HNT22 genes were not found in the precipitate fraction when the particle suspension was not added or HNT negative serum or plasma was used. From these results, it is considered that only when HNT22 antibodies are present, HNT22 particles aggregate with these antibodies and the anti-human IgG antibodies to form a precipitate fraction, and conversely, in a case where the HNT genes are present in the precipitate fraction, the anti-HNT antibodies are present.

### Example 14: Measurement of anti-HNT22 antibody in post-transfusion hepatitis cases and fulminant hepatic failure cases by anti-HNT22 antibody detection method utilizing HNT particle suspension

The present inventors examined the presence or absence of the anti-HNT22 antibodies for Case 4 mentioned in Example 6 who had transiently infected by HNT22 after blood transfusion and exhibited hepatic function aberration, and convalescence cases of sporadic fulminant non-A-G hepatic failure whose infection of known hepatitis viruses had been denied by using the method of Example 13 (Tables 4 and 6).

As a result, it was demonstrated that anti-HNT22 antibodies appeared after disappearance of the virus for both cases, and exhibited a appearance and disappearance pattern similar to that of the neutralizing antibodies observed in viral infection.

**Table 4: Appearance and disappearance of HNT22 gene and anti-HNT22 antibody in convalescence patients of sporadic fulminant non-A-G hepatic failure**

| Blood collection (day) | ALT (IU/L) | HCV antibody | GBV-C/HG V RNA | HNT22 gene | | HNT antibody |
|---|---|---|---|---|---|---|
| | | | | 1st PCR | 2nd PCR | |
| 0 | 2568 | - | - | + | - | - |
| 2 | 523 | - | - | + | + | NT |
| 7 | 152 | - | - | ++ | + | NT |
| 18 | 28 | - | - | + | + | NT |
| 31 | 16 | - | - | - | + | NT |
| 38 | 9 | - | - | - | - | + |

| | | | | | | |
|---|---|---|---|---|---|---|
| NT: not tested After admission ALT HCV GBV-C/HGV Blood collection day (IU/l) antibodyRNA | | | | | | |

### Example 15: Detection and sequencing of HNT22 gene subspecies

Because it was demonstrated that a plurality of genotypes existed for HNT22 virus (Example 12), and other viruses such as hepatitis B and C viruses show regional bias of genotypes, major HNT22 genotypes in the U.S.A. may be different from the Japanese major types. Therefore, HNT22 positive samples obtained from Americans were examined.

Nucleic acids were extracted from 100 µl each of sera from Americans confirmed to be HNT22 virus positive by the method described in Example 3 (designated as UM3-17, UM3-34, UM3-56, and UM3-73, respectively) using a commercially available nucleic acid extraction kit (High Pure Viral Nucleic Acid Kit, Boehringer Mannheim) according to the attached instructions to prepare samples.

PCR was performed using an oligonucleotide as shown in SEQ ID NO: 55 (NG055) and an oligonucleotide as shown in SEQ ID NO: 8 (NG063) selected based on the identified sequence of HNT22 virus as primers, and the samples obtained above as templates, and according to the following conditions. The locational relation of the primers used is shown in Fig. 13.

| PCR conditions | |
|---|---|
| (1) Reaction mixture composition | |
| Template nucleic acid solution | 10 µl |
| 10x Ex. Taq buffer | 5 µl . |
| 2.5 mM dNTP | 4 µl |
| Primer (NG055: 10 OD/ml) | 1 µl (OD at 280nm, same shall apply hereinafter) |
| Primer (NG063: 10 OD/ml) | I µl |
| Distilled water | 29.5 µl |
| Ex. Taq DNA polymerase | 1 µl |
| Total | 50 µl |

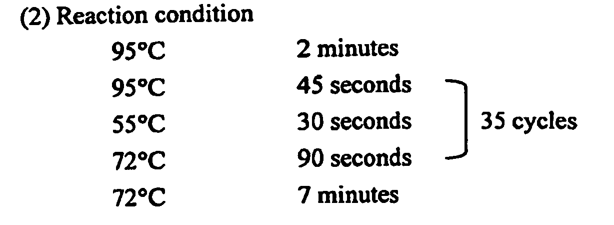

As a result, gene amplification was obtained for all of the samples. A plurality of amplification fragments were cloned into T vector for each sample in the same manner as in Example 1, and the gene sequences were determined. As a result clones exhibiting high homology to the already identified nucleotide sequences and, in addition, clones having a sequence exhibiting low homology to those sequences were obtained from UM3-17 and UM3-73 (referred to as U17-2 and U73-2 respectively hereinafter). Both of the sequences exhibited 30% homology to a corresponding region of the nucleotide sequences identified so far. From UM3-56 and UM3-34, only nucleotide sequences exhibiting high homology to the nucleotide sequences already identified were obtained.

An oligonucleotide (NT01) as shown in SEQ ID NO: 56 was prepared as a primer for further amplifying a nucleotide sequence of the 5' end of the above clone, based on comparison of sequences between the U 17-2 clone obtained above and HNT22 virus identified so far, and selection of a highly conserved region. PCR was performed using this oligonucleotide NT01 and an oligonucleotide as shown in SEQ ID NO: 57 (NG054) prepared based on the sequence of HNT22 virus already identified as primers, and a nucleic acid derived from UM3-17 as a template, and according to the following conditions. The obtained amplification fragment was sequenced in the same manner as described above. The relative locations of the primers used is shown in Fig. 13.

| PCR conditions | |
|---|---|
| (1) Reaction mixture composition | |
| Template nucleic acid solution | 10 µl |
| 10x Ex. Taq buffer | 5 µl |
| 2.5 mM dNTP | 4 µl |
| Primer (NTO1: 10 OD/ml) | 1 µl |
| Primer (NG054: 10 OD/ml) | 1 µl |
| Distilled water | 29.5 µl |
| Ex. Taq DNA polymerase | 1 µl |
| Total | 50 µl |

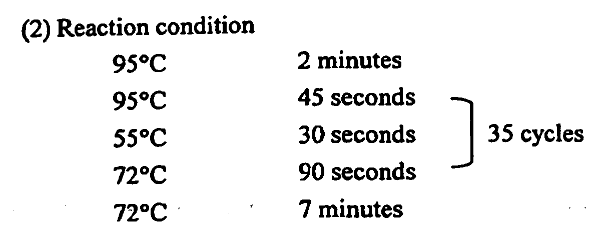

On the other hand, oligonucleotides as shown in SEQ ID NOS: 58 and 59 (primers NT03 and NT04, respectively) were prepared as primers for further amplifying a sequence of the 3' end of the above clones, based on the 3' end nucleotide sequence of U17-2 and the already identified sequence of HNT22 virus, and oligonucleotides as shown in SEQ ID NOS: 60 and 61 (primer NG065 and NG021, respectively) were prepared as primers for amplification, based on an already identified sequence around the 3' end of the nucleotide sequence of HNT22 virus. First, 3' end of the gene was amplified using NT03 and NG065, and a part of the obtained amplification fragment was amplified using NT04 and NG065 or NG021. The relative locations of the primers used is shown in Fig. 13. The amplification was performed with the following conditions using a nucleic acid derived from UM3-17 as a template. The nucleotide sequence of the amplification fragment was determined in the same manner as described above.

| PCR conditions | |
|---|---|
| First step PCR | |
| (1) Reaction mixture composition | |
| Template nucleic acid solution | 10 µl |
| 10x Ex. Taq buffer | 5 µl |
| 2.5 mM dNTP | 4 µl |
| Primer (NG03: 10 OD/ml) | 1 µl |
| Primer (NG065: 10 OD/ml) | 1 µl |
| Distilled water | 29.5 µl |
| Ex. Taq DNA polymerase | 1 µl |
| Total | 50 µl |

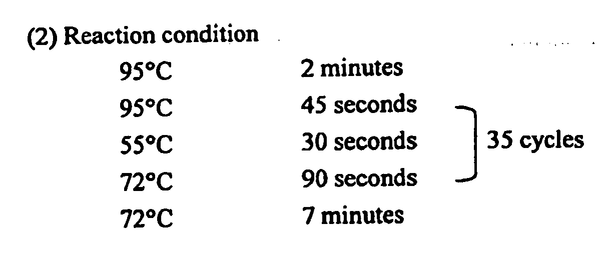

| Second step PCR | |
|---|---|
| (1) Reaction mixture composition | |
| Template nucleic acid solution | 5 µl |
| 10x Ex. Taq buffer | 5 µl |
| 2.5 mM dNTP | 4 µl |
| Primer (NT04: 10 OD/ml) | 0.5 µl |
| Primer (NG065: 10 OD/ml) or | |
| Primer (NG021: 10 OD/ml) | 0.5 µl |
| Distilled water | 34.5 µl |
| Ex. Taq DNA polymerase | 0.5 µl |
| Total | 50 µl |

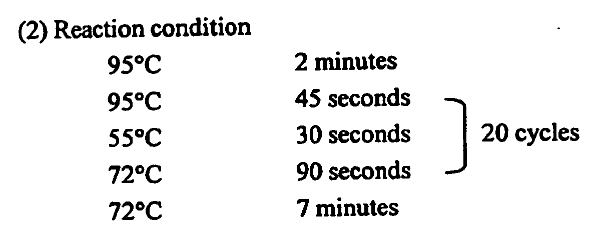

From the above procedure, clones 1-1, 1-9 and 1-10 were obtained as 5' end sequence clones of U17-2, and 2A-3 (when the primers in the second step PCR were the, _ combination of NT04/NG065), and 2B-1 and 2B-3 (when the primers in the second step PCR were the combination ofNT04/NG021) were obtained as 3' end sequence clones. Nucleotide sequences of these clones were determined in the same manner as described above, and ligated to obtain a nucleotide sequence of virus gene detected from sera derived from Americans (SEQ ID NO: 62).

By comparing this sequence with the nucleotide sequences of the HNT22 genes, it was found that extremely high homology can be observed for the 5' end and 3' end sequences whereas homology of the other sequences was as low as around 30%, and this sequence could not be detected by the detection methods of Examples 2 and 3. The virus having this gene was designated as TUSO1.

The nucleotide sequence of the TUS01 gene was searched for ORFs in the same manner as in Example 10. As a result, it was found that two ORFs, ORF1: nt590-nt2872 (761 amino acid residues) and ORF2: nt258-nt725 (156 amino acid residues) were present (Fig. 15). These existed at locations corresponding to those of ORF1 and ORF2 in the nucleotide sequence of the HNT22 genes.

Homology of each region was summarized in Table 7.

**Table 7: Homology of HNT22 gene and TUS01 gene**

| | HNT22²⁾ | TUS01 | Sequence homology |
|---|---|---|---|
| Full length | 3739nt | 3722nt | 63.7% |
| 5' untranslated region | 262nt | 257nt | 90.3% |
| | | | |
| Translated region | 2636nt | 2615nt | 54.7% |
| ORF1 | 2310nt | 2283nt | 54.8% |
| (Amino acid sequence) | 770aa | 761 aa | 37.0% |
| ORF2¹⁾ | 450nt | 468nt | 55.5% |
| (Amino acid sequence) | 150aa | 156aa | 38.8% |
| | | | |
| 3' untranslated region | 841nt | 850nt | 84.2% |

| | | | |
|---|---|---|---|
| 1) For ORF2, to make comparison based on TUS01, the frame from the second ATG codon was employed for HNT22. 2) Sequence used as HNT22 was that of TA278. | | | |

The results of the comparison of nucleotide sequences of highly homologous 5' untranslated region (5' end region) and 3' untranslated region (3' end region) are shown in Figs. 15 and 16.

When homology of the nucleotide sequence of the TUSO1. gene with known sequences was determined by using FASTA and BLAST in the same manner as in Example 4, all of those exhibiting high homology were nucleotide sequences of the HNT22 genes.

Judging from the above-mentioned characteristics, TUS01 virus is assumed to be a subspecies of HNT22 virus.

### Example 16: Simultaneous detection of HNT22 gene and TUS01 gene

A method capable of simultaneously detecting the TUS01 gene; which cannot be detected by the methods of Examples 2 and s, was studied.

By comparing both sequences NG054 and NG065 were selected as oligonucleotides containing a common sequence and considered to be suitable as PCR primers,. Nucleic acids were extracted from blood serum (100 µl) according to the method described in Example 2. The extracted nucleic acids were dissolved in distilled water (10 µl). The dissolved nucleic acids (10 µl) were added to a tube used exclusively for PCR containing solutions of NG054 and NG065 (1 µl, 10 OD/ml for each), Ex. Taq buffer at 10-fold concentration (5 µl, TaKaRa Shuzo), 2.5 mM dNTP (4 µl), and distilled water (29.5 µl), to which thermostable DNA polymerase (Ex. Taq Polymerase: TaKaRa Shuzo) was immediately added, and amplified by treatment at 95°C for 2 minutes, followed by a cycle of 95°C for 45 seconds, 55°C for 30 seconds, and 72°C for 120 seconds, which was repeated 35 times, and finally a reaction at 72°C for 7 minutes. The amplification products were separated by agarose gel electrophoresis, and the presence of bands of lengths predicted from the nucleotide sequences was confirmed. The amplification products for which bands were confirmed were sequenced. As a result, it was found that the HNT22 gene and the TUS01 gene could be simultaneously detected by this method.

### Industrial Applicability

According to the present invention, an as yet unknown etiologic virus of blood-borne infectious hepatitis whose cause had been indistinct was identified. This enables the establishment of methods for gene assay, antigen assay, and antibody assay, and the provision of kits for test of the hepatitis, and vaccines for prevention thereof.

## Claims

1. An isolated non-B, non-C, non-G hepatitis virus DNA which shows at least 60% homology to the sequence of SEQ ID NO:1.

2. The DNA of claim 1 wherein the homology is at least 80% .

3. The DNA of claim 2, consisting of SEQ ID NO: 1.

4. The DNA according to claim 1, wherein a fragment of between 200 nucleotides and 350 nucleotides long can be amplified by PCR using a pair of oligonucleotide primers consisting of:
(a) an oligonucleotide consisting of the sequence of SEQ ID NO:6 and an oligonucleotide consisting of the sequence of SEQ ID NO: 8; or
(b) an oligonucleotide consisting of the sequence of SEQ ID NO: 7 and an oligonucleotide consisting of the sequence of SEQ ID NO: 8.

5. A polynucleotide complementary to the full length of the DNA sequence as defined in any one of claims 1 to 3.

6. An oligonucleotide consisting of a sequence selected from:
(i) SEQ ID NO: 2, SEQ ID NO: 3, SEQ ID NO: 4, SEQ ID NO: 5, SEQ ID NO: 6, SEQ ID NO: 7, or SEQ ID NO: 8; or
(ii) a sequence complementary to the full length of SEQ. ID NO: 2, SEQ. ID NO: 3, SEQ. ID NO: 4, SEQ. ID NO: 5, SEQ. ID NO: 6, SEQ. ID NO: 7 or SEQ. ID NO. 8.

7. A method for detecting non-B, non-C, non-G hepatitis virus gene wherein:
(a) PCR is performed using a pair of oligonucleotide primers; and
(b) the length of each oligonucleotide primer is between 6 and 50 bases long; and
(c) each oligonucleotide primer is capable of hybridisation to:
(i) the DNA defined in any one of claims 1 to 4; or
(ii) the complement of the DNA defined in any one of claims 1 to 4.

8. The method according to claim 7, wherein PCR is performed using a pair of oligonucleotide primers selected from the oligonucleotides defined in claim 6.

9. The method according to claim 8, wherein PCR is performed by using a primer pair consisting of:
(a) the oligonucleotide sequence of SEQ. ID NO: 2 and the oligonucleotide sequence of SEQ. ID NO. 3; or
(b) the oligonucleotide sequence of SEQ. ID NO: 4 and the oligonucleotide sequence of SEQ. ID NO: 5.

10. The method according to claim 8, wherein PCR is performed by using a primer pair consisting of:
(a) the oligonucleotide sequence of SEQ. ID NO: 6 and the oligonucleotide sequence of SEQ. ID NO: 8; or
(b) the oligonucleotide sequence of SEQ. ID NO: 7 and the oligonucleotide sequence of SEQ. ID NO: 8.

11. A method for differentiating non-B, non-C, non-G hepatitis virus genotypes wherein:
(a) a sample is subjected to the method as defined in claim 9 and the method as defined in claim 10; and
(b) the results obtained from each method are compared.

12. A method for differentiating non-B, non-C, non-G hepatitis virus genotypes, comprising:
(a) performing hybridization using an oligonucleotide consisting of a sequence selected from SEQ ID NO: 45, SEQ ID NO: 46, SEQ ID NO: 47, SEQ ID NO: 48, SEQ ID NO: 49, SEQ ID NO: 50, SEQ ID NO:51, SEQ ID NO: 52, SEQ ID NO: 53, and SEQ ID NO: 54; and
(b) using the hybridization result to determine the genotype.

13. An isolated non-B, non-C, non-G hepatitis virus polypeptide consisting of:
(a) the amino acid sequence of SEQ ID NO: 9 or 10; or
(b) an amino acid sequence having at least 65% homology to the amino acid sequence of SEQ ID NO: 9 or 10.

14. The polypeptide according to claim 13 consisting of the amino acid sequence of SEQ ID NO: 9.

15. The polypeptide according to claim 13 consisting of the amino acid sequence of SEQ ID NO: 10.

16. A method for isolating non-B, non-C, non-G hepatitis virus particles containing the DNA as defined in claim 1, said method comprising isolating the non-B, non-C, non-G hepatitis virus particles based on the density of the particles.

17. Isolated non-B, non-C, non-G hepatitis particles containing the DNA as defined in claim 1.

18. A non-B, non-C, non-G hepatitis virus polypeptide encoded within an open reading frame of a DNA as defined in claim 1, obtained from virus particles as defined in claim 17.

19. A recombinant gene expression vector comprising a nucleotide sequence encoding the polypeptide of claim 13.

20. A cell transformed with the recombinant vector of claim 19.

21. A non-B, non-C, non-G hepatitis virus antigen peptide or an antigenic fragment thereof expressed by the transformant cell as defined in claim 20.

22. A method for producing a non-B, non-C, non-G hepatitis virus antigen peptide, which comprises:
(a) culturing the transformant cell as defined in claim 20 under conditions that allow the non-B, non-C, non-G hepatitis virus antigen peptide to be expressed; and
(b) collecting the expressed peptide.

23. A method for immunoiogicaiiy detecting a non-B, non-C, non-G hepatitis virus antibody using the polypeptide as defined in any one of claims 11 to 13 and 18 or the virus particles as defined in claim 17 as an antigen.

24. A method for producing an antibody against non-B, non-C, non-G hepatitis virus, which comprises:
(a) immunizing an animal with the polypeptide as defined in any one of claims 11 to 13 and 18 or the virus particles as defined in claim 17 as an immunogen; and
(b) isolating the antibody.

25. An isolated antibody obtainable by the method as defined in claim 24.

26. A method for immunologically detecting a non-B, non-C, non-G hepatitis virus antigen by using the antibody as defined in claim 25.

27. An isolated non-B, non-C, non-G hepatitis virus DNA, consisting of the sequence of SEQ ID NO: 62.

28. A polynucleotide having a nucleotide sequence complementary to the full length of a nucleotide sequence of the DNA as defined in claim 27.

29. A method for detecting a non-B, non-C, non-G hepatitis virus gene wherein:
(a) PCR is performed by using a pair of oligonucleotide primers; and
(b) the length of each oligonucleotide primer is between 6 and 50 bases long; and
(c) each oligonucleotide primer is capable of hybridisation to:
(i) the DNA defined in claim 27; or
(ii) the complement of the DNA defined in claim 27.

30. The method according to claim 29 wherein PCR is performed by using a primer pair consisting of:
(a) the oligonucleotide of SEQ ID NO: 57, and the oligonucleotide of SEQ ID NO: 60; or
(b) the oligonucleotide of SEQ ID NO: 57 and the oligonucleotide of SEQ ID NO: 61.

31. An isolated non-B, non-C, non-G hepatitis virus polypeptide consisting of the amino acid sequence of SEQ ID NO: 63 or 64.

32. A recombinant gene expression vector comprising a nucleotide sequence encoding all or an antigenic part of the amino acid sequence of either SEQ ID NO: 63 or 64.

33. A transformant cell, containing a nucleotide sequence encoding all or an antigenic part of an amino acid sequence of either SEQ ID NO: 63 or 64.

34. A non-B, non-C, non-G hepatitis virus antigen peptide or an antigenic fragment thereof expressed by the transformant cell as defined in claim 33.

35. A method for producing a non-B, non-C, non-G hepatitis virus antigen peptide comprising:
(a) culturing the transformant cell as defined in claim 33 under conditions that allow the non-B, non-C, non-G hepatitis virus antigen peptide to be expressed and
(b) collecting the expressed peptide.

36. A method for immunologically detecting a non-B, non-C, non-G hepatitis virus antibody using the polypeptide as defined in claim 31 as an antigen.

37. A method for producing an antibody against a non-B, non-C, non-G hepatitis virus comprising:
(a) immunizing an animal with the polypeptide as defined in claim 31 as an immunogen; and
(b) isolating the antibody.

38. An isolated antibody obtainable by the method as defined in claim 37.

39. A method for immunologically detecting a non-B, non-C, non-G hepatitis virus antigen using the antibody as defined in claim 38.

## Patentansprüche

1. Isolierte Non-B-, Non-C-, Non-G-Hepatitisvirus-DNA, die wenigstens 60 % Homologie mit der Sequenz von SEQ ID NO: 1 zeigt.

2. DNA nach Anspruch 1, wobei die Homologie wenigstens 80 % beträgt.

3. DNA nach Anspruche 2, bestehe aus SEQ ID NO: 1.

4. DNA nach Anspruch 1, wobei ein Fragment mit einer Länge zwischen 200 Nucleotiden und 350 Nucleotiden mittels PCR amplifiziert werden kann, wobei ein Paar von Oligonucleotidprimern verwendet wird, bestehend aus:
(a) einem aus der Sequenz von SEQ ID NO: 6 bestehenden Oligonucleotid und einem aus der Sequenz von SEQ ID NO: 8 bestehenden Oligonucleotid; oder
(b) einem aus der Sequenz von SEQ ID NO: 7 bestehenden Oligonucleotid und einem das aus der Sequenz von SEQ ID NO: 8 bestehenden Oligonucleotid.

5. Polynucleotid, das zu der vollständigen DNA-Sequenz gemäß irgendeinem der Ansprüche 1 bis 3 komplementär ist.

6. Oligonucleotid, bestehend aus einer Sequenz, die ausgewählt ist aus:
(i) SEQ ID NO: 2, SEQ ID NO: 3, SEQ ID NO: 4, SEQ ID NO: 5, SEQ ID NO: 6, SEQ ID NO: 7 oder SEQ ID NO: 8; oder
(ii) einer zu der vollständigen Sequenz von SEQ ID NO:2,SEQ ID NO:3, SEQ ID NO: 4, SEQ ID NO: 5, SEQ ID NO: 6, SEQ ID NO: 7 oder SEQ ID NO: 8 komplementären Sequenz.

7. Verfahren zum Nachweis eines Non-B-, Non-C-, Non-G-Hepatitisvirusgens, wobei:
(a) eine PCR mit einem Paar von Oligonucleotidprimern durchgeführt wird; und
(b) die Länge eines jeden Oligonucleotidprimers zwischen 6 und 50 Basen beträgt; und
(c) jeder Oligonucleotidprimer in der Lage ist, mit:
(i) der DNA gemäß irgendeinem der Ansprüche 1 bis 4; oder
(ii) dem Komplementärstrang der DNA gemäß irgendeinem der Ansprüche 1 bis 4
zu hybridisieren.

8. Verfahren nach Anspruch 7, wobei die PCR mit einem Paar von Oligonucleotidprimern durchgeführt wird, ausgewählt aus den Oligonucleotiden gemäß Anspruch 6.

9. Verfahren nach Anspruch 8, wobei die PCR mit einem Primerpaar durchgeführt wird, bestehend aus:
(a) der Oligonucleotidsequenz von SEQ ID NO: 2 und der Oligonucleotidsequenz von SEQ ID NO. 3 oder
(b) der Oligonucleotidsequenz von SEQ ID NO: 4 und der Öligonucleotidsequenz von SEQ ID NO: 5.

10. Verfahren nach Anspruch 8, wobei die PCR mit einem Primerpaar durchgeführt wird, bestehend aus:
(a) der Oligonucleotidsequenz von SEQ ID NO: 6 und der Oligonucleotidsequenz von SEQ ID NO:8; oder
(b) der Oligonucleotidesequenz von SEQ ID NO 7 und der Oligonucleotidsequenz von SEQ ID NO: 8.

11. Verfahren zum Differenzieren von Non-B-, Non-C-, Non-G-Hepatitisvirusgenotypen, wobei:
(a) eine Probe dem Verfahren gemäß Anspruch 9 und-dem Verfahren gemäß Anspruch 10 unterworfen wird; und
(b) die bei jedem Verfahren erhaltenen Ergebnisse verglichen werden.

12. Verfahren zum Differenzieren von Non-B-, Non-C-, Non-G-Hepatitisvirus-genotypen, umfassend:
(a) Durchführen einer Hybridisierung mit einem Oligonucleotid, das aus einer Sequenz besteht, die ausgewählt ist aus SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53 und SEQ ID NO: 54; und
(b) Verwenden des Hybridisierungsergebnisses zur Bestimmung des Genotyps.

13. Isoliertes Non-B-, Non-C-, Non-G-Hepatitisviruspolypeptid, bestehend aus:
(a) der Aminosäuresequenz von SEQ ID NO: 9 oder 10; oder
(b) einer Aminosäuresequenz mit wenigstens 65 % Homologie zu der Aminosäuresequenz von SEQ ID NO: 9 oder 10.

14. Polypeptid nach Anspruch 13, bestehend aus der Aminosäuresequenz von SEQ ID NO: 9.

15. Polypeptid nach Anspruch 13, bestehend aus der Aminosäuresequenz von SEQ ID NO: 10.

16. Verfahren zum Isolieren von Non-B-, Non-C-, Non-G-Hepatitisviruspartikeln, die die DNA gemäß Anspruch 1 enthalten, wobei das Verfahren das Isolieren der Non-B-, Non-C-, Non-G-Hepatitisviruspartikel auf Basis der Dichte der Partikel umfasst.

17. Isolierte Non-B-, Non-C-, Non-G-Hepatitispartikel, die die DNA gemäß Anspruch 1 enthalten.

18. Non-B-, Non-C-, Non-G-Hepatitisviruspolypeptid, das in einem offenen Leserahmen einer DNA gemäß Anspruch 1 codiert, erhalten aus Viruspartikeln gemäß Anspruch 17.

19. Rekombinanter Genexpressionsvektor, umfassend eine Nucleotidsequenz, die für das Polypeptid nach Anspruch 13 codiert.

20. Zelle, die mit dem rekombinanten Vektor nach Anspruch 19 transformiert ist.

21. Non-B-, Non-C-, Non-G-Hepatitisvirusantigenpeptid oder antigenes Fragment davon, exprimiert von der transformierten Zelle gemäß Anspruch 20.

22. Verfahren zur Herstellung eines Non-B-, Non-C-, Non-G-Hepatitisvirusantigenpeptids, welches umfasst:
(a) Kultivieren der transformierten Zelle gemäß Anspruch 20 unter Bedingungen, die die Expression des Non-B-, Non-C-, Non-G-Hepatitisvirusantigenpeptids erlauben; und
(b) Sammeln des exprimierten Peptids.

23. Verfahren zum immunologischen Nachweis eines Non-B-, Non-C-, Non-G-Hepatitisvirusantikörpers unter Verwendung des Polypeptids gemäß irgendeinem der Ansprüche 11 bis 13 und 18 oder der Viruspartikel gemäß Anspruch 17 als einem Antigen.

24. Verfahren zur Herstellung eines Antikörpers gegen Non-B-, Non-C, Non-G-Hepatitisvirus, welches umfasst:
(a) Immunisieren eines Tiers mit dem Polypeptid gemäß irgendeinem der Ansprüche 11 bis 13 und 18 oder Polypeptidiruspartikeln gemäß Anspruch 17 als einem Immunogen; und
(b) Isolieren des Antikörpers.

25. Isolierter Antikörper, erhältlich nach dem Verfahren gemäß Anspruch 24.

26. Verfahren zum immunologischen Nachweis eines Non-B-, Non-C-, Non-G-Hepatitisvirusantigens unter Verwendung des Antikörpers gemäß Anspruch 25.

27. Isolierte Non-B-, Noh-C-, Non-G-Hepatitisvirus-DNA, bestehend aus der Sequenz von SEQ ID:NO:62.

28. Polynucleotid mit einer Nucleotidsequenz, die zu der vollständigen Sequenz einer Nucleotidsequenz der DNA gemäß Anspruch 27 komplementär ist.

29. Verfahren zum Nachweis eines Non-B-, Non-C-, Non-G-Hepatitisvirusgens, wobei:
(a) eine PCR mit einem Paar von Oligonucleotidprimern durchgeführt wird; und
(b) die Länge eines jeden Oligonucleotidprimers zwischen 6 und 50 Basen beträgt; und
(c) jeder Oligonucleotidprimer in der Lage ist; mit:
(i) der DNA gemäß Anspruch 27; oder
(ii) dem Komplementärstrang der DNÄ gemäß Anspruch 27
zu hybridisieren.

30. Verfahren nach Anspruch 29, wobei die PCR mit einem Primerpaar durchgeführt wird, bestehend aus:
(a) dem Oligonucleotid von SEQ ID NO: 57 und dem Oligonucleotid von SEQ ID NO: 60; oder
(b) dem Oligonucleotid von SEQ ID NO: 57 und dem Oligonucleotid von SEQ ID NO: 61.

31. Isoliertes Non-B-, Non-C-, Non-G-Hepatitisviruspolypeptid, bestehend aus der Aminosäüresequenz von SEQ ID NO: 63 oder 64.

32. Rekombinanter Genexpressionsvektor, umfassend eine Nucleotidsequenz, die für die ganze oder einen antigenen Teil der Aminosäuresequenz von entweder SEQ ID NO: 63 oder 64 codiert.

33. Transformierte Zelle, enthaltend eine Nucleotidsequenz, die für die ganze oder einen antigenen Teil einer Aminosäuresequenz von entweder SEQ ID NO: 63 oder 64 codiert.

34. Non-B-, Non-C-, Non-G-Heqatitisvirusantigenpeptid oder antigenes Fragment davon, exprimiert von der transformierten Zelle gemäß Anspruch 33.

35. Verfahren zur Herstellung eines Non-B-, Non-C-, Non-G-Hepatitisvirus-antigenpeptids, umfassend:
(a) Kultivieren der transformierten,Zelle gemäß Anspruch 33 unter Bedingungen, die die Expression des Non-B-, Non-C-, Non-G-Hepatitisvirusantigenpeptids erlauben; und
(b) Sammeln des exprimierten Peptids.

36. Verfahren zum immunologischen Nachweis eines Non-B-, Non-C-, Non-G-Hepatitisvirusantikörpers unter Verwendung des Polypeptids gemäß Anspruch 31 als einem Antigen.

37. Verfahren zur Herstellung eines Antikörpers gagen ein Noh-B-, Non-C-, Non-G-Hepatitisvirus, umfassend.
(a) Immunisieren eines Tiers mit dem Polypeptid gemäß Anspruch 31 als einem Immunogen; und,
(b) Isolieren des Antikörpers.

38. Isolierter Antikörper, erhältlich nach dem Verfahren gemäß Anspruch 37.

39. Verfahren zum immunologischen Nachweis eines Non-B-, Non-C-, Non-G-Hepatitisvirusantigens unter Verwendung des Antikörpers gemäß Anspruch 38.

## Revendications

1. ADN isolé du virus de l'hépatite non-B, non-C, non-G qui montre au moins 60% d'homologie avec la séquence de SEQ ID NO : 1.

2. ADN selon la revendication 1 dans lequel l'homologie est au moins 80%.

3. ADN selon la revendication 2, consistant en SEQ ID NO : 1.

4. ADN selon la revendication 1, dans lequel un fragment d'une longueur d'entre 200 nucléotides et 350 nucléotides peut être amplifié par PCR en utilisant une paire d'amorces oligonucléotidiques consistant en :
(a) un oligonucléotide consistant en la séquence de SEQ ID NO: 6 et un oligonucléotide consistant en la séquence de SEQ ID NO : 8 ; ou
(b) un oligonucléotide consistant en la séquence de SEQ ID NO: 7 et un oligonucléotide consistant en la séquence de SEQ ID NO : 8.

5. Polynucléotide complémentaire de la longueur complète de la séquence d'ADN telle que définie dans l'une quelconque des revendications 1 à 3.

6. Oligonucléotide consistant en une séquence choisie parmi :
(i) SEQ ID NO : 2, SEQ ID NO : 3, SEQ ID NO : 4, SEQ ID NO : 5, SEQ ID NO : 6, SEQ ID NO : 7, ou SEQ ID NO : 8 ; ou
(ii) une séquence complémentaire de la longueur complète de SEQ ID NO : 2, SEQ ID NO : 3, SEQ ID NO : 4, SEQ ID NO : 5, SEQ ID NO : 6, SEQ ID NO : 7 ou SEQ ID NO : 8.

7. Procédé pour détecter le gène du virus de l'hépatite non-B, non-C, non-G dans lequel :
(a) une PCR est effectuée en utilisant une paire d'amorces oligonucléotidiques ; et
(b) la longueur de chaque amorce oligonucléotidique est entre 6 et 50 bases ; et
(c) chaque amorce oligonucléotidique est capable d'une hybridation avec :
(i) l'ADN défini dans l'une quelconque des revendications 1 à 4 ; ou
(ii) le complément de l'ADN défini dans l'une quelconque des revendications 1 à 4.

8. Procédé selon la revendication 7 dans lequel la PCR est effectuée en utilisant une paire d'amorces oligonucléotidiques choisies parmi les oligonucléotides définis à la revendication 6.

9. Procédé selon la revendication 8 dans lequel la PCR est effectuée en utilisant une paire d'amorces consistant en :
(a) l'oligonucléotide ayant la séquence de SEQ ID NO : 2 et l'oligonucléotide ayant la séquence de SEQ ID NO : 3 ; ou
(b) l'oligonucléotide ayant la séquence de SEQ ID NO : 4 et l'oligonucléotide ayant la séquence de SEQ ID NO : 5.

10. Procédé selon la revendication 8 dans lequel la PCR est effectuée en utilisant une paire d'amorces consistant en :
(a) l'oligonucléotide ayant la séquence de SEQ ID NO : 6 et l'oligonucléotide ayant la séquence de SEQ ID NO : 8 ; ou
(b) l'oligonucléotide ayant la séquence de SEQ ID NO : 7 et l'oligonucléotide ayant la séquence de SEQ ID NO : 8.

11. Procédé pour différencier les génotypes du virus de l'hépatite non-B, non-C, non-G dans lequel :
(a) un échantillon est soumis au procédé tel que défini dans la revendication 9 et au procédé tel que défini dans la revendication 10 ; et
(b) les résultats obtenus avec chaque procédé sont comparés.

12. Procédé pour différencier les génotypes du virus de l'hépatite non-B, non-C, non-G, comprenant :
(a) la réalisation d'une hybridation en utilisant un oligonucléotide consistant en une séquence choisie parmi SEQ ID NO : 45, SEQ ID NO : 46, SEQ ID NO : 47, SEQ ID NO : 48, SEQ ID NO : 49, SEQ ID NO : 50, SEQ ID NO : 51, SEQ ID NO : 52, SEQ ID NO : 53, et SEQ ID NO : 54 ; et
(b) l'utilisation du résultat de l'hybridation pour déterminer le génotype.

13. Polypeptide isolé du virus de l'hépatite non-B, non-C, non-G consistant en :
(a) la séquence d'acides aminés de SEQ ID NO : 9 ou 10 ; ou
(b) une séquence d'acides aminés ayant au moins 65% d'homologie avec la séquence d'acides aminés de SEQ ID NO : 9 ou 10.

14. Polypeptide selon la revendication 13 consistant en la séquence d'acides aminés de SEQ ID NO : 9.

15. Polypeptide selon la revendication 13 consistant en la séquence d'acides aminés de SEQ ID NO : 10.

16. Procédé pour isoler des particules du virus de l'hépatite non-B, non-C, non-G contenant l'ADN tel que défini dans la revendication 1, ledit procédé comprenant l'isolement des particules du virus de l'hépatite non-B, non-C, non-G sur la base de la densité des particules.

17. Particules isolées du virus de l'hépatite non-B, non-C, non-G contenant l'ADN tel que défini dans la revendication 1.

18. Polypeptide du virus de l'hépatite non-B, non-C, non-G codé au sein d'un cadre de lecture ouvert d'un ADN tel que défini dans la revendication 1, obtenu à partir de particules virales telles que définies dans la revendication 17.

19. Vecteur d'expression de gène recombinant comprenant une séquence de nucléotides codant pour le polypeptide de la revendication 13.

20. Cellule transformée avec le vecteur recombinant de la revendication 19.

21. Antigène peptidique du virus de l'hépatite non-B, non-C, non-G ou fragment antigénique de celui-ci exprimé par la cellule transformante telle que définie dans la revendication 20.

22. Procédé pour la production d'un antigène du virus de l'hépatite non-B, non-C, non-G, qui comprend :
(a) la mise en culture de la cellule transformante telle que définie à la revendication 20 dans des conditions qui permettent que le peptide antigène du virus de l'hépatite non-B, non-C, non-G soit exprimé ; et
(b) la récolte du peptide exprimé.

23. Procédé pour détecter par voie immunologique un anticorps du virus de l'hépatite non-B, non-C, non-G en utilisant le polypeptide tel que défini dans l'une quelconque des revendications 11 à 13 et 18, ou les particules virales telles que définies dans la revendication 17 en tant qu'antigène.

24. Procédé pour la production d'un anticorps contre le virus de l'hépatite non-B, non-C, non-G, qui comprend :
(a) l'immunisation d'un animal avec le polypeptide tel que défini dans l'une quelconque des revendications 11 à 13 et 18 ou avec les particules virales telles que définies dans la revendication 17 en tant qu'immunogène ; et
(b) l'isolement de l'anticorps.

25. Anticorps isolé pouvant être obtenu par le procédé tel que défini dans la revendication 24.

26. Procédé pour détecter par voie immunologique un antigène du virus de l'hépatite non-B, non-C, non-G dans lequel on utilise l'anticorps tel que défini dans la revendication 25.

27. ADN isolé du virus de l'hépatite non-B, non-C, non-G, consistant en la séquence de SEQ ID NO : 62.

28. Polynucléotide ayant une séquence de nucléotide complémentaire de la longueur complète d'une séquence de nucléotides de l'ADN tel que défini dans la revendication 27.

29. Procédé pour détecter un gène du virus de l'hépatite non-B, non-C, non-G dans lequel :
(a) une PCR est effectuée en utilisant une paire d'amorces oligonucléotidiques ; et
(b) la longueur de chaque amorce oligonucléotidique est entre 6 et 50 bases ; et
(c) chaque amorce oligonucléotidique est capable d'une hybridation avec :
(i) l'ADN défini dans la revendication 27 ; ou
(ii) le complément de l'ADN défini dans la revendication 27.

30. Procédé selon la revendication 29 dans lequel la PCR est effectuée en utilisant une paire d'amorces consistant en :
(a) l'oligonucléotide de SEQ ID NO : 57, et l'oligonucléotide de SEQ ID NO : 60 ; ou
(b) l'oligonucléotide de SEQ ID NO : 57, et l'oligonucléotide de SEQ ID NO : 61.

31. Polypeptide isolé du virus de l'hépatite non-B, non-C, non-G consistant en la séquence d'acides aminés de SEQ ID NO : 63 ou 64.

32. Vecteur d'expression de gène recombinant comprenant une séquence de nucléotides codant pour la totalité ou une partie antigène de la séquence d'acides aminés de SEQ ID NO : 63 ou 64.

33. Cellule transformante, contenant une séquence de nucléotides codant pour la totalité ou une partie antigène de la séquence d'acides aminés de SEQ ID NO : 63 ou 64.

34. Antigène peptidique du virus de l'hépatite non-B, non-C, non-G ou fragment antigénique de celui-ci exprimé par la cellule transformante telle que définie dans la revendication 33.

35. Procédé pour la production d'un antigène peptidique du virus de l'hépatite non-B, non-C, non-G comprenant :
(a) la mise en culture de la cellule transformante telle que définie dans la revendication 33 dans des conditions qui permettent que le peptide antigène du virus de l'hépatite non-B, non-C, non-G soit exprimé ; et
(b) la récolte du peptide exprimé.

36. Procédé pour détecter par voie immunologique un anticorps du virus de l'hépatite non-B, non-C, non-G en utilisant le polypeptide tel que défini dans la revendication 31 en tant qu'antigène.

37. Procédé pour la production d'un anticorps contre un virus de l'hépatite non-B, non-C, non-G, qui comprend :
(a) l'immunisation d'un animal avec le polypeptide tel que défini dans la revendication 31 en tant qu'immunogène ; et
(b) l'isolement de l'anticorps.

38. Anticorps isolé pouvant être obtenu par le procédé tel que défini dans la revendication 37.

39. Procédé pour détecter par voie immunologique un antigène du virus de l'hépatite non-B, non-C, non-G en utilisant l'anticorps tel que défini dans la revendication 38.


### Sequence Listing

<110> TAMURA, Ryoji
   <120> Non-B, Non-C, Non-G Hepatitis Virus Gene, Polynucleotide, Polypeptide, Virus Particle, Method for Isolating Virus Particle, and Method for Detecting Virus
   <130> OP763-PCT
   <150> JP 10-82962
   <151> 1998-03-13
   <150> JP 9-314196
   <151> 1997-10-09
   <150> JP 9-233246
   <151> 1997-07-25
   <160> 64
<210> 1
   <211> 3739
   <212> DNA
   <213> non-B, non-C, non-G hepatitis virus
   <220>
   <221> CDS
   <222> 589..2898
   <220>
   <221> CDS
   <222> 107..712
<400> 1
   attttgctac gtcactaacc acgtgacacc cacaggccaa ccgaatgcta tgtcatccat     60
   ttcctgggcc gggtctacgt cctcatataa gtaagtgcac ttccgaatgg ctgagttttc    120
   cacgcccgtc cgcagcggtg aagccacgga gggagatctc cgcgtcccga gggcgggtgc    180
   cgaaggtgag tttacacacc gaagtcaagg ggcaattcgg gctcgggact ggccgggcta    240
   tgggcaaggc tctgaaaaaa gcatgtttat tggcaggcat tacagaaaga aaagggcgct    300
   gtcactgtgt gctgtgcgaa caacaaagaa ggcttgcaaa ctactaatag taatgtggac    360
   cccacctcgc aatgatcaac actaccttaa ctggcaatgg tactcaagta tacttagctc    420
   ccacgctgct atgtgcgggt gtcccgacgc tgtcgctcat tttaatcatc ttgcttctgt    480
   gcttcgtgcc ccgcaaaacc caccccctcc cggtccccag cgaaacctgc ccctccgacg     540
   gctgccggct ctcccggctg cgccagaggc gcccggagat agagcaccat ggcctatggc    600
   tggtggcgcc gaaggagaag acggtggcgc aggtggagac gcagaccatg gaggcgccgc    660
   tggaggaccc gaagacgcag acctgctaga cgccgtggcc gccgcagaaa cgtaaggaga    720
   cgccgcagag gagggaggtg gaggaggaga tataggagat ggaaaagaaa gggcaggcgc    780
   agaaaaaaag ctaaaataat aataagacaa tggcaaccaa actacagaag gagatgtaac    840
   atagtaggct acatccctgt actaatatgt ggcgaaaata ctgtcagcag aaactatgcc    900
   acacactcag acgataccaa ctacccagga ccctttgggg ggggtatgac tacagacaaa    960
   tttactttaa gaattctgta tgacgagtac aaaaggttta tgaactactg gacagcatct 1020
   aacgaagacc tagacctttg tagatatcta ggagtaaacc tgtacttttt cagacaccca 1080
   gatgtagatt ttatcataaa aattaatacc atgcctcctt ttctagacac agaactcaca 1140
   gcccctagca tacacccagg catgctagcc ctagacaaaa gagcaagatg gatacctagc 1200
   ttaaaatcta gaccgggaaa aaaacactat attaaaataa gagtaggggc accaagaatg 1260
   ttcactgata aatggtaccc ccaaacagat ctttgtgaca tggtgcttct aactgtctat 1320
   gcaaccgcag cggatatgca atatccgttc ggctcaccac taactgactc tgtggttgtg 1380
   aacttccagg ttctgcaatc catgtatgat aaaacaatta gcatattacc agacgaaaaa 1440
   tcacaaagag aaattctact taacaagata gcaagttaca ttccctttta taataccaca 1500
   caaactatag cccaattaaa gccatttata gatgcaggca atgtaacatc aggcgcaaca 1560
   gcaacaacat gggcatcata cataaacaca accaaattta ctacagcaac cacaacaact 1620
   tatgcatatc caggcaccaa cagaccccca gtaactatgt taacctgtaa tgactcctgg 1680
   tacagaggaa cagtatataa cacacaaatt caacagttac caataaaagc agctaaatta 1740
   tacttagagg caacaaaaac cttgctagga aacaccttca caaatgagga ctacacacta 1800
   gaatatcatg gaggactgta cagctcaata tggctatccc ctggtagatc ttactttgaa 1860
   acaacaggag catatacaga cataaagtac aatccattca cagacagagg agaaggcaac 1920
   atgttatgga tagactggct aagcaaaaaa aacatgaact atgacaaagt acaaagtaaa 1980
   tgcttaatat cagacctacc tctatgggca gcagcatatg gatatgtaga attttgtgca 2040
   aaaagtacag gagaccaaaa catacacatg aatgccaggc tactaataag aagtcccttt 2100
   acagacccac aactactagt acacacagac cccacaaaag gctttgttcc ttactcttta 2160
   aactttggaa atggtaaaat gccaggaggt agtagtaatg tgcctattag aatgagagct 2220
   aaatggtatc caacattatt tcaccagcaa gaagtactag aggccttagc acagtcaggc 2280
   ccctttgcat accactcaga cattaaaaaa gtatctctgg gtatgaaata ccgttttaag 2340
   tggatctggg gtggaaaccc cgttcgccaa caggttgtta gaaatccctg caaagaaacc 2400
   cactcctcgg gcaatagagt ccctagaagc ttacaaatcg ttgacccgaa atacaactca 2460
   ccggaactca cattccatac ctgggacttc agacgtggcc tctttggccc gaaagctatt 2520
   cagagaatgc aacaacaacc aacaactact gacatttttt cagcaggccg caagagaccc 2580
   aggagggaca ccgaggtgta ccactccagc caagaagggg agcaaaaaga aagcttactt 2640
   ttccccccag tcaagctcct cagacgagtc cccccgtggg aagactcgca gcaggaggaa 2700
   agcgggtcgc aaagctcaga ggaagagacg cagaccgtct cccagcagct caagcagcag 2760
   ctgcagcaac agcgaatcct gggagtcaaa ctcagactcc tgttcaacca agtccaaaaa   2820
   atccaacaaa atcaagatat caaccctacc ttgttaccaa ggggggggga tctagcatcc   2880
   ttatttcaaa tagcaccata aacatgtttg gtgaccccaa accttacaac ccttccagta 2940
   atgactggaa agaggagtac gaggcctgta gaatatggga cagacccccc agaggcaacc 3000
   taagagatac ccctttctac ccctgggccc ccaaggaaaa ccagtaccgt gtaaacttta 3060
   aacttggatt ccaataaagc taggccgtgg gactttcact tgtcggtgtc tgcttataaa 3120
   agtaactaag cactccgagc gaagcgagga gtgcgaccct tgggggctca acgccttcgg 3180
   agccgcgcgc tacgccttcg gctgcgcgcg gcacctcaga cccccgctcg tgctgacacg 3240
   ctcgcgcgtg tcagaccact tcgggctcgc gggggtcggg aaatttacta aacagactcc 3300
   gagttgccat tggactcagg agctatgaat cagtaacgaa agtgagtggg gccagacttc 3360
   gccataaggc ctttatcttc ttgccatttg tcagtaacag gggtcgccat agacttcggc 3420
   ctccacttta ccttgtaaaa actaccaaaa tggccgttcc agtgacgtca cagccgccat 3480
   tttaagtagc tgacgtcaag gattgacgta aaggttaaag gtcatcctcg gcggaagcta 3540
   cacaaaatgg tggacaacat cttccgggtc aaaggttgtg cgtacgtcac aagtcacgtg 3600
   gaggggaccc gctgtaaccc ggaagtaggc cccgtcacgt gacttaccac gtgtgtacac 3660
   gtcaccgccg ccattttgtt ttacaaaatg gctgacttcc ttcctctttt ttgaaaaaag 3720
   gcgccaaaaa accgtcggc 3739
<210> 2
   <211> 20
   <212> DNA
   <213> Artificial Sequence
   <220>
   <223> Synthetic DNA
<400> 2
   gcagcagcat atggatatgt 20
<210> 3
   <211> 20
   <212> DNA
   <213> Artificial Sequence
   <220>
   <223> Synthetic DNA
<400> 3
   tgactgtgct aaagcctcta 20
<210> 4
   <211> 20
   <212> DNA
   <213> Artificial Sequence
   <220>
   <223> Synthetic DNA
<400> 4
   catacacatg aatgccaggc 20
<210> 5
   <211> 20
   <212> DNA
   <213> Artificial Sequence
   <220>
   <223> Synthetic DNA
<400> 5
   gtacttcttg ctggtgaaat 20
<210> 6
   <211> 24
   <212> DNA
   <213> Artificial Sequence
   <220>
   <223> Synthetic DNA
<400> 6
   acagacagag gagaaggcaa catg 24
<210> 7
   <211> 24
   <212> DNA
   <213> Artificial Sequence
   <220>
   <223> Synthetic DNA
<400> 7
   ggcaacatgy trtggataga ctgg 24
<210> 8
   <211> 25
   <212> DNA
   <213> Artificial Sequence
   <220>
   <223> Synthetic DNA
<400> 8
   ctggcatttt accatttcca aagtt 25
<210> 9
   <211> 770
   <212> PRT
   <213> non-B, non-C, non-G hepatitis virus
<400> 9
   Met Ala Tyr Gly Trp Trp Arg Arg Arg Arg Arg Arg Trp Arg Arg
     1               5                  10                  15
   Trp Arg Arg Arg Pro Trp Arg Arg Arg Trp Arg Thr Arg Arg Arg
                  20                  25                  30
   Arg Pro Ala Arg Arg Arg Gly Arg Arg Arg Asn Val Arg Arg Arg
                   35                  40                  45
   Arg Arg Gly Gly Arg Trp Arg Arg Arg Tyr Arg Arg Trp Lys Arg
                   50                  55                  60
   Lys Gly Arg Arg Arg Lys Lys Ala Lys Ile Ile Ile Arg Gln Trp
                   65                  70                  75
   Gln Pro Asn Tyr Arg Arg Arg Cys Asn Ile Val Gly Tyr Ile Pro
                   80                  85                  90
   Val Leu Ile Cys Gly Glu Asn Thr Val Ser Arg Asn Tyr Ala Thr
                   95                  100                 105
   His Ser Asp Asp Thr Asn Tyr Pro Gly Pro Phe Gly Gly Gly Met
                   110                 115                 120
   Thr Thr Asp Lys Phe Thr Leu Arg Ile Leu Tyr Asp Glu Tyr Lys
                   125                 130                 135
   Arg Phe Met Asn Tyr Trp Thr Ala Ser Asn Glu Asp Leu Asp Leu
                   140                 145                 150
   Cys Arg Tyr Leu Gly Val Asn Leu Tyr Phe Phe Arg His Pro Asp
                   155                 160                 165
   Val Asp Phe Ile Ile Lys Ile Asn Thr Met Pro Pro Phe Leu Asp
                   170                 175                 180
   Thr Glu Leu Thr Ala Pro Ser Ile His Pro Gly Met Leu Ala Leu
                   185                 190                 195
   Asp Lys Arg Ala Arg Trp Ile Pro Ser Leu Lys Ser Arg Pro Gly
                   200                 205                 210
   Lys Lys His Tyr Ile Lys Ile Arg Val Gly Ala Pro Arg Met Phe
                   215                 220                 225
   Thr Asp Lys Trp Tyr Pro Gln Thr Asp Leu Cys Asp Met Val Leu
                   230                 235                 240
   Leu Thr Val Tyr Ala Thr Ala Ala Asp Met Gln Tyr Pro Phe Gly
                   245                 250                 255
   Ser Pro Leu Thr Asp Ser Val Val Val Asn Phe Gln Val Leu Gln
                   260                 265                 270
   Ser Met Tyr Asp Lys Thr Ile Ser Ile Leu Pro Asp Glu Lys Ser
                   275                 280                 285
   Gln Arg Glu Ile Leu Leu Asn Lys Ile Ala Ser Tyr Ile Pro Phe
                   290                 295                 300
   Tyr Asn Thr Thr Gln Thr Ile Ala Gln Leu Lys Pro Phe Ile Asp
                   305                 310                 315
   Ala Gly Asn Val Thr Ser Gly Ala Thr Ala Thr Thr Trp Ala Ser
                   320                 325                 330
   Tyr Ile Asn Thr Thr Lys Phe Thr Thr Ala Thr Thr Thr Thr Tyr
                   335                 340                 345
   Ala Tyr Pro Gly Thr Asn Arg Pro Pro Val Thr Met Leu Thr Cys
                   350                 355                 360
   Asn Asp Ser Trp Tyr Arg Gly Thr Val Tyr Asn Thr Gln Ile Gln
   365 370 375
   Gln Leu Pro Ile Lys Ala Ala Lys Leu Tyr Leu Glu Ala Thr Lys
   380 385 390
   Thr Leu Leu Gly Asn Thr Phe Thr Asn Glu Asp Tyr Thr Leu Glu
   395 400 405
   Tyr His Gly Gly Leu Tyr Ser Ser Ile Trp Leu Ser Pro Gly Arg
   410 415 420
   Ser Tyr Phe Glu Thr Thr Gly Ala Tyr Thr Asp Ile Lys Tyr Asn
   425 430 435
   Pro Phe Thr Asp Arg Gly Glu Gly Asn Met Leu Trp Ile Asp Trp
   440 445 450
   Leu Ser Lys Lys Asn Met Asn Tyr Asp Lys Val Gln Ser Lys Cys
   455 460 465
   Leu Ile Ser Asp Leu Pro Leu Trp Ala Ala Ala Tyr Gly Tyr Val
   470 475 480
   Glu Phe Cys Ala Lys Ser Thr Gly Asp Gln Asn Ile His Met Asn
   485 490 495
   Ala Arg Leu Leu Ile Arg Ser Pro Phe Thr Asp Pro Gln Leu Leu
   500 505 510
   Val His Thr Asp Pro Thr Lys Gly Phe Val Pro Tyr Ser Leu Asn
   515 520 525
   Phe Gly Asn Gly Lys Met Pro Gly Gly Ser Ser Asn Val Pro Ile
   530 535 540
   Arg Met Arg Ala Lys Trp Tyr Pro Thr Leu Phe His Gln Gln Glu
   545 550 555
   Val Leu Glu Ala Leu Ala Gln Ser Gly Pro Phe Ala Tyr His Ser
   560 565 570
   Asp Ile Lys Lys Val Ser Leu Gly Met Lys Tyr Arg Phe Lys Trp
   575 580 585
   Ile Trp Gly Gly Asn Pro Val Arg Gln Gln Val Val Arg Asn Pro
   590 595 600
   Cys Lys Glu Thr His Ser Ser Gly Asn Arg Val Pro Arg Ser Leu
   605 610 615
   Gln Ile Val Asp Pro Lys Tyr Asn Ser Pro Glu Leu Thr Phe His
   620 625 630
   Thr Trp Asp Phe Arg Arg Gly Leu Phe Gly Pro Lys Ala Ile Gln
   635 640 645
   Arg Met Gln Gln Gln Pro Thr Thr Thr Asp Ile Phe Ser Ala Gly
   650 655 660
   Arg Lys Arg Pro Arg Arg Asp Thr Glu Val Tyr His Ser Ser Gln
   665 670 675
   Glu Gly Glu Gln Lys Glu Ser Leu Leu Phe Pro Pro Val Lys Leu
                   680                 685                 690
   Leu Arg Arg Val Pro Pro Trp Glu Asp Ser Gln Gln Glu Glu Ser
                   695                 700                 705
   Gly Ser Gln Ser Ser Glu Glu Glu Thr Gln Thr Val Ser Gln Gln
                   710                 715                 720
   Leu Lys Gln Gln Leu Gln Gln Gln Arg Ile Leu Gly Val Lys Leu
                   725                 730                 735
   Arg Leu Leu Phe Asn Gln Val Gln Lys Ile Gln Gln Asn Gln Asp
                   740                 745                 750
   Ile Asn Pro Thr Leu Leu Pro Arg Gly Gly Asp Leu Ala Ser Leu
                   755                 760                 765
   Phe Gln Ile Ala Pro
                   770
<210> 10
   <211> 202
   <212> PRT
   <213> non-B, non-C, non-G hepatitis virus
<400> 10
   Met Ala Glu Phe Ser Thr Pro Val Arg Ser Gly Glu Ala Thr Glu
   1               5                   10                  15
   Gly Asp Leu Arg Val Pro Arg Ala Gly Ala Glu Gly Glu Phe Thr
                   20                  25                  30
   His Arg Ser Gln Gly Ala Ile Arg Ala Arg Asp Trp Pro Gly Tyr
                   35                  40                  45
   Gly Gln Gly Ser Glu Lys Ser Met Phe Ile Gly Arg His Tyr Arg
                   50                  55                  60
   Lys Lys Arg Ala Leu Ser Leu Cys Ala Val Arg Thr Thr Lys Lys
                   65                  70                  75
   Ala Cys Lys Leu Leu Ile Val Met Trp Thr Pro Pro Arg Asn Asp
                   80                  85                  90
   Gln His Tyr Leu Asn Trp Gln Trp Tyr Ser Ser Ile Leu Ser Ser
                   95                  100                 105
   His Ala Ala Met Cys Gly Cys Pro Asp Ala Val Ala His Phe Asn
                   110                 115                 120
   His Leu Ala Ser Val Leu Arg Ala Pro Gln Asn Pro Pro Pro Pro
                   125                 130                 135
   Gly Pro Gln Arg Asn Leu Pro Leu Arg Arg Leu Pro Ala Leu Pro
                   140                 145                 150
   Ala Ala Pro Glu Ala Pro Gly Asp Arg Ala Pro Trp Pro Met Ala
                   155                 160                 165
   Gly Gly Ala Glu Gly Glu Asp Gly Gly Ala Gly Gly Asp Ala Asp
                   170                 175                 180
   His Gly Gly Ala Ala Gly Gly Pro Glu Asp Ala Asp Leu Leu Asp
                   185                 190                 195
   Ala Val Ala Ala Ala Glu Thr
                   200
<210> 11
   <211> 500
   <212> DNA
   <213> non-B, non-C, non-G hepatitis virus
<400> 11
   gatcttactt tgaaacaaca ggagcatata cagacataaa gtacaatcca ttcacagaca 60
   gaggagaagg caacatgtta tggatagact ggctaagcaa aaaaaacatg aactatgaca 120
   aagtacaaag taaatgctta atatcagacc tacctctatg ggcagcagca tatggatatg 180
   tagaattttg tgcaaaaagt acaggagacc aaaacataca catgaatgcc aggctactaa 240
   taagaagtcc ctttacagac ccacaactac tagtacacac agaccccaca aaaggctttg 300
   ttccttactc tttaaacttt ggaaatggta aaatgccagg aggtagtagt aatgtgccta 360
   ttagaatgag agctaaatgg tatccaacat tatttcacca gcaagaagta ctagaggcct 420
   tagcacagtc aggccccttt gcataccact cagacattaa aaaagtatct ctgggtatga 480
   aataccgttt taagtggatc 500
<210> 12
   <211> 41
   <212> DNA
   <213> Artificial Sequence
   <220>
   <223> Synthetic DNA
<400> 12
   aaggatccgt cgacatcgat aatacggggg gggggggggg g 41
<210> 13
   <211> 41
   <212> DNA
   <213> Artificial Sequence
   <220>
   <223> Synthetic DNA
<400> 13
   aaggatccgt cgacatcgat aatacgaaaa aaaaaaaaaa a 41
<210> 14
   <211> 41
   <212> DNA
   <213> Artificial Sequence
   <220>
   <223> Synthetic DNA
<400> 14
   aaggatccgt cgacatcgat aatacgtttt tttttttttt t 41
<210> 15
   <211> 20
   <212> DNA
   <213> Artificial Sequence
   <220>
   <223> Synthetic DNA
<400> 15
   aaggatccgt cgacatcgat aatacgcccc cccccccccc c 41
<210> 16
   <211> 803
   <212> DNA
   <213> non-B, non-C, non-G hepatitis virus
<400> 16
   cctacttgag aatataacaa gttacattcc cttttataat accacacaaa ctatagccca 60
   attaaagcca tttatagatg caggcaatgt aacatcaggc gcaacagcaa caacatgggc 120
   atcatacata aacacaacca aatttactac agcaaccaca acaacttatg catatccagg 180
   caccaacaga cccccagtaa ctatgttaac ctgtaatgac tcctggtaca gaggaacagt 240
   atataacaca caaattcaac agttaccaat aaaagcagct aaattatact tagaggcaac 300
   aaaaaccttg ctaggaaaca ccttcacaaa tgaggactac acactagaat atcatggagg 360
   actgtacagc tcaatatggc tatcccctgg tagatcttac tttgaaacaa caggagcata 420
   tacagacata aagtacaatc cattcacaga cagaggagaa ggcaacatgt tatggataga 480
   ctggctaagc aaaaaaaaca tgaactatga caaagtacaa agtaaatgct taatatcaga 540
   cctacctcta tgggcagcag catatggata tgtagaattt tgtgcaaaaa gtacaggaga 600
   ccaaaacata cacatgaatg ccaggctact aataagaagt ccctttacag acccacaact 660
   actagtacac acagacccca caaaaggctt tgttccttac tctttaaact ttggaaatgg 720
   taaaatgcca ggaggtagta gtaatgtgcc tattagaatg agagctaaat ggtatccaac 780
   attatttcac cagcaagaag tac 803
<210> 17
   <211> 1205
   <212> DNA
   <213> non-B, non-C, non-G hepatitis virus
<400> 17
   catacacatg aatgccaggc tactaataag aagtcccttt acagacccac aactactagt 60
   acacacagac cccacaaaag gctttgttcc ttactcttta aactttggaa atggtaaaat 120
   gccaggaggt agtagtaatg tgcctattag aatgagagct aaatggtatc caacattatt 180
   tcaccagcaa gaagtactag aggccttagc acagtcaggc ccctttgcat accactcaga 240
   cattaaaaaa gtatctctgg gtatgaaata ccgttttaag tggatctggg gtggaaaccc 300
   cgttcgccaa caggttgtta gaaatccctg caaagaaacc cactcctcgg gcaatagagt 360
   ccctagaagc ttacaaatcg ttgacccgaa atacaactca ccggaactca cattccatac 420
   ctgggacttc agacgtggcc tctttggccc gaaagctatt cagagaatgc aacaacaacc 480
   aacaactact gacatttttt cagcaggccg caagagaccc aggagggaca ccgaggtgta 540
   ccactccagc caagaagggg agcaaaaaga aagcttactt ttccccccag tcaagctcct 600
   cagacgagtc cccccgtggg aagactcgca gcaggaggaa agcgggtcgc aaagctcaga 660
   ggaagagacg cagaccgtct cccagcagct caagcagcag ctgcagcaac agcgaatcct 720
   gggagtcaaa ctcagactcc tgttcaacca agtccaaaaa atccaacaaa atcaagatat 780
   caaccctacc ttgttaccaa ggggggggga tctagcatcc ttatttcaaa tagcaccata 840
   aacatgtttg gtgaccccaa accttacaac ccttccagta atgactggaa agaggagtac 900
   gaggcctgta gaatatggga cagacccccc agaggcaacc taagagatac ccctttctac 960
   ccctgggccc ccaaggaaaa ccagtaccgt gtaaacttta aacttggatt ccaataaagc 1020
   taggccgtgg gactttcact tgtcggtgtc tgcttataaa agtaactaag cactccgagc 1080
   gaagcgagga gtgcgaccct tgggggctca acgccttcgg agccgcgcgc tacgccttcg 1140
   gctgcgcgcg gcacctcaga cccccgctcg tgctgacacg ctcgcgcgtg tcagaccact 1200
   tcggg 1205
<210> 18
   <211> 20
   <212> DNA
   <213> Artificial Sequence
   <220>
   <223> Synthetic DNA
<400> 18
   ggtgcctgga tatgcataag 20
<210> 19
   <211> 20
   <212> DNA
   <213> Artificial Sequence
   <220>
   <223> Synthetic DNA
<400> 19
   tgcccatgtt gttgctgttg 20
<210> 20
   <211 32
   <212> DNA
   <213> Artificial Sequence
   <220>
   <223> Synthetic DNA
   <220>
   <221> misc_difference
   <222> 27..31
   <223> n=a, t, g or c
<400> 20
   aaggatccgt cgacatcgat aatacgnnnn ng 32
<210> 21
   <211> 631
   <212> DNA
   <213> non-B, non-C, non-G hepatitis virus
<400> 21
   tatgactaca gacaaattta ctttaagaat tctgtatgac gagtacaaaa ggtttatgaa    60
   ctactggaca gcatctaacg aagacctaga cctttgtaga tatctaggag taaacctgta   120
   ctttttcaga cacccagatg tagattttat cataaaaatt aataccatgc ctccttttct   180
   agacacagaa ctcacagccc ctagcataca cccaggcatg ctagccctag acaaaagagc   240
   aagatggata cctagcttaa aatctagacc gggaaaaaaa cactatatta aaataagagt   300
   aggggcacca agaatgttca ctgataaatg gtacccccaa acagatcttt gtgacatggt   360
   gcttctaact gtctatgcaa ccgcagcgga tatgcaatat ccgttcggct caccactaac   420
   tgactctgtg gttgtgaact tccaggttct gcaatccatg tatgataaaa caattagcat   480
   attaccagac gaaaaatcac aaagagaaat tctacttaac aagatagcaa gttacattcc   540
   cttttataat accacacaaa ctatagccca attaaagcca tttatagatg caggcaataa   600
   accatcaggc acaacagcaa caacatgggc a                                  631
<210> 22
   <211> 20
   <212> DNA
   <213> Artificial Sequence
   <220>
   <223> Synthetic DNA
<400> 22
   tcacttgtcg gtgtctgctt 20
<210> 23
   <211> 20
   <212> DNA
   <213> Artificial Sequence
   <220>
   <223> Synthetic DNA
<400> 23
   ctaagcactc cgagcgtagc 20
<210> 24
   <211> 614
   <212> DNA
   <213> non-B, non-C, non-G hepatitis virus
<400> 24
   ctaagcactc cgagcgtagc gaggagtgcg acccttgggg gctcaacgcc ttcggagcca 60
   cgcgctacgc cttcggctgc gcgcggcacc tcagaccccc gctcgtgctg acacgctcgc 120
   gcgtgtcaga ccacttcggg ctcgcggggg tcgggaaatt tactaaacag actccgagtt 180
   gccattggac tcaggagcta tgaatcagta acgaaagtga gtggggccag acttcgccat 240
   aaggccttta tcttcttgcc atttgtcagt aacaggggtc gccatagact tcggcctcca 300
   ctttaccttg taaaaactac caaaatggcc gttccagtga cgtcacagcc gccattttaa 360
   gtagctgacg tcaaggattg acgtaaaggt taaaggtcat cctcggcgga agctacacaa 420
   aatggtggac aacatcttcc gggtcaaagg ttgtgcgtac gtcacaagtc acgtggaggg 480
   gacccgctgt aacccggaag taggccccgt cacgtgactt accacgtgtg tacacgtcac 540
   cgccgccatt ttgttttaca aaatggctga cttccttcct cttttttgaa aaaaggcgcc 600
   aaaaaaccgt cggc 614
<210> 25
   <211> 302
   <212> DNA
   <213> non-B, non-C, non-G hepatitis virus
<400> 25
   ccgcagagga gggaggtgga ggaggagata taggagatgg aaaagaaagg gcaggcgcag 60
   aaaaaaagct aaaataataa taagacaatg gcaaccaaac tacagaagga gatgtaacat 120
   agtaggctac atccctgtac taatatgtgg cgaaaatact gtcagcagaa actatgccac 180
   acactcagac gataccaact acccaggacc ctttgggggg ggtatgacta cagacaaatt 240
   tactttaaga attctgtatg acgagtacaa aaggtttatg aactactgga cagcatctaa 300
   cg 302
<210> 26
   <211> 831
   <212> DNA
   <213> non-B, non-C, non-G hepatitis virus
<400> 26
   attttgctac gtcactaacc acgtgacacc cacaggccaa ccgaatgcta tgtcatccat 60
   ttcctgggcc gggtctacgt cctcatataa gtaagtgcac ttccgaatgg ctgagttttc 120
   cacgcccgtc cgcagcggtg aagccacgga gggagatctc cgcgtcccga gggcgggtgc 180
   cgaaggtgag tttacacacc gaagtcaagg ggcaattcgg gctcgggact ggccgggcta 240
   tgggcaaggc tctgaaaaaa gcatgtttat tggcaggcat tacagaaaga aaagggcgct 300
   gtcactgtgt gctgtgcgaa caacaaagaa ggcttgcaaa ctactaatag taatgtggac 360
   cccacctcgc aatgatcaac actaccttaa ctggcaatgg tactcaagta tacttagctc 420
   ccacgctgct atgtgcgggt gtcccgacgc tgtcgctcat tttaatcatc ttgcttctgt 480
   gcttcgtgcc ccgcaaaacc caccccctcc cggtccccag cgaaacctgc ccctccgacg 540
   gctgccggct ctcccggctg cgccagaggc gcccggagat agagcaccat ggcctatggc 600
   tggtggcgcc gaaggagaag acggtggcgc aggtggagac gcagaccatg gaggcgccgc 660
   tggaggaccc gaagacgcag acctgctaga cgccgtggcc gccgcagaaa cgtaaggaga 720
   cgccgcagag gagggaggtg gaggaggaga tataggagat ggaaaagaaa gggcaggcgc 780
   agaaaaaaag ctaaaataat aataagacaa tggcaaccaa actacagaag g 831
<210> 27
   <211> 20
   <212> DNA
   <213> Artificial Sequence
   <220>
   <223> . Synthetic DNA.
<400> 27
   atctacatct gggtgtctga 20
<210> 28
   <211> 20
   <212> DNA
   <213> Artificial Sequence
   <220>
   <223> Synthetic DNA
<400> 28
   cgttagatgc tgtccagtag
<210> 29
   <211> 20
   <212> DNA
   <213> Artificial Sequence
   <220>
   <223> Synthetic DNA
<400> 29
   cgccacatat tagtacaggg 20
<210> 30
   <211> 20
   <212> DNA
   <213> Artificial Sequence
   <220>
   <223> Synthetic DNA
<400> 30
   ccttctgtag tttggttgcc 20
<210> 31
   <211> 467
   <212> DNA
   <213> non-B, non-C, non-G hepatitis virus
<400> 31
   ccatgcctcc ttttctagac acagaactca cagcccctag catacaccca ggcatgctag 60
   ccctagacaa aagagcaaga tggataccta gcttaaaatc tagaccggga aaaaaacact 120
   atattaaaat aagagtaggg gcaccaaaaa tgttcactga taaatggtac ccccaaacag 180
   atctttgtga catggtgctt ctaactgtct atgcaaccgc agcggatatg caatatccgt 240
   tcggctcacc actaactgac tctgtggttg tgaacttcca ggttctgcaa tccatgtatg 300
   ataaaacaat tagcatatta ccagacgaaa aagaagaaag agcaaatcta cttaagcaga 360
   taacaagtta cattcccttt tataatacca cacaaactat agcccaatta aagccattta 420
   tagatgcagg caatgtaaca tcaagcacaa cagcaacaac atgggca 467
<210> 32
   <211> 748
   <212> DNA
   <213> non-B, non-C, non-G hepatitis virus
<400> 32
   tcactaacca cgtgacaccc acaggccaac cgaatgctat gtcatccatt tcctgggccg 60
   ggtctacgtc ctcatataag taagtgcact tccgaatggc tgagttttcc acgcccgtcc 120
   gcagcggtga agccacggag ggagatctcc gcgtcccgag ggcgggtgcc gaaggtgagt 180
   ttacacaccg aagtcaaggg gcaattcggg ctcgggactg gccgggctat gggcaaggct 240
   ctgaaaaaag catgtttatt ggcaggcatt acagaaagaa aagggcgctg tcactgtgtg 300
   ctgtgcgaac aacaaagaag gcttgcaaac tactaatagt aatgtggacc ccacctcgca    360
   atgatcaaca ctaccttaac tggcaatggt actcaagtat acttagctcc cacgctgcta    420
   tgtgcgggtg tcccgacgct gtcgctcatt ttaatcatct tgcttctgtg cttcgtgccc    480
   cgcaaaaccc accccctccc ggtccccagc gaaacctgcc cctccgacgg ctgccggctc    540
   tcccggctgc gccagaggcg cccggagata gagcaccatg gcctatggct ggtggcgccg    600
   aaggagaaga cggtggcgca ggtggagacg cagaccatgg aggcgccgct ggaggacccg    660
   aagacgcaga cctgctagac gccgtggccg ccgcagaaac gtaaggagac gccgcagagg    720
   agggaggtgg aggaggagat ataggaga                                       748
<210> 33
   <211> 197
   <212> DNA
   <213> non-B, non-C, non-G hepatitis virus
<400> 33
   aaagtaacta agcactccga gcgaagcgag gagtgcgacc cttgggggct caacgccttc     60
   ggagccgcgc gctacgcctt cggctgcgcg cggcacctca gacccccgct cgtgctgaca    120
   cgctcgcgcg tgtcagacca cttcgggctc gcgggggtcg ggaaatttac taaacagact    180
   ccgagttgcc attggac                                                   197
<210> 34
   <211> > 20
   <212> DNA .
   <213> Artificial Sequence
   <220>
   <223> Synthetic DNA
<400> 34
   tgcccatgtt gttgctgttg 20
<210> 35
   <211> 20
   <212> DNA
   <213> Artificial Sequence
   <220>
   <223> Synthetic DNA
<400> 35
   ccatgcctcc ttttctagac 20
<210> 36
   <211> 20
   <212> DNA
   <213> Artificial Sequence
   <220>
   <223> Synthetic DNA
<400> 36
   tctcctatat ctcctcctcc 20
<210> 37
   <211> 20
   <212> DNA
   <213> Artificial Sequence
   <220>
   <223> Synthetic DNA
<400> 37
   tcactaacca cgtgacaccc 20
<210> 38
   <211> 20
   <212> DNA
   <213> Artificial Sequence
   <220>
   <223> Synthetic DNA
<400> 38
   aaagtaacta agcactccga 20
<210> 39
   <211> 20
   <212> DNA
   <213> Artificial Sequence
   <220>
   <223> Synthetic DNA
<400> 39
   gtccaatggc aactcggagt 20
<210> 40
   <211> 20
   <212> DNA
   <213> Artificial Sequence
   <220>
   <223> Synthetic DNA
<400> 40
   caccaggagc atatacagac 20
<210> 41
   <211> 20
   <212> DNA
   <213> Artificial Sequence
   <220>
   <223> Synthetic DNA
<400> 41
   caaggtatgt tgcccgtttg 20
<210> 42
   <211> 20
   <212> DNA
   <213> Artificial Sequence
   <220>
   <223> Synthetic DNA
<400> 42
   cgaaccacgt aacaaatggc 20
<210> 43
   <211> 20
   <212> DNA
   <213> Artificial Sequence
   <220>
   <223> Synthetic DNA
<400> 43
   tgttgcccgt ttgtcctcta 20
<210> 44
   <211> 20
   <212> DNA
   <213> Artificial Sequence
   <220>
   <223> Synthetic DNA
<400> 44
   ggcactagta aactgagcca 20
   <210> 45
   <211> 222
   <212> DNA
   <213> non-B, non-C, non-G hepatitis virus
<400> 45
   ctaagyaaaa aaaacatgaa vtatgacaaa strcaaagta artgcytart akcagaccta   60
   ccwctrtggg cagcagcata tggwtatgta gaattytgyg cwaaaagyac aggagacvva  120
   aacagacaca tgaatgccag rctactaata agaagtccyt ttacagaccc vcarctavta  180
   gtacacacag accccacwaa aggctttgtw ccytaytctk ta                     222
<210> 46
   <211> 222
   <212> DNA
   <213> non-B, non-C, non-G hepatitis virus
<400> 46
   ctaacyaaar atrvctcasw rtaydvvaar vvacaragya aatgtctvat asvdracwtg   60
   ccyytrtgrg cctcwgtrta yggatwywcv gaatactgca gyaaagtwac aggagacwca  120
   aacagagarc acaactgyag atgtgthaty agaagcccct ayacagtrcc wcarctgyta  180
   gaycacaaca ayvscctyag rggwtaygtd ccvtayagya th                     222
<210> 47
   <211> 222
   <212> DNA
   <213> non-B, non-C, non-G hepatitis virus
<400> 47
   ctagtaaaga cagactctag atatgacaag acacgcagca aatgccttat agaaaaacta 60
   ccyctatggg cttcagtata cggktacgca gartactgcg ccaaggccac aggagactct 120
   aacagagaca tgaacgccag agtagttata aggagcccct acactacacc tcaaatgata 180
   gaycacaaca ayvscctcag rggwtaygtd ccvtayagyt th 222
<210> 48
   <211> 222
   <212> DNA
   <213> non-B, non-C, non-G hepatitis virus
<400> 48
   ctatcaaaat cagacgccct ttacaccaaa ggacaaagca aatgtgaaat atttgactta 60
   cccctctggg ccgccctaaa tggctacaca gaattctgct ccaaaagcac aggagacaca 120
   gcaggacacc taaatgccag actagtgata agatgcccat acacataccc catgctagta 180
   gaccactcaa acgacctaac aggctttgta ctgtacagca aa 222
<210> 49
   <211> 222
   <212> DNA
   <213> non-B, non-C, non-G hepatitis virus
<400> 49
   ctaagcaaat cagatgcagt atactctgaa aaacaaagca aatgtgctat atttgaccta 60
   ccactatggg cctccttctt tggatacgca gaattctgct ctaaaagcac aggagacaca 120
   gccagagcat acaacgccag agtatgtgtt agatgtccct acacagagcc acagctgcta 180
   aaccacaaca acccctctca ggggttcgtg ttttactcct ac 222
<210> 50
   <211> 222
   <212> DNA
   <213> non-B, non-C, non-G hepatitis virus
<400> 50
   ctgagcaaac cagacagcat atacgacccc tctaagagca aatgcctact aaaagacttt 60
   cccctgtggt gcatggtata cgggtacgca gactactgca gaaaggtcac aggagactca 120
   gccagactac tagactgcag agtatgtgtt agatgcccgt acacataccc tcagcttata 180
   aaacacaaca atgacaactg gggctttgtc ccctatagcg aa 222
<210> 51
   <211> 222
   <212> DNA
   <213> non-B, non-C, non-G hepatitis virus
<400> 51
   tgtcttaaaa acgataccac gttcaaagac acaccatcca gactacccgt aaaagacata   60
   cccctatggg cctgtttcat gggataccag gactactgct cgaaacactt tcatgacgaa  120
   ggcagaaaca aagaggccag agtcacaata atatccccat acacagaacc accactgacc  180
   tcgaaagaca accactcaat gggattcata ccatatgact at                     222
<210> 52
   <211> 222
   <212> DNA
   <213> non-B, non-C, non-G hepatitis virus
<400> 52
   tgcactaaaa atgacacaca attcagagat gtccccggca gactcaccat atcagacgtc   60
   cccctgtacg cggccctcct gggctacgag gactactgta tcaaatacta ccacgacaaa  120
   ggcctaccta aagaagtcag ggtcaccata cagtgtccat acacagatcc ccccctctat  180
   gacaaagaca acacagacat gggctaccta ccttatgact ac                     222
<210> 53
   <211> 225
   <212> DNA
   <213> non-B, non-C, non-G hepatitis virus
<400> 53
   ctcacwaaac caracacasa gtttgaccck gtacagtgca aatgygtmwt astagacatw   60
   ccmytgtggg ccgccttytt yggctaccca gactacatag aragccarct aggcccytty  120
   cargaycacg aractgtkgg catagtgttc gtgtgytgcc cctacactca rccrcccatg  180
   tacaarccwg gcarvrtaca aamtggctay gttttctatg acact                  225
<210> 54
   <211> 225
   <212> DNA
   <213> non-B, non-C, non-G hepatitis virus
<400> 54
   cacagcaaag taaacacaga cctcagagac agaggcatct actgcctact agaagacatg   60
   cccctgtggg ccatgacctt tggatacagt gactatgtga gcacacaact aggcccaaac  120
   gtggaccacg agactcaagg cctggtatgc agatctagtc cttacactga gccccccatg  180
   tatgacaaga ctaacccaaa cagtggctac gtagcatatg acaca                  225
<210> 55
   <211> 23
   <212> DNA
   <213> Artificial Sequence
   <220>
   <223> Synthetic DNA
<400> 55
   tggtggcgcc gaaggagaag acg 23
<210> 56
   <211> 20
   <212> DNA
   <213> Artificial Sequence
   <220>
   <223> Synthetic DNA
<400> 56
   gctcttatgt acctcctgcg 20
<210> 57
   <211> 20
   <212> DNA
   <213> Artificial Sequence
   <220>
   <223> Synthetic DNA
<400> 57
   tttgctacgt cactaaccac 20
<210> 58
   <211> 20
   <212> DNA
   <213> Artificial Sequence
   <220>
   <223> Synthetic DNA
<400> 58
   gtagaccatg aaacagcagg 20
<210> 59
   <211> 20
   <212> DNA
   <213> Artificial Sequence
   <220>
   <223> Synthetic DNA
<400> 59
   tcaaccacct atgtatgaca 20
<210> 60
   <211> 27
   <212> DNA
   <213> Artificial Sequence
   <220>
   <223> Synthetic DNA
<400> 60
   gccgacggtt ttttggcgcc ttttttc 27
<210> 61
   <211> 20
   <212> DNA
   <213> Artificial Sequence
   <220>
   <223> Synthetic DNA
<400> 61
   aaagaggaag gaagtcagcc 20
<210> 62
   <211> 3722
   <212> DNA
   <213> non-B, non-C, non-G hepatitis virus
   <220>
   <221> CDS
   <222> 590..2872
   <220>
   <221> CDS
   <222> 258.. 725
<400> 62
<210> 63
   <211> 761
   <212> PRT
   <213> non-B, non-C, non-G hepatitis virus
<400> 63
   Met Ala Tyr Trp Phe Arg Arg Trp Gly Trp Arg Pro Arg Arg Arg
   1 5 10 15
   Trp Arg Arg Trp Arg Arg Arg Arg Arg Arg Leu Pro Arg Arg Arg
   20 25 30
   Thr Arg Arg Ala Val Arg Gly Leu Gly Arg Arg Arg Lys Pro Arg
   35 40 45
   Val Arg Arg Arg Arg Arg Thr Arg Arg Arg Thr Tyr Arg Arg Gly
   50 55 60
   Trp Arg Arg Arg Arg Tyr Ile Arg Arg Gly Arg Arg Lys Lys Lys
   65 70 75
   Leu Ile Leu Thr Gln Trp Asn Pro Ala Ile Val Lys Arg Cys Asn
   80 85 90
   Ile Lys Gly Gly Leu Pro Ile Ile Ile Cys Gly Glu Pro Arg Ala
   95 100 105
   Ala Phe Asn Tyr Gly Tyr His Met Glu Asp Tyr Thr Pro Gln Pro
   110 115 120
   Phe Pro Phe Gly Gly Gly Met Ser Thr Val Thr Phe Ser Leu Lys
   125 130 135
   Ala Leu Tyr Asp Gln Tyr Leu Lys His Gln Asn Arg Trp Thr Phe
   140 145 150
   Ser Asn Asp Gln Leu Asp Leu Ala Arg Tyr Arg Gly Cys Lys Leu
   155 160 165
   Arg Phe Tyr Arg Ser Pro Val Cys Asp Phe Ile Val His Tyr Asn
   170 175 180
   Leu Ile Pro Pro Leu Lys Met Asn Gln Phe Thr Ser Pro Asn Thr
   185 190 195
   His Pro Gly Leu Leu Met Leu Ser Lys His Lys Ile Ile Ile Pro
   200 205 210
   Ser Phe Gln Thr Arg Pro Gly Gly Arg Arg Phe Val Lys Ile Arg
   215 220 225
   Leu Asn Pro Pro Lys Leu Phe Glu Asp Lys Trp Tyr Thr Gln Gln
   230 235 240
   Asp Leu Cys Lys Val Pro Leu Val Ser Ile Thr Ala Thr Ala Ala
   245 250 255
   Asp Leu Arg Tyr Pro Phe Cys Ser Pro Gln Thr Asn Asn Pro Cys
   260 265 270
   Thr Thr Phe Gln Val Leu Arg Lys Asn Tyr Asn Thr Val Ile Gly
   275 280 285
   Thr Ser Val Lys Asp Gln Glu Ser Thr Gln Asp Phe Glu Asn Trp
   290 295 300
   Leu Tyr Lys Thr Asp Ser His Tyr Gln Thr Phe Ala Thr Glu Ala
   305 310 315
   Gln Leu Gly Arg Ile Pro Ala Phe Asn Pro Asp Gly Thr Lys Asn
   320 325 330
   Thr Lys Gln Gln Ser Trp Gln Asp Asn Trp Ser Lys Lys Asn Ser
   335 340 345
   Pro Trp Thr Gly Asn Ser Gly Thr Tyr Pro Gln Thr Thr Ser Glu
   350 355 360
   Met Tyr Lys Ile Pro Tyr Asp Ser Asn Phe Gly Phe Pro Thr Tyr
   365 370 375
   Arg Ala Gln Lys Asp Tyr Ile Leu Glu Arg Arg Gln Cys Asn Phe
   380 385 390
   Asn Tyr Glu Val Asn Asn Pro Val Ser Lys Lys Val Trp Pro Gln
   395 400 405
   Pro Ser Thr Thr Thr Pro Thr Val Asp Tyr Tyr Glu Tyr His Cys
   410 415 420
   Gly Trp Phe Ser Asn Ile Phe Ile Gly Pro Asn Arg Tyr Asn Leu
   425 430 435
   Gln Phe Gln Thr Ala Tyr Val Asp Thr Thr Tyr Asn Pro Leu Met
   440 445 450
   Asp Lys Gly Lys Gly Asn Lys Ile Trp Phe Gln Tyr Leu Ser Lys
   455 460 465
   Lys Gly Thr Asp Tyr Asn Glu Lys Gln Cys Tyr Cys Thr Leu Glu
   470 475 480
   Asp Met Pro Leu Trp Ala Ile Cys Phe Gly Tyr Thr Asp Tyr Val
   485 490 495
   Glu Thr Gln Leu Gly Pro Asn Val Asp His Glu Thr Ala Gly Leu
   500 505 510
   Ile Ile Met Ile Cys Pro Tyr Thr Gln Pro Pro Met Tyr Asp Lys
   515 520 525
   Asn Arg Pro Asn Trp Gly Tyr Val Val Tyr Asp Thr Asn Phe Gly
   530 535 540
   Asn Gly Lys Met Pro Ser Gly Ser Gly Gln Val Pro Val Tyr Trp
   545 550 555
   Gln Cys Arg Trp Arg Pro Met Leu Trp Phe Gln Gln Gln Val Leu
   560 565 570
   Asn Asp Ile Ser Lys Thr Gly Pro Tyr Ala Tyr Arg Asp Glu Tyr
   575 580 585
   Lys Asn Val Gln Leu Thr Leu Tyr Tyr Asn Phe Ile Phe Asn Trp
   590 595 600
   Gly Gly Asp Met Tyr Tyr Pro Gln Val Val Lys Asn Pro Cys Gly
                   605                 610                 615
   Asp Ser Gly Ile Val Pro Gly Ser Gly Arg Phe Thr Arg Glu Val
                   620                 625                 630
   Gln Val Val Ser Pro Leu Ser Met Gly Pro Ala Tyr Ile Phe His
                   635                 640                 645
   Tyr Phe Asp Ser Arg Arg Gly Phe Phe Ser Glu Lys Ala Leu Lys
                   650                 655                 660
   Arg Met Gln Gln Gln Gln Glu Phe Asp Glu Ser Phe Thr Phe Lys
                   665                 670                 675
   Pro Lys Arg Pro Lys Leu Ser Thr Ala Ala Ala Glu Ile Leu Gln
                   680                 685                 690
   Leu Glu Glu Asp Ser Thr Ser Gly Glu Gly Lys Ser Pro Leu Gln
                   695                 700                 705
   Gln Glu Glu Lys Glu Val Glu Val Leu Gln Thr Pro Thr Val Gln
                   710                 715                 720
   Leu Gln Leu Gln Arg Asn Ile Gln Glu Gln Leu Ala Ile Lys Gln
                   725                 730                 735
   Gln Leu Gln Phe Leu Leu Leu Gln Leu Leu Lys Thr Gln Ser Asn
                   740                 745                 750
   Leu His Leu Asn Pro Gln Phe Leu Ser Pro Ser
                   755                 760
<210> 64
   <211> 165
   <212> PRT
   <213> non-B, non-C, non-G hepatitis virus
<400> 64